# EUROPEAN PATENT APPLICATION

(11) **EP 3 950 698 A1**
(43) Date of publication of application: **09.02.2022**
(21) Application number: 20777639.4
(22) Date of filing: 27.03.2020
(51) Int. Cl.: C07H 21/02, C07H 21/04

(54) **METHOD FOR PRODUCING OLIGONUCLEOTIDE HAVING PHOSPHOROTHIOATE SITE**

(30) Priority: 28.03.2019 JP 2019065158
(71) Applicant: AJINOMOTO CO., INC., Chuo-ku Tokyo 104-8315 (JP)
(72) Inventor: ICHIMARU, Taisuke, Kawasaki-shi, Kanagawa 210-8681 (JP); TAKAHASHI, Daisuke, Kawasaki-shi, Kanagawa 210-8681 (JP); HIRAI, Kunihiro, Kawasaki-shi, Kanagawa 210-8681 (JP); YAMASHITA, Ken, Kawasaki-shi, Kanagawa 210-8681 (JP)
(74) Representative: Mewburn Ellis LLP
(86) International application number: PCT/JP2020/014308
(87) International publication number: WO 2020/196890

(57) **Abstract**

A method for producing an oligonucleotide having a phosphorothioated site, including a step of obtaining a phosphite form or a phosphorous acid diester form by coupling a nucleoside, nucleotide or oligonucleotide wherein a 5'-hydroxy group is not protected, and other group is optionally protected by a protecting group used for nucleic acid synthesis or bonded to a solid phase carrier, and a nucleoside, nucleotide or oligonucleotide wherein a 3'-hydroxy group or a 3'-amino group is modified by a method for forming a phosphite form or a phosphorous acid diester form, and other group is optionally protected by a protecting group used for nucleic acid synthesis in the presence of an antioxidant.

## Description

### [Technical Field]

The present invention relates to methods for producing oligonucleotides having a phosphorothioated site, and are useful in the field of nucleic acid synthesis.

### [Background Art]

Nucleic acid drugs such as antisense, siRNA (small interfering RNA), aptamer and the like are attracting attention as next-generation drugs following antibody drugs. While nucleic acid drugs are expected to have high specificity and effectiveness like antibody drugs, they can be produced by chemical synthesis like conventional drugs composed of low-molecular-weight compounds. In research and development of new nucleic acid drugs, modified nucleic acids in which the phosphorus atom at the binding site between nucleic acids is modified has been studied. A representative example is a nucleic acid drug of a type having a phosphorothioated site (phosphorothioated binding site between nucleic acids) in which a phosphorus atom is S-modified and, for example, Fomivirsen, Mipomersen, Oblimersen, Alicaforsen and the like can be mentioned.

The production method of oligonucleotide having a phosphorothioated site includes solid phase synthesis (solid phase method) and a liquid phase method. To overcome the defects of the solid phase method and the liquid phase method, a liquid phase method using a pseudo solid phase protecting group is also developed (patent documents 1 - 9).

### [Document List]

### [Patent documents]

patent document 1: WO 2012/157723
patent document 2: WO 2013/122236
patent document 3: WO 2017/104836
patent document 4: WO 2005/070859
patent document 5: WO 2013/179412
patent document 6: WO 2014/077292
patent document 7: WO 2017/086397
patent document 8: WO 2018/203574
patent document 9: WO 2018/212236

### [Summary of Invention]

### [Technical Problem]

For oligonucleotide synthesis having a phosphorothioated site, currently, the phosphoramidite method, the H-phosphonate method and the like are widely used, and the dihalophosphine method and the oxazaphospholidine method are also known. In the phosphoramidite method, the dihalophosphine method and the oxazaphospholidine method, a phosphite form is synthesized and, in oxazaphosphoridine the H-phosphonate method, a phosphorous acid diester form is synthesized, in each of which a sulfurization reaction is further performed to obtain the desired oligonucleotide having a phosphorothioated site. However, the present inventors have conducted studies and newly found a problem that when synthesized according to such method, an oxidation reaction of the phosphorus atom also proceeds in the stage of the production of the above-mentioned phosphite form or phosphorous acid diester form and a phosphotriester form or a phosphodiester form (hereinafter sometimes to be referred to as "PO impurity") is by-produced, which in turn inhibits the subsequent sulfurization reaction, and the desired phosphorothioated oligonucleotide cannot be synthesized efficiently. In this "PO impurity", a site that is originally to be phosphorothioated is oxidized into a phosphotriester form or a phosphodiester form. The problem of the present invention is to solve this newly-found problem and provide a desired method for producing an oligonucleotide having a phosphorothioated site in a higher yield.

### [Solution to Problem]

The present inventors have found that this new problem can be solved by co-presence of a specific antioxidant in the stage of synthesizing a phosphite form by using a formation method of a phosphite form (e.g., phosphoramidite method, dihalophosphine method and oxazaphospholidine method), or the stage of synthesizing a phosphorous acid diester form by using a formation method of a phosphorous acid diester form (e.g., H-phosphonate method), which resulted in the completion of the present invention. The present invention provides the following.

[1] A method for producing an oligonucleotide having a phosphorothioated site, comprising a step of obtaining a phosphite form or a phosphorous acid diester form by coupling a nucleoside, nucleotide or oligonucleotide wherein a 5'-hydroxy group is not protected, and other group is optionally protected by a protecting group used for nucleic acid synthesis or bonded to a solid phase carrier (hereinafter sometimes to be referred to as "nucleic acid A"), and a nucleoside, nucleotide or oligonucleotide wherein a 3'-hydroxy group or a 3'-amino group is modified by a method for forming a phosphite form or a phosphorous acid diester form, and other group is optionally protected by a protecting group used for nucleic acid synthesis (hereinafter sometimes to be referred to as "nucleic acid B") in the presence of an antioxidant (step (1)).
[2] The method for producing an oligonucleotide having a phosphorothioated site of the above-mentioned [1], wherein the 3'-hydroxy group or the 3'-amino group in nucleic acid B is modified by a method selected from a phosphoramidite method, an H-phosphonate method, a dihalophosphine method and an oxazaphospholidine method.
[3] The method for producing an oligonucleotide having a phosphorothioated site of the above-mentioned [1] or [2], further comprising a step of reacting the phosphite form or the phosphorous acid diester form obtained in step (1) with a sulfurizing reagent (step (2)).
[4] A method for producing an oligonucleotide having a phosphorothioated site, comprising
   (1) a step of obtaining a phosphite form or a phosphorous acid diester form by coupling a nucleoside, nucleotide or oligonucleotide wherein a 5'-hydroxy group is not protected, and other group is optionally protected by a protecting group used for nucleic acid synthesis or bonded to a solid phase carrier (hereinafter sometimes to be referred to as "nucleic acid A"), and a nucleoside, nucleotide or oligonucleotide wherein a 3'-hydroxy group or a 3'-amino group is modified by a method selected from a phosphoramidite method, an H-phosphonate method, a dihalophosphine method and an oxazaphospholidine method, and other group is optionally protected by a protecting group used for nucleic acid synthesis (hereinafter sometimes to be referred to as "nucleic acid B") in the presence of an antioxidant, and
   (2) a step of reacting the obtained phosphite form or phosphorous acid diester form with a sulfurizing reagent.
[5] The method for producing an oligonucleotide having a phosphorothioated site of any of the above-mentioned [1] to [4], wherein the antioxidant is selected from a phosphorus-based antioxidant, a sulfur-based antioxidant, and a phosphorus-sulfur-based antioxidant.
[6] The method for producing an oligonucleotide having a phosphorothioated site of any of the above-mentioned [1] to [4], wherein the antioxidant is selected from
   a phosphorus-based antioxidant selected from
   (1) P-(R)₃
   (2) P-(OR)₃
   (3) P(R)₂(OR)
   (4) PR(OR)₂
   (5) PH(O) (R)₂
   (6) PH(O)(OR)₂, and
   (7) PH(O)R(OR)
      (in the above-mentioned formulas (1) - (7), each R is optionally the same or different and independently an optionally substituted aryl group or a lower alkyl group, and two Rs are optionally bonded to each other to form a ring together with the adjacent other atom;
      wherein the above-mentioned antioxidant is optionally a dimer in which R portions of the same or different antioxidants are bonded,
      a sulfur-based antioxidant selected from
   (8) R-S-R, and
   (9) 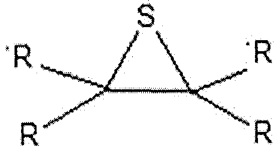 (in the above-mentioned formulas (8) and (9), each R is optionally the same or different and independently a lower alkyl group or an optionally substituted aryl group; wherein the above-mentioned antioxidant is optionally a dimer in which R portions of the same or different antioxidants are bonded, and a phosphorus-sulfur-based antioxidant represented by
   (10) (OR) ₂P (S) -S-M-S-P (S) (OR)₂ wherein each R is optionally the same or different and independently a lower alkyl group or an optionally substituted aryl group, and M is Zn, Ni or Cu; wherein the above-mentioned antioxidant is optionally a dimer in which R portions of the same or different antioxidants are bonded.
[7] The method for producing an oligonucleotide having a phosphorothioated site of any of the above-mentioned [1] to [4], wherein the antioxidant is selected from
   a phosphorus-based antioxidant selected from
   (1) P-(R)₃
   (2) P-(OR)₃
   (3) P(R)₂(OR)
   (4) PR(OR)₂, and
   (5) PH(O)R(OR)
      (in the above-mentioned formulas (1) - (5), each R is optionally the same or different and independently an optionally substituted aryl group or a lower alkyl group, and two Rs are optionally bonded to each other to form a ring together with the adjacent other atom;
      wherein the above-mentioned antioxidant is optionally a dimer in which R portions of the same or different antioxidants are bonded), and
      a sulfur-based antioxidant represented by
   (6) 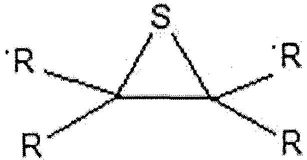 (in the above-mentioned formula (6), each R is optionally the same or different and independently a lower alkyl group or an optionally substituted aryl group;
      wherein the above-mentioned antioxidant is optionally a dimer in which R portions of the same or different antioxidants are bonded.)
[8] The method for producing an oligonucleotide having a phosphorothioated site of any of the above-mentioned [1] to [4], wherein the antioxidant is selected from triphenylphosphine, methyldiphenylphosphine, triethyl phosphite, ethoxydiphenylphosphine, diethoxyphenylphosphine, 9,10-dihydro-9-oxa-10-phosphaphenanthrene 10-oxide (phosphorus-based antioxidant); and isobutylene sulfide (sulfur-based antioxidant).
[9] The method for producing an oligonucleotide having a phosphorothioated site of any of the above-mentioned [1] to [4], wherein the antioxidant is a phosphorus-based antioxidant.
[10] The method for producing an oligonucleotide having a phosphorothioated site of any of the above-mentioned [1] to [5], wherein the antioxidant is a phosphine represented by P-(R)₃.
[11] The method for producing an oligonucleotide having a phosphorothioated site of any of the above-mentioned [1] to [10], wherein the nucleic acid A is a nucleoside, nucleotide or oligonucleotide wherein the 5'-hydroxy group is not protected, and other group is optionally protected by a protecting group used for nucleic acid synthesis.
[12] The method for producing an oligonucleotide having a phosphorothioated site of any of the above-mentioned [1] to [11], wherein the nucleic acid A is a nucleoside, nucleotide or oligonucleotide wherein a 5'-hydroxy group is not protected, at least one group selected from an amino group and an imino group of a nucleic acid base, a 2'-hydroxy group, a 3'-hydroxy group and a 3'-amino group of a ribose residue, and a 3'-hydroxy group and a 3'-amino group of a deoxyribose residue is protected by a protecting group unremovable under acidic conditions but removable under basic conditions (hereinafter sometimes to be referred to as "pseudo-solid-phase protecting group"), and other group is optionally further protected by a protecting group used for nucleic acid synthesis (hereinafter sometimes to be referred to as "nucleic acid a") (preferably, 3'-position of the ribose residue and the deoxyribose residue is a hydroxy group), or
   a nucleoside, nucleotide or oligonucleotide wherein a 5'-hydroxy group is not protected, one OH of a 3'-terminal phosphate group is replaced by -OLⁿ¹-OH wherein Lⁿ¹ is an organic group, the hydroxy group of -OLⁿ¹-OH is protected by a protecting group unremovable under acidic conditions but removable under basic conditions, and other group is optionally protected by a protecting group used for nucleic acid synthesis (hereinafter sometimes to be referred to as "nucleic acid α"), and
   the nucleic acid B is a nucleoside, nucleotide or oligonucleotide wherein a 3'-hydroxy group or 3'-amino group is modified by a method for forming a phosphite form or a phosphorous acid diester form (preferably, a method selected from a phosphoramidite method, an H-phosphonate method, a dihalophosphine method and an oxazaphospholidine method), a 5'-hydroxy group is protected by a temporary protecting group removable under acidic conditions, and other group is optionally protected by a protecting group selected from protecting groups unremovable under acidic conditions but removable under basic conditions (hereinafter sometimes to be referred to as "pseudo-solid-phase protecting group") and protecting groups used for nucleic acid synthesis (hereinafter sometimes to be referred to as "nucleic acid b").
[13] The method for producing an oligonucleotide having a phosphorothioated site of any of the above-mentioned [1] to [12], wherein the nucleic acid B is a nucleoside, nucleotide or oligonucleotide wherein a 3'-hydroxy group is modified by a method for forming a phosphite form or a phosphorous acid diester form (preferably, a method selected from a phosphoramidite method, an H-phosphonate method, a dihalophosphine method and an oxazaphospholidine method), and other group is optionally protected by a protecting group used for nucleic acid synthesis.
[14] The method for producing an oligonucleotide having a phosphorothioated site of any of the above-mentioned [3] to [13], wherein the sulfurizing reagent used in step (2) is selected from 5-[(N,N-dimethylaminomethylidene)amino]-3H-1,2,4-dithiazole-3-thione(DDTT), 3H-1,2-benzodithiol-3-one-1,1-dioxide (Beaucage reagent), 3H-1,2-benzodithiol-3-one, phenylacetyl disulfide (PADS), tetraethylthiuram disulfide (TETD), dipentamethylenethiuram tetrasulfide, 5-phenyl-3H-1,2,4-dithiazol-3-one (POS), 3-amino-1,2,4-dithiazole-5-thione (ADTT, Xanthane hydride), N-[(2-cyanoethyl)thio]phthalimide and sulfur.
[15] The method for producing an oligonucleotide having a phosphorothioated site of any of the above-mentioned [1] to [14], further comprising a step of removing all protecting groups of the obtained oligonucleotide having a phosphorothioated site, and then isolating an unprotected oligonucleotide having a phosphorothioated site.
[16] A method for producing an oligonucleotide having a phosphorothioated site, comprising a step of obtaining a phosphite form or a phosphorous acid diester form by coupling (1) a nucleoside, nucleotide or oligonucleotide wherein a 3'-hydroxy group or 3'-amino group is not protected, and other group is optionally protected by a protecting group used for nucleic acid synthesis or bonded to a solid phase carrier (nucleic acid A-1) (preferably, the 3'-position in nucleic acid A-1 is a hydroxy group), and a nucleoside, nucleotide or oligonucleotide wherein a 5'-hydroxy group is modified by a method for forming a phosphite form or a phosphorous acid diester form (preferably, a method selected from a phosphoramidite method, an H-phosphonate method, a dihalophosphine method and an oxazaphospholidine method), and other group is optionally protected by a protecting group used for nucleic acid synthesis (nucleic acid B-1), in the presence of an antioxidant (step (1-1)).
[17] The method for producing an oligonucleotide having a phosphorothioated site of the above-mentioned [16], further comprising a step of reacting the phosphite form or the phosphorous acid diester form obtained in step (1-1) with a sulfurizing reagent (step (2-1)).

### [Advantageous Effects of Invention]

According to the present invention, the by-production of a phosphotriester form or a phosphodiester form ("PO impurity") can be suppressed by the co-presence of a specific antioxidant in the step of synthesizing a phosphite form or a phosphorous acid diester form by using a method widely used in the art for the formation of a phosphite form or a phosphorous acid diester form, such as a phosphoramidite method, an H-phosphonate method and the like, and the subsequent sulfurization reaction can be performed more efficiently. In this "PO impurity", a site that is originally to be phosphorothioated is converted to a phosphodiester form by oxidation. Therefore, by performing the present invention in all coupling steps, or necessary coupling steps, in nucleic acid chain elongation in oligonucleotide synthesis, the desired oligonucleotide having a phosphorothioated site can be produced in a higher yield. In the production of an oligonucleotide having a phosphorothioated site, the nucleic acid elongation cycle is repeated as appropriate. Thus, even if a trace amount of impurity of 1% or less is by-produced in the reaction step for each base elongation, when it comes to the desired oligonucleotide elongation form, the amount of impurities becomes large. Therefore, in order to obtain high-purity oligonucleotide, it is essential to suppress by-production of impurities in each reaction single step, and the significance of the present invention that suppresses by-production of "PO impurity" is extremely high.

### [Description of Embodiments]

Unless otherwise specified in the sentences, any technical terms and scientific terms used in the present specification, have the same meaning as those generally understood by those of ordinary skill in the art the present invention belongs to. Any methods and materials similar or equivalent to those described in the present specification can be used for practicing or testing the present invention, and preferable methods and materials are described in the following. All publications and patents referred to in the specification are hereby incorporated by reference so as to describe and disclose constructed products and methodology described in, for example, publications usable in relation to the described invention.

### [Production method of oligonucleotide having a phosphorothioated site]

The production method of the present invention is a method for producing an oligonucleotide having a phosphorothioated site that includes the following step (1), and further includes step (2).

### step (1)

A step of obtaining a phosphite form or a phosphorous acid diester form by coupling a nucleoside, nucleotide or oligonucleotide wherein a 5'-hydroxy group is not protected, and other group is optionally protected by a protecting group used for nucleic acid synthesis or bonded to a solid phase carrier (nucleic acid A), and a nucleoside, nucleotide or oligonucleotide wherein a 3'-hydroxy group or a 3'-amino group is modified by a method for forming a phosphite form or a phosphorous acid diester form (e.g., a method selected from a phosphoramidite method, an H-phosphonate method, a dihalophosphine method, and an oxazaphospholidine method), and other group is optionally protected by a protecting group used for nucleic acid synthesis (nucleic acid B), in the presence of an antioxidant.

### step (2)

A step of reacting the obtained phosphite form or phosphorous acid diester form with a sulfurizing reagent.

In the step of elongating a nucleotide chain by a phosphoramidite method known in the art and the like, all or necessary binding sites between nucleic acids can be phosphorothioated in a high yield by performing the above-mentioned step (1), and further, step (2) of the present invention in the elongation step in which the phosphorothioated site is desired to be introduced. The production method of oligonucleotide of the present invention is combined with a production method of an oligonucleotide in which the binding site between nucleic acids is a phosphotriester bond or a phosphodiester bond and which is generally used in the field of nucleic acid synthesis (sometimes to be referred to as "production method of general oligonucleotide" in the following), the cycle of nucleic acid elongation is appropriately repeated depending on the desired oligonucleotide having a phosphorothioated site to elongate the oligonucleotide having a phosphorothioated site, whereby the desired oligonucleotide having a phosphorothioated site can be produced. Such embodiment is also encompassed in the scope of the present invention.

The above-mentioned step (1) and step (2) of the present invention are described in detail below.

While the above-mentioned step (1) and step (2) can be performed under any of the solid-phase and liquid-phase reaction conditions, they are more preferably performed under liquid-phase conditions.

### step (1) coupling reaction in the presence of an antioxidant

The modification for the formation of the phosphite form or phosphorous acid diester form of the 3'-hydroxy group or 3'-amino group of nucleic acid B in step (1) can be performed, for example, by a method known in the art which is selected from a phosphoramidite method, an H-phosphonate method, a dihalophosphine method, an oxazaphospholidine method, and the like.

These methods are widely known to those of ordinary skill in the art, and the phosphite form and the phosphorous acid diester form that can be formed in the process of coupling reaction are also easily understood by those of ordinary skill in the art.

When a coupling product is obtained using nucleic acid B having 3'-amino group instead of nucleic acid B having 3'-hydroxy group and by applying a method such as a phosphoramidite method, an H-phosphonate method, a dihalophosphine method, an oxazaphospholidine method or the like, the binding between the 3'-position and phosphorus atom is -N-P bond instead of -O-P bond. This case is also referred to as a "phosphite form" or a "phosphorous acid diester form" for convenience as in the case of -O-P bond in the present specification. The corresponding "PO impurity" is also referred to as "phosphotriester form" or "phosphodiester form" in the same manner.

Here, nucleic acid B is preferably a nucleoside, nucleotide or oligonucleotide wherein the 3'-hydroxy group is modified by a method for forming a phosphite form or a phosphorous acid diester form, and other group is optionally protected by a protecting group used for nucleic acid synthesis.

The modification by a phosphoramidite method can be performed by referring to, for example, S. L. Beaucage, Protocols for Oligonucleotides and Analogs (Chapter 3) 1993, pages 33-62; M. H. Caruthers et al., Method in Enzymology 1987, 154, 287-313; S. L. Beaucage and M. H. Caruthers, Tetrahedron Letters 1981, 22, 1859-1862; Chemical Society of Japan ed., 5th Edition, Jikken Kagaku Kouza 16, Organic Compound IV, 2010, pp. 377-381, and the like.

The modification using an H-phosphonate method can be performed by referring to, for example, B. F. Froehler, Protocols for Oligonucleotides and Analogs (Chapter 4) 1993, pages 63-80; Chemical Society of Japan ed., 5th Edition, Jikken Kagaku Kouza 16, Organic Compound IV, 2010, pp. 381-384 page and the like.

The modification using a dihalophosphine derivative (dihalophosphine method) can be performed by referring to, for example, The Chemical Society of Japan ed. 4th Edition Jikken Kagaku Kouza 22, Metal Complexes/Transition Metal Clusters, 1999, pages 426-431, and the like.

The modification using an oxazaphospholidine method can be performed by referring to, for example, N. Oka et al., J. Am. Chem. Soc., 2008, 130, pages 16031-16037; N. Oka et al., Organic Letters., 2009, Vol. 11, No. 4, pages 967-970; WO 2011/08682 and the like.

Thus, nucleic acid B wherein the 3'-hydroxy group or a 3'-amino group is modified by a method for forming a phosphite form or a phosphorous acid diester form (e.g., a method selected from a phosphoramidite method, an H-phosphonate method, a dihalophosphine method, and an oxazaphospholidine method) is led to the corresponding phosphite form or phosphorous acid diester form by coupling with nucleic acid A in the presence of an antioxidant.

As the above-mentioned method for forming a phosphite form or a phosphorous acid diester form includes many improved methods known in the art (e.g., WO 2014/010250), and these can also be used for practicing the present invention. In addition, when nucleic acid B modified by a method for forming a phosphite form or a phosphorous acid diester form is commercially available, the commercially available product can also be used for practicing the present invention.

As the antioxidant to be used, it is preferable to select one having an oxidation preventive effect sufficient to prevent by-production of a phosphotriester form or a phosphodiester form ("PO impurity") due to the oxidation of a phosphorus atom of the produced phosphite form or phosphorous acid diester form, and select an antioxidant that does not react with nucleic acid B (phosphoramidite form, H-phosphonate form, etc.) in the coupling step to adversely affect the coupling step itself. Furthermore, when step (2) following the coupling reaction is intended to be performed in the same reactor in the practice under liquid-phase conditions, it is preferable to select an antioxidant that does not adversely affect the subsequent reaction and does not induce by-production of impurities. From such aspect, examples of preferable antioxidant include phosphorus-based antioxidant, sulfur-based antioxidant, and phosphorus-sulfur-based antioxidant. Specific examples include antioxidants represented by the following formulas.

As the phosphorus-based antioxidant, the following can be mentioned.
(1) P-(R)₃ (sometimes to be referred to as "phosphines")
(2) P-(OR)₃ (sometimes to be referred to as "phosphorous acid esters (phosphites)")
(3) P(R)₂(OR) (sometimes to be referred to as "phosphinous acid esters")
(4) PR(OR)₂ (sometimes to be referred to as "phosphonus acid esters")
(5) PH(O)(R)₂ (sometimes to be referred to as "phosphine oxides")
(6) PH(O)(OR)₂ (sometimes to be referred to as "phosphonic acid esters")
(7) PH(O)R(OR) (sometimes to be referred to as "phosphinic acid esters")
(in the above-mentioned formulas (1) to (7), each R is optionally the same or different and independently an optionally substituted aryl group or a lower alkyl group, and two Rs are optionally bonded to each other to form a ring together with the adjacent other atom; and
the above-mentioned antioxidant is optionally a dimer in which R portions of the same or different antioxidants are bonded.)

Examples of the above-mentioned dimer include those shown by the following structural formulas: 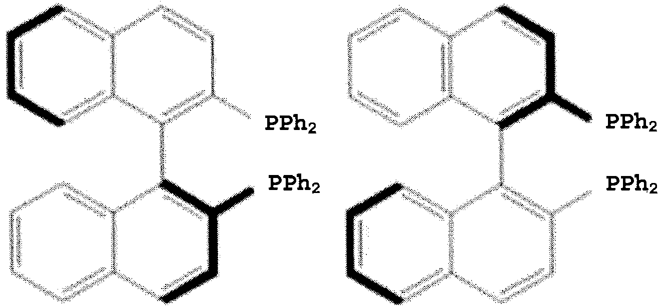 **(S)-BINAP (R)-BINAP**

In the present specification, the "aryl group" is, for example, a "C₆₋₁₄ aryl group", such as phenyl, 1-naphthyl, 2-naphthyl, 1-anthryl, 2-anthryl, and 9-anthryl.

The "aryl group" is optionally substituted by 1 to 3 substituents selected from lower alkyl group, lower alkoxy group, and lower cycloalkyl group (e.g., toluoyl, methoxyphenyl).

In the present specification, the "lower alkyl" is, for example, a "C₁₋₆ alkyl group" such as methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

In the present specification, the "lower alkoxy group" is, for example, a "C₁₋₆ alkoxy group" such as methoxy, ethoxy, propoxy, isopropoxy, butoxy, isobutoxy, sec-butoxy, tert-butoxy, pentyloxy, and hexyloxy.

In the present specification, the "lower cycloalkyl group is, for example, a "C₃₋₁₀ cycloalkyl group" such as cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, and adamantyl.

The same applies to other antioxidants below.

As the sulfur-based antioxidant, the following can be mentioned.
(8) R-S-R
(9)

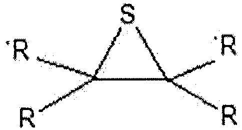

(in the above-mentioned formulas (8) and (9), each R is optionally the same or different and independently a lower alkyl group or an optionally substituted aryl group; and the above-mentioned antioxidant is optionally a dimer in which R portions of the same or different antioxidants are bonded.)

Examples of the phosphorus-sulfur-based antioxidant include the following.
(10) (OR)₂P (S) -S-M-S-P (S) (OR)₂
wherein each R is optionally the same or different and independently a lower alkyl group or an optionally substituted aryl group, and M is Zn, Ni or Cu;
the above-mentioned antioxidant is optionally a dimer in which R portions of the same or different antioxidants are bonded.

Among the above-mentioned antioxidants, more preferred are phosphines represented by the formula: P-(R)₃, phosphorous acid esters (or phosphites) represented by the formula: P-(OR)₃, phosphinous esters represented by the formula: P(R)₂(OR), phosphonus acid esters represented by the formula: PR(OR)₂, phosphinic acid esters represented by the formula: PH(O)R(OR), and sulfur-based antioxidant represented by

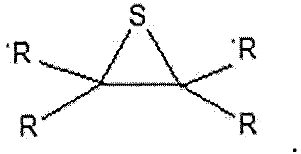

As the phosphines represented by the formula: P-(R)₃, triphenylphosphine and methyldiphenylphosphine are preferred. Among others, triphenylphosphine is more preferred. As the phosphorous acid esters (or phosphite) represented by the formula: P-(OR)₃, triethyl phosphite (P(OEt)₃) is preferred.

As the phosphinous esters represented by the formula: P(R)₂(OR), ethoxydiphenylphosphine is preferred. As the phosphonus acid esters represented by the formula: PR(OR)₂, diethoxyphenylphosphine is preferred.

As the phosphinic acid esters represented by the formula: PH(O)R(OR), 9,10-dihydro-9-oxa-10-phosphaphenanthrene 10-oxide represented by

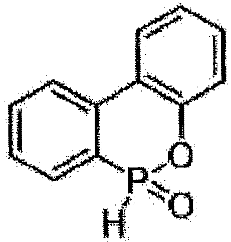

is preferred.

As the sulfur-based antioxidant represented by

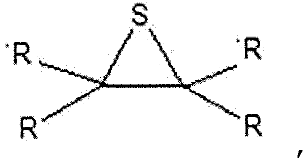

isobutylene sulfide represented by

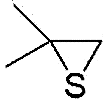

is preferred.

The amount of the antioxidant to be used is preferably as small as possible in consideration of the influence on the subsequent reaction. It is generally preferably 0.1 - 2 mol, more preferably 0.2 - 1 mol, per 1 mol of the nucleic acid A used in the coupling reaction.

The solvent to be used when this step is performed under liquid-phase conditions is not particularly limited as long as it is a solvent in which nucleic acid A, nucleic acid B, antioxidant and the like are dissolved and does not adversely affect the reaction. Examples thereof include methylene chloride, acetonitrile, chloroform, tetrahydrofuran, acetone, ethyl acetate, CPME, toluene, cyclohexane, mixed solvents thereof, and the like. Among these, chloroform, tetrahydrofuran, toluene, methylene chloride, acetonitrile, and mixed solvents thereof are preferred.

The concentrations of nucleic acid A and nucleic acid B to be used in this step in a solution are not particularly limited as long as they are dissolved in the solvent.

The amount of nucleic acid B to be used is preferably 1 - 3 mol, more preferably 1.2 - 2.5 mol, per 1 mol of the nucleic acid A to be used.

The coupling reaction in this step can be performed by adding an activator. For example, as an activator of the phosphoramidite method, tetrazole derivatives (e.g., 5-ethylthio-1H-tetrazole (ETT), 5-benzylthio-1H-tetrazole, and the like), imidazole derivatives (e.g., 4,5-dicyanoimidazole, and the like) and the like, which are widely used in the art, can be mentioned. It is preferably a tetrazole derivative, more preferably 5-ethylthio-1H-tetrazole. As an activator of the H-phosphonate method, a condensing agent alone, a combination of a condensing agent and a condensing additive, pivaloyl chloride and the like can be mentioned. As the condensing agent alone, HATU (O-(7-azabenzotriazol-1-yl)-1,1,3,3-tetramethyluronium hexafluorophosphate), HCTU (O-(1H-6-chlorobenzotriazol-1-yl)-1,1,3,3-tetramethyluronium hexafluorophosphate), Pybop (hexafluorophosphoric acid (benzotriazol-1-yloxy)tripyrrolidinophosphonium), BopCl (bis (2-oxo-3-oxazolidinyl)phosphine acid chloride), TBTU (O-(benzotriazol-1-yl)-N,N,N',N'-tetramethyluronium tetrafluoroborate), HBTU (2-(1H-benzotriazol-1-yl)-1,1,3,3-tetramethyluronium hexafluorophosphate) and the like can be mentioned. As the combination of a condensing agent and a condensing additive, combinations of condensing agents such as DIC (diisopropylcarbodiimide), EDC·HCl (1-ethyl-3-(3-dimethylaminopropyl)carbodiimide hydrochloride, WSC·HCl), DCC (dicyclohexylcarbodiimide) and the like and condensing additives such as HOAt (1-hydroxy-7-azabenzotriazole), HOCt (1-hydroxy-1H-1,2,3-triazole-5-carboxylic acid ethyl ester), HOBt (1-hydroxybenzotriazole), HOOBt (3,4-dihydro-3-hydroxy-4-oxo-1,2,3-benzotriazine), DMAP (N,N-dimethyl-4-aminopyridine) and the like can be mentioned.

While the reaction temperature of the coupling reaction in this step is not particularly limited as long as the reaction proceeds, it is preferably -10°C to 60°C, more preferably 5°C to 35°C. While the reaction time varies depending on the kind of the oligonucleotide to be used, the kind of antioxidant, the kind of solvent, the reaction temperature and the like, it is, for example, 5 min to 24 hr.

This step can be performed, for example, by adding nucleic acid B and an antioxidant to a solution in which nucleic acid A is dissolved, and then adding an activator. This is one embodiment, and the present invention is not limited to this embodiment.

In the present invention, in order to suppress the formation of by-products during step (2), a step of quenching nucleic acid B activated by the phosphoramidite method or the like can be further included before step (2).

As the quenching agent, those known in the art can be used as the quenching agent in this step. Only one kind of a quenching agent may be used, or two or more kinds thereof may be used in combination. Examples of the quenching agent include alcohols (e.g., optionally halogenated monohydric alcohols such as methanol, 2-propanol, t-butanol, 2,2,2,-trifluoroethanol (TFE), tetrahydrofurfuryl alcohol, furfurylalcohol, 2,3-O-isopropylidene-D-ribofuranose, 3'-O-triisopropylsilyl-thymidine and the like, optionally halogenated polyhydric alcohols such as ethylene glycol, diethylene glycol and the like), phenols (e.g., 4-nitrophenol, pentafluorophenol, and the like) and amines (e.g., morpholine, and the like).

The quenching agent is preferably at least one selected from alcohols and amines, more preferably at least one selected from methanol, 2-propanol, t-butanol, 2,2,2-trifluoroethanol, tetrahydrofurfuryl alcohol, and morpholine. Among others, 2,2,2-trifluoroethanol is preferred.

The amount of the quenching agent to be used in this step is preferably 1 - 20 mol, more preferably 1 - 15 mol, further preferably 1 - 10 mol, per 1 mol of the amount of the nucleic acid B in step (1).

The temperature of the reaction solution after addition of a quenching agent is not particularly limited as long as the nucleic acid B can be quenched and is preferably 5°C - 40°C, more preferably 15°C - 30°C.

### step (2) sulfurization reaction by sulfurizing reagent

The sulfurizing reagent to be used in this step is not particularly limited as long as it is capable of converting a phosphite bond or a phosphorous acid diester bond to a phosphorothioate bond. 5-[(N,N-dimethylaminomethylidene)amino]-3H-1,2,4-dithiazole-3-thione (DDTT), 3H-1,2-benzodithiol-3-one-1,1-dioxide (Beaucage reagent), 3H-1,2-benzodithiol-3-one, phenylacetyl disulfide (PADS), tetraethylthiuram disulfide (TETD), dipentamethylenethiuram tetrasulfide, 5-phenyl-3H-1,2,4-dithiazol-3-one (POS), 3-amino-1,2,4-dithiazole-5-thione (ADTT, xanthan hydride), sulfur, cyanoethylmethylthiosulfonate, N-[(2-cyanoethyl)thio]succinimide, N-[(2-cyanoethyl)thio]morpholine-3,5-dione, and N-[(2-cyanoethyl)thio]phthalimide are preferable.

Since the reaction can proceed favorably, 5-[(N,N-dimethylaminomethylidene)amino]-3H-1,2,4-dithiazole-3-thione (DDTT), 3H-1,2-benzodithiol-3-one-1,1-dioxide (Beaucage reagent), 3H-1,2-benzodithiol-3-one, phenylacetyl disulfide (PADS), 5-phenyl-3H-1,2,4-dithiazol-3-one (POS), cyanoethylmethylthiosulfonate, and N-[(2-cyanoethyl)thio]phthalimide are more preferable, 5-[(N,N-dimethylaminomethylidene)amino]-3H-1,2,4-dithiazole-3-thione (DDTT), 3H-1,2-benzodithiol-3-one-1,1-dioxide (Beaucage reagent), 5-phenyl-3H-1,2,4-dithiazol-3-one (POS) cyanoethylmethylthiosulfonate, and N-[(2-cyanoethyl)thio]phthalimide are further preferable, and 5-[(N,N-dimethylaminomethylidene)amino]-3H-1,2,4-dithiazole-3-thione (DDTT), 5-phenyl-3H-1,2,4-dithiazol-3-one (POS), cyanoethylmethylthiosulfonate, and N-[(2-cyanoethyl)thio]phthalimide are particularly preferable. Such sulfurizing reagent can be used by diluting with a suitable solvent at a concentration of 0.05 - 2 M. Such diluent solvent is not particularly limited as long as it is inert to the reaction and, for example, dichloromethane, acetonitrile, pyridine or a mixed solvent of any of these can be mentioned.

The amount of the sulfurizing reagent to be used is, for example, 1 - 50 mol, preferably 1 - 5 mol, per 1 mol of the amount of nucleic acid A to be used in step (1).

The reaction temperature is not particularly limited as long as the reaction proceeds, and 0°C - 60°C is preferable, 10°C - 40°C is more preferable. The reaction time varies depending on the kind of the phosphite form or phosphorous acid diester form, the kind of the sulfurizing reagent to be used, reaction temperature and the like, and is, for example, 1 min to 3 hr.

The sulfurization reaction with a sulfurizing reagent in step (2) may be performed for each coupling reaction in the presence of an antioxidant in step (1), or may be collectively performed after nucleoside, nucleotide, or oligonucleotide is elongated by several times of the coupling reaction in the presence of an antioxidant in step (1). Those of ordinary skill in the art can perform the present invention by appropriately selecting the manner of the sulfurization reaction according to the reaction conditions such as the method for forming the phosphite form or the phosphorous acid diester form to be used, and the like.

The above steps (1) and/or step (2) can be performed regardless of the presence or absence of a protecting group for the hydroxy group at the 5'-position of nucleic acid B. For example, the below-mentioned separation step can also be performed after step (1), or step (2), and further, the below-mentioned separation step can also be performed continuously from step (1). In the nucleic acid elongation step, when the hydroxy group at the 5'-position of nucleic acid B is protected, the deprotection of the protecting group is an arbitrary step and can be appropriately performed according to the synthesis plan.

The step (1) and step (2) of the present invention have been described in detail above. By performing the present invention, the drawbacks of the conventional methods can be improved, and the desired phosphorothioated site can be more efficiently introduced into the nucleic acid junction of oligonucleotide. The present invention is superior as a method for producing an oligonucleotide having a phosphorothioated site.

In performing the present invention, the following various steps, which are performed as necessary in the process of producing oligonucleotides in the art, can also be performed.

In each step, the resultant product can be isolated for each step and then the next step can be performed, or each step can be performed continuously as appropriate.

### [deprotection step]

In this step, the protecting group of the 5'-hydroxy group of the oligonucleotide having a phosphorothioated site and obtained in step (2) is removed by an acid to obtain an oligonucleotide having a phosphorothioated site in which the 5'-hydroxy group is not protected. This step can be performed according to a method generally performed in the art (e.g., WO 2012/157723, WO 2013/122236, WO 2017/104836).

### [neutralization step]

A base may be added to the reaction solution after the above-mentioned deprotection step to neutralize the acid used. In the production method of the present invention, the acid used in the deprotection step can be removed from the reaction system by the below-mentioned [solid-liquid separation or extraction] and washing as necessary. Thus, the neutralization step is not essential. This step can be performed according to a method generally performed in the art (e.g., WO 2012/157723, WO 2013/122236, WO 2017/104836).

### [solid-liquid separation or extraction step]

In this step, when any of the groups of the oligonucleotide obtained above and having a phosphorothioated site wherein the 5'-hydroxy group is not protected is protected by a protecting group unremovable under acidic conditions but removable under basic conditions (pseudo-solid-phase protecting group), a polar solvent is added to the reaction solution containing the oligonucleotide to allow for precipitation and purification of the oligonucleotide (solid-liquid separation), or a polar solvent is added to the reaction solution to allow for separation of the layers between the polar solvent and the non-polar solvent, and the oligonucleotide is transferred into the non-polar solvent layer to perform purification (extraction). This step can be performed according to the methods described in detail in, for example, WO 2012/157723, WO 2013/122236, and WO 2017/104836.

When the oligonucleotide having a phosphorothioated site obtained in step (2) has the finally desired nucleic acid sequence, it may be successively subjected to the "deprotection step" and the "isolation step". That is, a step of isolating an unprotected oligonucleotide having a phosphorothioated site after removing all protecting groups from the oligonucleotide having a phosphorothioated site obtained in step (2) can be further contained. This step can be performed according to the methods described in detail in, for example, WO 2012/157723, WO 2013/122236, WO 2017/104836.

The progress of the reaction in each of the above-mentioned steps can be confirmed by a method similar to conventional liquid phase organic synthesis reaction. That is, the reaction can be traced by thin layer silica gel chromatography, high performance liquid chromatography and the like.

In the above, the steps (1) and (2) of the present invention have been described in detail, and a method capable of efficiently introducing a phosphorothioated site into a desired site in the process of nucleic acid elongation has been described. The present invention is characterized in that, for the introduction of a phosphorothioated site, a coupling reaction is performed between an appropriately protected nucleic acid A and nucleic acid B in the presence of a specific antioxidant. As long as this feature can be utilized, the production method of the present invention can be widely applied to appropriately protected nucleoside, nucleotide or oligonucleotide (optionally having a phosphorothioated site) regardless of the structure of the nucleic acid and the like. The oligonucleotide produced in the present invention includes not only oligonucleotides in which all phosphorus atoms are S-modified phosphorothioated sites (All PS form), but also oligonucleotides having phosphorothioated site in which only a specific partial phosphorus atom is S-modified and the remaining phosphorus atoms are not S-modified (Mixed Backbone Oligonucleotide, generally sometimes referred to as "MBO")).

The nucleic acid A and nucleic acid B to which the production method of the present invention is applied are described in detail below.

In one embodiment in which the production method of the present invention is applied,
the nucleic acid A is a nucleoside, nucleotide or oligonucleotide wherein a 5'-hydroxy group is not protected, and other group is optionally protected by a protecting group used for nucleic acid synthesis or bonded to a solid phase carrier, and
the nucleic acid B is a nucleoside, nucleotide or oligonucleotide wherein a 3'-hydroxy group or 3'-amino group is modified by a method for forming a phosphite form or a phosphorous acid diester form (e.g., a method selected from a phosphoramidite method, an H-phosphonate method, a dihalophosphine method and an oxazaphospholidine method), and other group is optionally protected by a protecting group used for nucleic acid synthesis.

In a more preferred embodiment, nucleic acid B is a nucleoside, nucleotide or oligonucleotide wherein a 3'-hydroxy group is modified by a method for forming a phosphite form or a phosphorous acid diester form, and other group is optionally protected by a protecting group used for nucleic acid synthesis.

In the present specification, the "nucleoside" to be the constitutional unit of oligonucleotide means a compound wherein a nucleic acid base is bonded to the 1'-position of a sugar (e.g., 2-deoxyribose or ribose, or 2-deoxyribose or ribose wherein 2-position carbon atom and 4-position carbon atom are bonded by a divalent organic group, or the like) by N-glycosidation.

In the present specification, the "sugar" also encompasses an amino sugar wherein a hydroxy group is replaced by an amino group, ribose wherein a 2-hydroxy group is replaced by a halogen atom, and ribose having an intramolecularly crosslinked structure (LNA; BNA).

Examples of the 2-deoxyribose or ribose wherein 2-position carbon atom and 4-position carbon atom are bonded by a divalent organic group include the following compounds.

In the following formulas, R is any of a hydrogen atom, a substituted or unsubstituted hydrocarbon group, a substituted or unsubstituted hydroxy group, and a substituted or unsubstituted amino group, and R' is either a hydrogen atom or a hydroxy group.

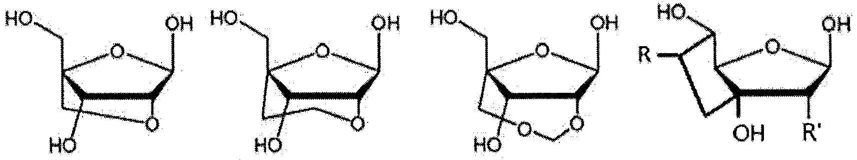

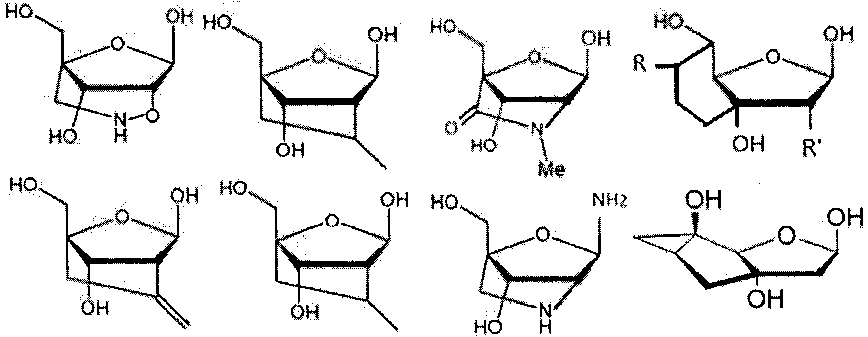

Examples of the amino sugar include 2-deoxyribose wherein 3-hydroxy group is replaced by amino group, ribose wherein 3-hydroxy group is replaced by amino group, and ribose wherein 3-hydroxy group is replaced by amino group and 2-hydroxy group is replaced by halogen, shown below (in the following formulas, X^{s} is a halogen atom).

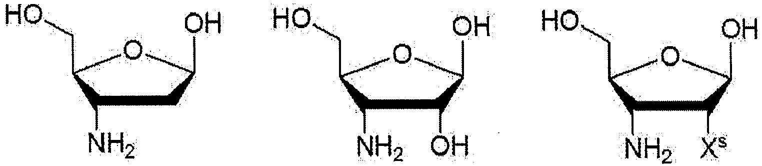

In the present specification, the "phosphate group" encompasses not only -O-P(O)(OH)₂ but also a group wherein oxygen atom is replaced by sulfur atom or NH (e.g., -OP(S) (OH)₂, -NH-P(O) (OH)₂, -NH-P(S) (OH)₂). In addition, a group wherein hydroxy group (-OH) in phosphate group is replaced by - OR^{p} wherein R^{p} is an organic group such as a protecting group of phosphate group or the like (e.g., protected phosphate group) is also encompassed in the "phosphate group".

In the present specification, the "nucleotide" means a compound wherein phosphate group is bonded to nucleoside. Examples of the nucleotide wherein 3'-hydroxy group or 5'-hydroxy group is replaced by phosphate group include the compounds shown by the following formulas (in the following formulas, R^{m1} and R^{m2} are each independently a hydrogen atom or an organic group (excluding nucleoside residue), X^{m} is a hydrogen atom, a hydroxy group or a halogen atom, R₁ and R₂ are each a hydrogen atom or an alkyl group, R₁ and R₂ may be bonded to each other to form a 5- or 6-membered ring together with other atoms adjacent thereto, and R₃ and R₄ are each a hydrogen atom or a phenyl group).

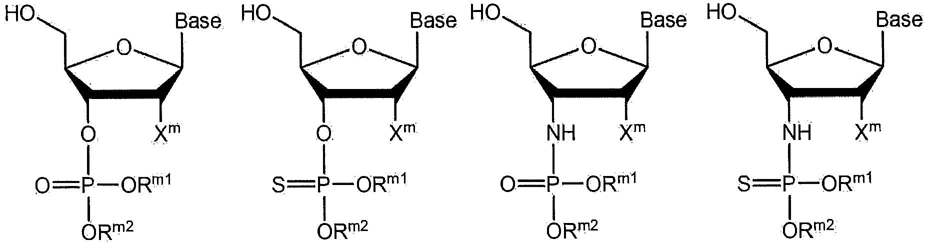

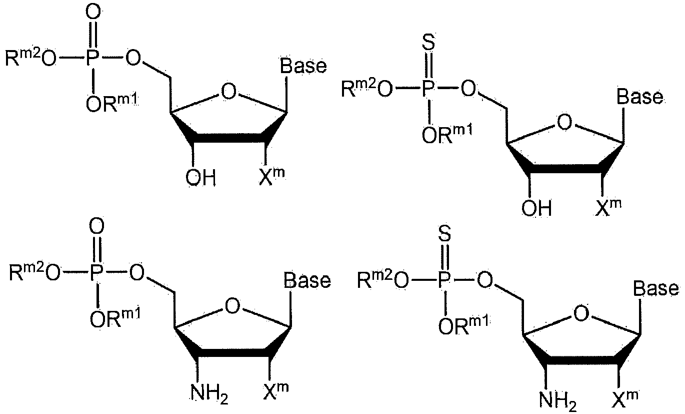

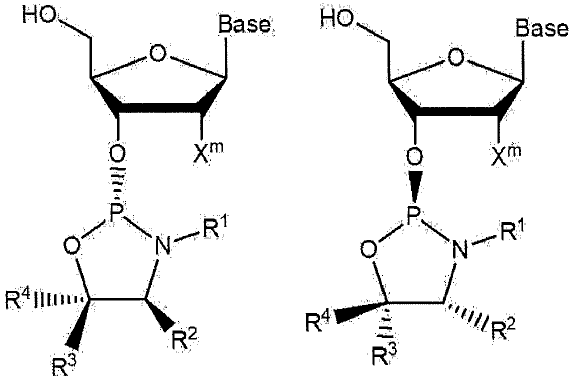

Preferable examples of other nucleotide in which the 3'-hydroxy group or the 5'-hydroxy group is replaced with a phosphate group include those represented by the following formulas (I'), (II') and (III').

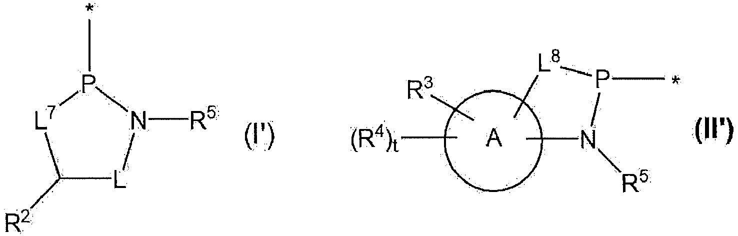

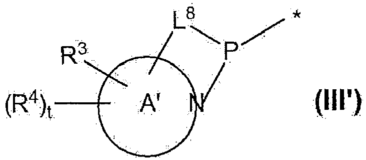

[in the above-mentioned formulas (I'), (II') and (III'), * shows a binding position with a 3'-hydroxy group or a 3'-amino group;
L⁷ is -O- or -S-;
L⁸ is -L-O-, -L-S-, -L-C(R¹)(R²)-O-, -L-C(R¹)(R²)-S-, -L^{s}-O- or-L^{s}-S-;
ring A is a 3- to 20-membered monocyclic, bicyclic or polycyclic ring having 0 - 10 hetero atoms independently selected from optionally substituted oxygen, nitrogen, sulfur, phosphorus and silicon;
ring A' is a 3- to 20-membered monocyclic, bicyclic or polycyclic ring having 1 - 10 hetero atoms independently selected from optionally substituted oxygen, nitrogen, sulfur, phosphorus and silicon and having a -N(R⁶)- moiety;
L is a covalent bond or optionally substituted C₁₋₆ alkylene, wherein one or more methylene units may be independently replaced with -L'-;
each L' is independently a covalent bond, optionally substituted divalent C₁₋₃ alkylene, -C(R³)(R⁴)-, -C(R³)(R⁴)-C(R³) (R⁴)-, -Cy- or -C(R³)[C(R⁴)₃]-;
R¹, R², R³, R⁴ and R⁵ are each independently a hydrogen, -L^{s}-R, a halogen, -CN, -NO₂, -L^{s}-Si(R)₃, -OR, -SR or -N(R)₂;
each L^{S} is independently a covalent bond or an optionally substituted divalent straight chain or branched chain group selected from a C₁₋₃₀ aliphatic group, and a C₁₋₃₀ hetero aliphatic group having 1 - 10 hetero atoms independently selected from oxygen, nitrogen, sulfur, phosphorus and silicon, wherein one or more methylene units may be independently replaced with an optionally substituted group selected from C₁₋₆ alkylene, C₁₋₆ alkenylene, -C≡C-, a divalent C₁-C₆ hetero aliphatic group having 1 - 5 hetero atoms independent selected from oxygen, nitrogen, sulfur, phosphorus and silicon, -C(R')₂-, -Cy-, -O-, -S-, -S-S-, -N(R')-, -C(O)-, -C(S)-, -C(NR')-,-C(O)N(R')-, -N(R')C(O)N(R')-, -N(R')C(O)O-, -S(O)-, -S(O)₂-,-S(O)₂N(R')-, -C(O)S-, -C(O)O-, -P(O)(OR')-, -P(O) (SR')-,-P(O)(R')-, -P(O) (NR')-, -P(S) (OR')-, -P(S)(SR')-, -P(S)(R')-,-P(S) (NR')-, -P(R')-, -P(OR')-, -P(SR')-, -P(NR')-,-P(OR') [B(R')₃]-, -OP(O) (OR')O-, -OP(O) (SR')O-, -OP(O)(R')O-,-OP(O)(NR')O-, -OP(OR')O-, -OP(SR')O-, -OP(NR')O-, -OP(R')O- and -OP(OR') [B(R')₃]O-, and one or more carbon atoms may be independently replaced with Cy^{L};
each -Cy- is independently an optionally substituted divalent group selected from a C₃₋₂₀ alicyclic ring, a C₆₋₂₀ aryl ring, a 5- to 20-membered heteroaryl ring having 1 - 10 hetero atoms independently selected from oxygen, nitrogen, sulfur, phosphorus and silicon, and a 3 - 20-membered heterocyclyl ring having 1 - 10 hetero atoms independently selected from oxygen, nitrogen, sulfur, phosphorus and silicon;
each Cy^{L} is independently an optionally substituted tetravalent group selected from a C₃₋₂₀ alicyclic ring, a C₆₋₂₀ aryl ring, a 5- to 20-membered heteroaryl ring having 1 - 10 hetero atoms independently selected from oxygen, nitrogen, sulfur, phosphorus and silicon, and a 3- to 20-membered heterocyclyl ring having 1 - 10 hetero atoms independently selected from oxygen, nitrogen, sulfur, phosphorus and silicon;
each R' is independently -R, -C(O)R, -CO₂R or -SO₂R;
t is 0 - 20;
R⁶ is R';
R⁷ is -OH or -SH;
R⁸ is -L-R⁷, -L-C (R¹) (R²)-R⁷ or -L^{s}-R⁷;
at least one of R¹, R², R³ and R⁴ is not a hydrogen;
each R is independently a hydrogen, or an optionally substituted group selected from a C₁₋₃₀ aliphatic group, a C₁₋₃₀ hetero aliphatic group having 1 - 10 hetero atoms independently selected from oxygen, nitrogen, sulfur, phosphorus and silicon, a C₆₋₃₀ aryl group, a C₆₋₃₀ arylaliphatic group, a C₆₋₃₀ arylheteroaliphatic group having 1 - 10 hetero atoms independently selected from oxygen, nitrogen, sulfur, phosphorus and silicon, a 5- to 30-membered heteroaryl group having 1 - 10 hetero atoms independently selected from oxygen, nitrogen, sulfur, phosphorus and silicon, and a 3- to 30-membered heterocyclyl group having 1 - 10 hetero atoms independently selected from oxygen, nitrogen, sulfur, phosphorus and silicon, or two R groups may be joined to independently form a covalent bond; or
two or more Rs on the same atom may independently form, together with the atom, an optionally substituted 3- to 30-membered monocyclic, bicyclic or polycyclic ring having 0 - 10 hetero atoms independently selected from oxygen, nitrogen, sulfur, phosphorus and silicon in addition to the atom; or two or more R groups on two or more atoms may independently form, together with the intervening atoms, an optionally substituted 3- to 30-membered monocyclic, bicyclic or polycyclic ring having 0 - 10 hetero atoms independently selected from oxygen, nitrogen, sulfur, phosphorus and silicon in addition to the intervening atoms,
provided that R¹ and R² are not the same, and R³ and R⁴ are not the same.]

In the present specification, the "oligonucleotide" means a compound wherein one or more nucleotides are bonded to nucleoside. Unless particularly indicated, the "oligonucleotide" also encompasses phosphorothioated oligonucleotide wherein oxygen atom of phosphate group is replaced by sulfur atom, oligonucleotide wherein -O- of phosphate group is replaced by -NH-, and oligonucleotide wherein hydroxy group (-OH) in phosphate group is replaced by - OR^{p} wherein R^{p} is an organic group. While the number of nucleosides in the oligonucleotide of the present invention is not particularly limited, it is preferably 3 - 50, more preferably 5-30.

In the present specification, the "3'-amino group" means an amino group bonded to the 3'-position carbon atom of nucleoside, nucleotide or oligonucleotide.

In the present specification, the "5'-amino group" means an amino group bonded to the 5'-position carbon atom of nucleoside, nucleotide or oligonucleotide.

In the present specification, the "3'-phosphate group" means a phosphate group bonded to the 3'-position carbon atom of nucleotide or oligonucleotide.

In the present specification, the "5'-phosphate group" means a phosphate group bonded to the 5'-position carbon atom of nucleotide or oligonucleotide.

In the present specification, the "nucleic acid base" is not particularly limited as long as it can be used for the synthesis of nucleic acid and includes, for example, pyrimidine bases such as cytosyl group, uracil group, thyminyl group and the like, and purine bases such as adenyl group, guanyl group and the like can be mentioned. In addition to the above-mentioned groups, a modified nucleic acid base, which is a nucleic acid base substituted by any 1 to 3 substituents (e.g., halogen atom, alkyl group, aralkyl group, alkoxy group, acyl group, alkoxyalkyl group, a hydroxy group, amino group, monoalkylamino, dialkylamino, carboxy, cyano, nitro etc.) at any position(s) (e.g., 8-bromoadenyl group, 8-bromoguanyl group, 5-bromocytosyl group, 5-iodocytosyl group, 5-bromouracil group, 5-iodouracil group, 5-fluorouracil group, 5-methylcytosyl group, 8-oxoguanyl group, hypoxanthinyl group etc.), are also encompassed in the nucleic acid base. The "optionally protected nucleic acid base" means, for example, that an amino group may be protected by the below-mentioned "amino group protecting group" in an adenyl group, a guanyl group or a cytosyl group, which is a nucleic acid base having an amino group, and a nucleic acid base wherein the amino group therein is protected by a protecting group sustainable under the deprotection conditions of the 5'-position of the nucleotide is preferable.

In the present specification, the "protecting group used for nucleic acid synthesis" is not particularly limited as long as it is generally used in the field of nucleic acid synthesis, and examples thereof include the following.

### 1) Hydroxy-protecting group (protecting group for hydroxy group in nucleic acid base portion is described in the protecting group for nucleic acid base portion described later).

Unless otherwise specified, the "hydroxy-protecting group" is not particularly limited and for example, any protecting group described in PROTECTIVE GROUPS IN ORGANIC SYNTHESIS, 3rd ed., JOHN WILLY&SONS (1999) and the like can be mentioned. Specifically, a methyl group, benzyl group, p-methoxybenzyl group, a tert-butyl group, methoxymethyl group, methoxyethyl group, 2-tetrahydropyranyl group, ethoxyethyl group, cyanoethyl group, cyanoethoxymethyl group, phenylcarbamoyl group, 1,1-dioxothiomorpholine-4-thiocarbamoyl group, acetyl group, pivaloyl group, benzoyl group, trimethylsilyl group, triethylsilyl group, triisopropylsilyl group, tert-butyldimethylsilyl group, [(triisopropylsilyl)oxy]methyl (Tom) group, 1-(4-chlorophenyl)-4-ethoxypiperidin-4-yl (Cpep) group and the like can be mentioned. The protecting group of the optionally protected hydroxy group is preferably a triethylsilyl group, a triisopropylsilyl group or a tert-butyldimethylsilyl group, particularly preferably a tert-butyldimethylsilyl group, from the aspects of economic efficiency and easy availability.

### 2) amino-protecting group

Unless otherwise specified, the "amino-protecting group" is not particularly limited, and examples thereof include the protecting groups described in Greene's PROTECTIVE GROUPS IN ORGANIC SYNTHESIS, 4th edition, Wiley-Interscience, 2006 and the like. Specific examples of the protecting group include, pivaloyl group, pivaloyloxymethyl group, acetyl group, trifluoroacetyl group, phenoxyacetyl group, 4-isopropylphenoxyacetyl group, 4-tert-butylphenoxyacetyl group, benzoyl group, isobutyryl group, (2-hexyl)decanoyl group, dimethylformamidinyl group, 1-(dimethylamino)ethylidene group, 9-fluorenylmethyloxycarbonyl group. Among them, acetyl group, phenoxyacetyl group, 4-isopropylphenoxyacetyl group, benzoyl group, isobutyryl group, (2-hexyl)decanoyl group, dimethylformamidinyl group, and 1-(dimethylamino)ethylidene group is preferable.

### 3) nucleic acid base-protecting group

In an adenyl group, a guanyl group or a cytosyl group, which is a nucleic acid base having an amino group, the amino group may be protected by the aforementioned "amino-protecting group". A nucleic acid base wherein the amino group therein is protected by a protecting group sustainable under the deprotection conditions of the 5'-position of the nucleotide is preferred.

The carbonyl group of the nucleic acid base is also optionally protected, and can be protected, for example, by reacting phenol, 2,5-dichlorophenol, 3-chlorophenol, 3,5-dichlorophenol, 2-formylphenol, 2-naphthol, 4-methoxyphenol, 4-chlorophenol, 2-nitrophenol, 4-nitrophenol, 4-acetylaminophenol, pentafluorophenol, 4-pivaloyloxybenzyl alcohol, 4-nitrophenethyl alcohol, 2-(methylsulfonyl)ethanol, 2-(phenylsulfonyl)ethanol, 2-cyanoethanol, 2-(trimethylsilyl)ethanol, dimethylcarbamoyl chloride, diethylcarbamoyl chloride, ethylphenylcarbamoyl chloride, 1-pyrrolidinecarbonyl chloride, 4-morpholinecarbonyl chloride, diphenylcarbamoyl chloride and the like. In some cases, the carbonyl-protecting group does not need to be particularly introduced.

In a more preferred embodiment in which the production method of the present invention is applied, the nucleic acid A is a nucleoside, nucleotide or oligonucleotide wherein a 5'-hydroxy group is not protected, at least one group selected from an amino group and an imino group of a nucleic acid base, a 2'-hydroxy group, a 3'-hydroxy group and a 3'-amino group of a ribose residue, and a 3'-hydroxy group and a 3'-amino group of a deoxyribose residue is protected by a protecting group unremovable under acidic conditions but removable under basic conditions (pseudo-solid-phase protecting group), and other group is optionally further protected by a protecting group used for nucleic acid synthesis (hereinafter sometimes to be referred to as "nucleic acid a"), or
a nucleoside, nucleotide or oligonucleotide wherein a 5'-hydroxy group is not protected, one OH of a 3'-terminal phosphate group is replaced by -OLⁿ¹-OH wherein Lⁿ¹ is an organic group, the hydroxy group of -OLⁿ¹-OH is protected by a protecting group unremovable under acidic conditions but removable under basic conditions (pseudo-solid-phase protecting group), and other group is optionally protected by a protecting group used for nucleic acid synthesis (hereinafter sometimes to be referred to as "nucleic acid α"), and
the nucleic acid B is a nucleoside, nucleotide or oligonucleotide wherein a 3'-hydroxy group or 3'-amino group is modified by a method selected from a phosphoramidite method, an H-phosphonate method, a dihalophosphine method and an oxazaphospholidine method, a 5'-hydroxy group is protected by a temporary protecting group removable under acidic conditions, and other group is optionally protected by a protecting group selected from protecting groups unremovable under acidic conditions but removable under basic conditions (pseudo-solid-phase protecting group) and protecting groups used for nucleic acid synthesis (hereinafter sometimes to be referred to as "nucleic acid b").

Examples of the "protecting group used for nucleic acid synthesis" in nucleic acid a, nucleic acid α and nucleic acid b include the same protecting groups as described above for nucleic acid A and nucleic acid B.

As the "protecting group unremovable under acidic conditions but removable under basic conditions (pseudo-solid-phase protecting group)" in nucleic acid a, nucleic acid α and nucleic acid b, each independently, a protecting group having a linear aliphatic hydrocarbon group having a carbon number of not less than 10 or an organic group having at least one aliphatic hydrocarbon group having one or more branched chains and having a total carbon number of not less than 14 and not more than 300 can be mentioned. The pseudo-solid-phase protecting group imparts hydrophobicity to nucleic acid a, nucleic acid α, nucleic acid b, and step (1) and oligonucleotide having a phosphorothioated site obtained in step (2), and improves solubility in non-polar solvents. It can also decrease solubility in polar solvents. The nucleoside, nucleotide or oligonucleotide which is protected by such pseudo solid phase protecting group can perform a coupling reaction in the liquid phase of a non-polar solvent (step (1)), and can perform solid-liquid separation of the reaction solution obtained in step (2) by adding a polar solvent thereto to cause precipitation of the oligonucleotide having a phosphorothioated site and protected by the pseudo-solid-phase protecting group. Alternatively, a polar solvent is added to the reaction solution obtained in step (2), layers are separated between the polar solvent and non-polar solvent, and the oligonucleotide having a phosphorothioated site is transferred to the non-polar solvent, whereby the extraction thereof can be performed. As such pseudo solid phase protecting group, for example, those described in WO 2012/157723, WO 2013/122236, WO 2017/104836, WO 2013/179412, WO 2014/077292, WO 2017/086397 can be used.

The "protecting group used for nucleic acid synthesis" is not particularly limited as long as it is generally used in the field of nucleic acid synthesis, and includes the above-mentioned low-molecular-weight protecting groups. For purification by solid-liquid separation after step (2), the pseudo-solid-phase protecting group is preferably a protecting group having a linear aliphatic hydrocarbon group having a carbon number of not less than 10. For purification by extraction after step (2), the pseudo-solid-phase protecting group is preferably a protecting group having an organic group having at least one aliphatic hydrocarbon group having one or more branched chains and having a total carbon number of not less than 14 and not more than 300.

A pseudo-solid-phase protecting group preferable for solid-liquid separation is explained first. Examples of the pseudo-solid-phase protecting group preferable for solid-liquid separation include a protecting group having a C₆₋₁₄ hydrocarbon ring bonded, via a linker, to a hydrocarbon group wherein a linear aliphatic hydrocarbon group having a carbon number of not less than 10 is bonded via a single bond or a linker.

The aforementioned linear aliphatic hydrocarbon group having a carbon number of not less than 10 is preferably selected from a linear C₁₀₋₄₀ alkyl group and a linear C₁₀₋₄₀ alkenyl group, more preferably a linear C₁₀₋₄₀ alkyl group, further preferably a linear C₁₀₋₃₀ alkyl group, particularly preferably a linear C₁₂₋₂₈ alkyl group, most preferably a linear C₁₄₋₂₆ alkyl group.

The aforementioned linker is preferably selected from -O-, -C(=O)-, -C(=O)O-, -OC(=O)-, -C(=O)NH-, -NHC(=O)-, -S-, -SO-, - SO₂-, and -Si(R') (R")O-, -Si(R')(R")- (R', R" are each independently a hydrogen atom or a C₁₋₂₂ hydrocarbon group), more preferably selected from -O-, -C(=O)-, -C(=O)O-, -OC(=O)-, -C(=O)NH- and -NHC(=O)-, further preferably -O-.

The aforementioned C₆₋₁₄ hydrocarbon ring is preferably selected from a benzene ring, a naphthalene ring and a cyclohexane ring, more preferably selected from a benzene ring and a cyclohexane ring, further preferably a benzene ring.

A pseudo-solid-phase protecting group preferable for solid-liquid separation is preferably a protecting group having a benzene ring bonded, via -O-, to a hydrocarbon group wherein a linear C₁₀₋₄₀ alkyl group is bonded via a single bond or -O-.

A pseudo-solid-phase protecting group preferable for extraction is explained now. The "branched chain" of the "organic group having at least one aliphatic hydrocarbon group having one or more branched chains and having a total carbon number of not less than 14 and not more than 300" is a linear or branched chain saturated aliphatic hydrocarbon group, and is preferably a C₁₋₆ alkyl group, more preferably a C₁₋₄ alkyl group, further preferably a methyl group or an ethyl group. The "branched chain" is optionally substituted by one or more halogen atoms.

The "aliphatic hydrocarbon group" of the "organic group having at least one aliphatic hydrocarbon group having one or more branched chains and having a total carbon number of not less than 14 and not more than 300" is a linear saturated or unsaturated aliphatic hydrocarbon group, and is a C₂₋₃₀₀ alkyl group (preferably C₃₋₁₀₀ alkyl group, more preferably C₃₋₆₀ alkyl group), a C₂₋₃₀₀ alkenyl group (preferably C₃₋₁₀₀ alkenyl group, more preferably C₃₋₆₀ alkenyl group) or a C₂₋₃₀₀ alkynyl group (preferably C₃₋₁₀₀ alkynyl group, more preferably C₃₋₆₀ alkynyl group).

The position of the "aliphatic hydrocarbon group having one or more branched chains" of the "organic group having at least one aliphatic hydrocarbon group having one or more branched chains and having a total carbon number of not less than 14 and not more than 300" is not particularly limited, and may be present on the terminal (monovalent group) or a position other than the terminal (e.g., divalent group).

Examples of the "aliphatic hydrocarbon group having one or more branched chains" include branched isomers of propyl group, a butyl group, a pentyl group, a hexyl group, heptyl group, octyl group, nonyl group, decyl group, undecyl group, dodecyl group (lauryl group), tridecyl group, myristyl group, cetyl group, stearyl group, arachyl group, behenyl group, oleyl group, linolyl group, lignoceryl group and the like, and is a monovalent group having one or more branched chains and a divalent group induced therefrom. The "aliphatic hydrocarbon group having one or more branched chains" is preferably a 3,7,11-trimethyldodecyl group, a 3,7,11,15-tetramethylhexadecyl group (hereinafter sometimes to be also referred to as 2,3-dihydrophytyl group), a 2,2,4,8,10,10-hexamethylundecan-5-yl group or the like.

When the "organic group having at least one aliphatic hydrocarbon group having one or more branched chains and having a total carbon number of not less than 14 and not more than 300" contains a plurality of "aliphatic hydrocarbon groups having one or more branched chains", they may be the same or different.

The moiety other than the "aliphatic hydrocarbon group having one or more branched chains" in the "organic group having at least one aliphatic hydrocarbon group having one or more branched chains and having a total carbon number of not less than 14 and not more than 300" can be determined freely. For example, it optionally has a moiety such as -O-, -S-, -CO-, -NH-, -COO-, -OCONH-, -CONH-, -NHCO-, and a hydrocarbon group (monovalent group or divalent group) and the like. Examples of the "hydrocarbon group" include an aliphatic hydrocarbon group, an aromatic aliphatic hydrocarbon group, a monocyclic saturated hydrocarbon group, an aromatic hydrocarbon group and the like. Specifically, for example, monovalent groups such as alkyl group, alkenyl group, alkynyl group, cycloalkyl group, aryl group, aralkyl group and the like, and divalent groups derived therefrom are used. As the "alkyl group", a C₁₋₆ alkyl group is preferable, for example, methyl, ethyl, propyl, isopropyl, butyl, isobutyl, sec-butyl, tert-butyl, pentyl, hexyl and the like can be mentioned. As the "alkenyl group", a C₂₋₆ alkenyl group is preferable, for example, vinyl, 1-propenyl, allyl, isopropenyl, butenyl, isobutenyl and the like can be mentioned. As the "alkynyl group", a C₂₋₆ alkynyl group is preferable, for example, ethynyl, propargyl, 1-propynyl and the like can be mentioned. As the "cycloalkyl group", a C₃₋₆ cycloalkyl group is preferable, for example, cyclopropyl, cyclobutyl, cyclopentyl, cyclohexyl can be mentioned. As the "aryl group", a C₆₋₁₄ aryl group is preferable, for example, phenyl, 1-naphthyl, 2-naphthyl, biphenylyl, 2-anthryl and the like can be mentioned. Of these, a C₆₋₁₀ aryl group is more preferable, and phenyl is particularly preferable. As the "aralkyl group", a C₇₋₂₀ aralkyl group is preferable, for example, benzyl, 1-phenylethyl, 2-phenylethyl, 1-phenylpropyl, naphthylmethyl, 1-naphthylethyl, 1-naphthylpropyl and the like can be mentioned. Of these, a C₇₋₁₆ aralkyl group (C₆₋₁₀ aryl-C₁₋₆ alkyl group) is more preferable, and benzyl is particularly preferable. The "hydrocarbon group" is optionally substituted by a substituent selected from a halogen atom (chlorine atom, bromine atom, fluorine atom, iodine atom), an oxo group and the like.

The "total carbon number" in the "organic group having at least one aliphatic hydrocarbon group having one or more branched chains and having a total carbon number of not less than 14 and not more than 300" is not less than 14, preferably not less than 16, more preferably not less than 18, and not more than 300, preferably not more than 200, more preferably not more than 160. The number of the branched chain in the "organic group having at least one aliphatic hydrocarbon group having one or more branched chains and having a total carbon number of not less than 14 and not more than 300" is not particularly limited, and not less than 2 is preferable, not less than 3 is more preferable, not less than 4 is more preferable, not less than 8 is more preferable, not less than 10 is further preferable. When the number of the branched chain is higher, nucleoside or oligonucleotide protected by a pseudo-solid-phase protecting group having an organic group having at least one aliphatic hydrocarbon group having one or more branched chains and having a total carbon number of not less than 14 and not more than 300 is dissolved well in an organic solvent (particularly, non-polar solvent) even when the oligonucleotide chain is long.

As the "organic group having at least one aliphatic hydrocarbon group having one or more branched chains and having a total carbon number of not less than 14 and not more than 300", a group having the same or different divalent group represented by the formula (A):

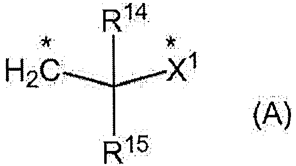

wherein
* is the bonding position to the adjacent atom;
R¹⁴ and R¹⁵ are each independently a hydrogen atom or a C₁₋₄ alkyl group; and
X¹ is a single bond or a C₁₋₄ alkylene group, provided that
R¹⁴ and R¹⁵ are not hydrogen atoms at the same time, is preferable.

Examples of the group having the divalent group represented by the formula (A) include a group represented by any of the following formulas (B) - (D). In the definition of each symbol in the formulas (B) - (D), the carbon number, number of repeat units (m₁, n₀ - n₂) and the like are shown for convenience, and can be appropriately changed within the range of the above-mentioned definitions so that the total number can be not less than 14 (preferably not less than 16, more preferably not less than 18) and not more than 300 (preferably not more than 200, more preferably not more than 160). The formulas (B) - (D) are explained in order below.

The formula (B) is as described below.

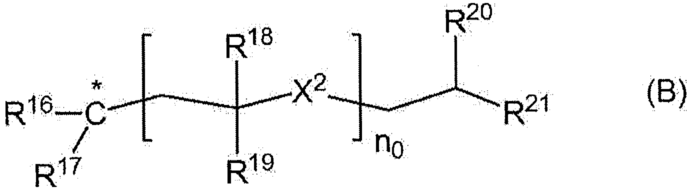

wherein
* is the bonding position to the adjacent atom;
R¹⁶ and R¹⁷ are hydrogen atoms or joined to show =O;
n₀ is an integer of 2 - 40;
R¹⁸ and R¹⁹ in the number of n₀ are each independently a hydrogen atom or a C₁₋₄ alkyl group;
X² in the number of n₀ are each independently a single bond or a C₁₋₄ alkylene group;
R²⁰ is a hydrogen atom or a C₁₋₄ alkyl group; and
R²¹ is a C₁₋₄ alkyl group,
provided that R¹⁸ and R¹⁹ are not hydrogen atoms at the same time, and when n₀ is 2, R²⁰ is a C₁₋₄ alkyl group.

As the group of the formula (B), a group wherein
R¹⁶ and R¹⁷ are each a hydrogen atom;
n₀ is an integer of 2 - 40;
R¹⁸ and R¹⁹ in the number of n₀ are each independently a hydrogen atom, a methyl group or an ethyl group;
X² in the number of n₀ are each independently a single bond, a methylene group or an ethylene group; and
R²⁰ is a hydrogen atom, a methyl group or an ethyl group (provided that R¹⁸ and R¹⁹ are not hydrogen atoms at the same time, and when n₀ is 2, R²⁰ is methyl or an ethyl group) is preferable.

The group of the formula (B) is more preferably a branched isomer having a carbon number of 14 - 160 of myristyl group, cetyl group, stearyl group, arachyl group, behenyl group or the like, of which a 2,3-dihydrophytyl group, a 3,7,11-trimethyldodecyl group, and a 2,2,4,8,10,10-hexamethyl-5-dodecanoyl group are particularly preferable.

The formula (C) is as described below.

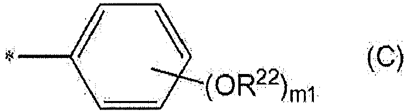

wherein
* is the bonding position to the adjacent atom;
OR²² in the number of m₁ are each independently a hydroxy group substituted by a group represented by the formula (B); and
m₁ is an integer of 1 - 3.]

The "group represented by the formula (B)" in the formula (C) is as described above except that * therein is the bonding position to O (i.e., adjacent atom).

In the group of the formula (C), R²² is more preferably a branched isomer group having a carbon number of 14 - 30 of myristyl group, cetyl group, stearyl group, arachyl group, behenyl group or the like, of which a 2,3-dihydrophytyl group, a 3,7,11-trimethyldodecyl group are particularly preferable.

The formula (D) is as described below.

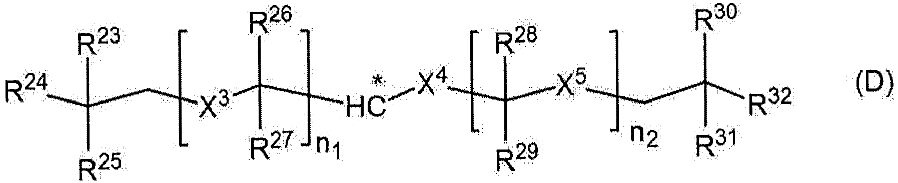

wherein
* is the bonding position to Q;
n₁ is an integer of 1 - 10;
n₂ is an integer of 1 - 10;
R²⁶ and R²⁷ in the number of n₁ are each independently a hydrogen atom or a C₁₋₄ alkyl group;
X³ in the number of n₁ are each independently a single bond or a C₁-₄ alkylene group;
R²⁸ and R²⁹ in the number of n₂ are each independently a hydrogen atom or a C₁₋₄ alkyl group;
X⁵ in the number of n₂ are each independently a single bond or a C₁₋₄ alkylene group;
X⁴ is a single bond or a C₁₋₄ alkylene group; and
R²³, R²⁴, R²⁵, R³⁰, R³¹ and R³² are each independently a hydrogen atom or a C₁₋₄ alkyl group,
provided that R²⁶ and R²⁷, and/or R²⁸ and R²⁹ are not hydrogen atoms at the same time, and when n₁+n₂ is 2, two or more of R²³, R²⁴ and R²⁵ are each independently a C₁₋₄ alkyl group, or two or more of R³⁰, R³¹ and R³² are each independently a C₁₋₄ alkyl group.

As the group of the formula (D), a group wherein
n₁ is an integer of 1 - 5;
n₂ is an integer of 1 - 5;
R²⁶ and R²⁷ in the number of n₁ are each independently a hydrogen atom, a methyl group or an ethyl group;
X³ in the number of n₁ are each independently a single bond, a methylene group or an ethylene group;
R²⁸ and R²⁹ in the number of n₂ are each independently a hydrogen atom, a methyl group or an ethyl group;
X⁵ in the number of n₂ are each independently a single bond, a methylene group or an ethylene group;
X⁴ is a single bond, a methylene group or an ethylene group; and
R²³, R²⁴, R²⁵, R³⁰, R³¹ and R³² are each independently a hydrogen atom or a C₁₋₄ alkyl group,
provided that R²⁶ and R²⁷, and/or R²⁸ and R²⁹ are not hydrogen atoms at the same time, and when n₁+n₂ is 2, two or more of R²³, R²⁴ and R²⁵ are each independently a C₁₋₄ alkyl group, or two or more of R³⁰, R³¹ and R³² are each independently a C₁₋₄ alkyl group is more preferable.

As a particularly preferable group of the formula (D), a group wherein
n₁ is an integer of 1 - 5;
n₂ is an integer of 1 - 5;
R²⁶ and R²⁷ in the number of n₁ are each independently a hydrogen atom or a methyl group;
X³ in the number of n₁ are each independently a single bond or a methylene group;
R²⁸ and R²⁹ in the number of n₂ are each independently a hydrogen atom or a methyl group;
X⁵ in the number of n₂ are each independently a single bond or a methylene group;
X⁴ is a single bond or a methylene group; and
R²³, R²⁴, R²⁵, R³¹, R³¹ and R³² are methyl groups,
provided that R²⁶ and R²⁷, and/or R²⁸ and R²⁹ are not hydrogen atoms at the same time
can be mentioned.

Specific examples of the "organic group having at least one aliphatic hydrocarbon group having one or more branched chains and having a total carbon number of not less than 14 and not more than 300" include the following groups. In each group, * shows a bonding position; in the formula, n₃ is an integer of not less than 3; and n₄ is appropriately determined such that the total carbon number of the groups is not less than 14 and not more than 300.

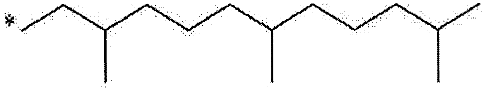

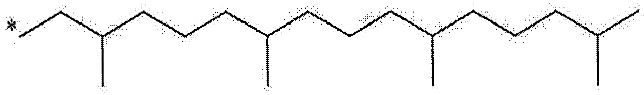

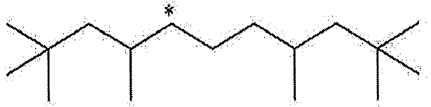

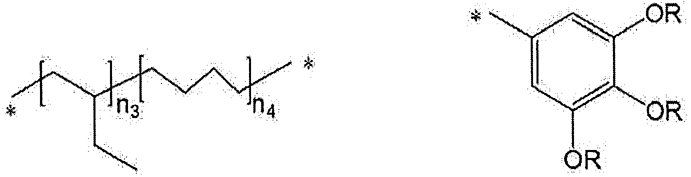

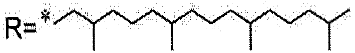

Specific preferable examples of the "organic group having at least one aliphatic hydrocarbon group having one or more branched chains and having a total carbon number of not less than 14 and not more than 300" include the following groups:
3,7,11,15-tetramethylhexadecyl group (alias: 2,3-dihydrophytyl group);
3,7,11-trimethyldodecyl group;
2,2,4,8,10,10-hexamethyl-5-dodecanoyl group;
3,4,5-tri(3',7',11',15'-tetramethylhexadecyloxy)benzyl group; and
3,5-di(3',7',11',15'-tetramethylhexadecyloxy)benzyl group.

The pseudo-solid-phase protecting group is more preferably a group represented by the following formula (g-I) (hereinafter sometimes to be abbreviated as "pseudo-solid-phase protecting group (g-I)").
**L-Y-Z (g-I)
wherein
** is the bonding position to the protected group;
L is a single bond, or a group represented by the formula (a1) or (a1'):

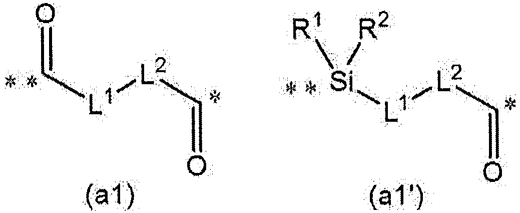

wherein
* is the bonding position to Y;
** is as defined above;
R¹ and R² are each independently a C₁₋₂₂ hydrocarbon group;
L¹ is an optionally substituted divalent C₁₋₂₂ hydrocarbon group;
L² is a single bond or a group represented by ***C(=O)O-R⁴-N(R⁵)**** or ***C(=O)N(R³)-R⁴-N(R⁵)**** wherein *** is the bonding position to L¹, **** is the bonding position to C=O, R₄ is a C₁₋₂₂ alkylene group, R³ and R⁵ are each independently a hydrogen atom or a C₁₋₂₂ alkyl group, or R³ and R⁵ are optionally joined to form a ring;
Y is a single bond, an oxygen atom or NR (wherein R is a hydrogen atom, an alkyl group or an aralkyl group); and
Z is a group represented by the formula (a2), the formula (a2') or the formula (a2"):

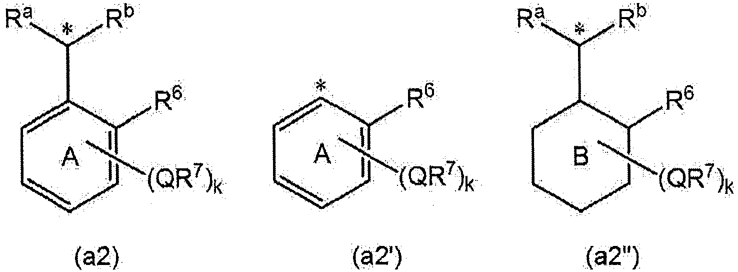

wherein
* indicates a bonding position;
R⁶ is a hydrogen atom, or when R^{b} is a group represented by the following formula (a3), R⁶ of ring A or ring B is optionally shows, together with R⁸, a single bond or -O- to form, together with ring A or ring B and ring C, a fused ring;
k is an integer of 1 - 4;
Q in the number of k are each independently -O-, -C(=O)-, -C(=O)O-, -OC(=O)-, -NR⁷-, -C(=O)NH- or -NHC(=O)-;
R⁷ in the number of k are each independently a hydrocarbon group wherein a linear aliphatic hydrocarbon group having a carbon number of not less than 10 is bonded via a single bond or a linker, or an organic group having at least one aliphatic hydrocarbon group having one or more branched chains and having a total carbon number of not less than 14 and not more than 300;
ring A and ring B, each independently, optionally has, in addition to QR⁷ in the number of k, a substituent selected from the group consisting of a halogen atom, a C₁₋₆ alkyl group optionally substituted by a halogen atom, and a C₁₋₆ alkoxy group optionally substituted by a halogen atom;
Rₐ is a hydrogen atom; and
R_{b} is a hydrogen atom, or a group represented by the formula (a3):

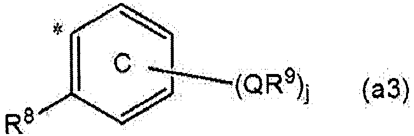

wherein
* indicates a bonding position;
j is an integer of 0 to 4;
Q in the number of j are each independently as defined above;
R⁹ in the number of j are each independently a hydrocarbon group wherein a linear aliphatic hydrocarbon group having a carbon number of not less than 10 is bonded via a single bond or a linker, or an organic group having at least one aliphatic hydrocarbon group having one or more branched chains and having a total carbon number of not less than 14 and not more than 300;
R⁸ is a hydrogen atom, or optionally shows, together with R⁶ of ring A or ring B, a single bond or -O- to form, together with ring A or ring B and ring C, a fused ring; and
ring C optionally has, in addition to QR⁹ in the number of j, a substituent selected from the group consisting of a halogen atom, a C₁₋₆ alkyl group optionally substituted by a halogen atom, and a C₁₋₆ alkoxy group optionally substituted by a halogen atom, or
R^{a} and R^{b} are joined to form an oxo group.

The linear aliphatic hydrocarbon groups having a carbon number of not less than 10, that R⁷ in the formula (a2), the formula (a2') and the formula (a2"), and R⁹ in the formula (a3) have, are each independently preferably selected from linear C₁₀-₄₀ alkyl group and linear C₁₀₋₄₀ alkenyl group, more preferably linear C₁₀₋₄₀ alkyl group, further preferably linear C₁₀-₃₀ alkyl group, particularly preferably linear C₁₂-₂₈ alkyl group, most preferably linear C₁₄-₂₆ alkyl group. The linkers that R⁷ in the formula (a2), the formula (a2') and the formula (a2"), and R⁹ in the formula (a3) have are each independently preferably -O-, -C(=O)-, -C(=O)O-,-OC(=O)-, -C(=O)NH- or -NHC(=O)-, more preferably -O-.

The "hydrocarbon group wherein a linear aliphatic hydrocarbon group having a carbon number of not less than 10 is bonded via a single bond or a linker" for R⁷ in the formula (a2), the formula (a2') and the formula (a2"), and R⁹ in the formula (a3) is preferably a linear C₁₀₋₄₀ alkyl group, a benzyl group to which 1 to 3 linear C₁₀₋₄₀ alkyl groups are bonded via -O-, or a cyclohexylmethyl group to which 1 to 3 linear C₁₀₋₄₀ alkyl groups are bonded via -O-.

The "organic groups having at least one aliphatic hydrocarbon groups having one or more branched chains and having a total carbon number of not less than 14 and not more than 300", each which is one embodiment of R⁷ in the formula (a2), the formula (a2') and the formula (a2"), and R⁹ in the formula (a3), are each independently preferably a group having a divalent group represented by the above-mentioned formula (A), more preferably a group represented by any of the above-mentioned formulas (B) - (D), further preferably a group represented by the above-mentioned formula (B), particularly preferably a 2,3-dihydrophytyl group, a 3,7,11-trimethyldodecyl group, or a 2,2,4,8,10,10-hexamethyl-5-dodecanoyl group.

Q in the formula (a2), the formula (a2'), the formula (a2") and the formula (a3) is preferably -O-, -C(=O)NH- or-NHC(=O)-, more preferably -O-.

In the formula (g-I), a preferred embodiment of L represented by the formula (a1) is a group wherein
L¹ is a divalent C₁₋₂₂ hydrocarbon group (wherein one or two or more -CH₂- constituting the hydrocarbon group may be substituted by -O-, -C(=O)-, etc.) or CH₂-O-1,4-phenylene-O-CH₂; and
L² is a single bond or a group represented by ***C(=O)N(R³)-R⁴-N(R⁵)**** wherein *** is the bonding position to L¹, **** is the bonding position to C=O, R⁴ is a C₁₋₆ alkylene group, R³ and R⁵ are each independently a hydrogen atom or an optionally substituted C₁₋₆ alkyl group, or R³ and R⁵ are optionally joined to form an optionally substituted C₁₋₆ alkylene group.

Another preferred embodiment of L represented by the formula (a1) is a group wherein
L¹ is a divalent C₁₋₂₂ hydrocarbon group; and
L² is a single bond.

Another preferred embodiment of L represented by the formula (a1) is a group wherein
L¹ is an ethylene group; and
L² is a group represented by ***C(=O)N(R³)-R⁴-N(R⁵)**** wherein *** is the bonding position to L¹, **** is the bonding position to C=O, R⁴ is a C₁₋₂₂ alkylene group, R³ and R⁵ are each independently a hydrogen atom or a C₁₋₂₂ alkyl group, or R³ and R⁵ are optionally joined to form a ring.

Another preferred embodiment of L represented by the formula (a1) is a group wherein
L¹ is an ethylene group; and
L² is a group represented by ***C(=O)N(R³)-R⁴-N(R⁵)**** wherein *** is the bonding position to L¹, **** is the bonding position to C=O, and N(R³)-R⁴-N(R⁵) moiety forms a 1,4-piperazinediyl group.

Another preferred embodiment of L represented by the formula (a1) is a group wherein
L¹ is an ethylene group; and
L² is a group represented by ***C(=O)N(R³)-R⁴-N(R⁵)**** wherein *** is the bonding position to L¹, **** is the bonding position to C=O, R⁴ is a pentylene group or a hexylene group, and R³ and R⁵ are each independently a hydrogen atom or a methyl group.

Particularly preferred embodiment of L represented by the formula (a1) is a succinyl group which is easily available and economical.

In the formula (g-I), L represented by the formula (a1') is explained below.
L¹ in the formula (a1') is preferably a divalent C₆₋₁₀ aromatic hydrocarbon group, more preferably a phenylene group.
L² in the formula (a1') is preferably a single bond.
A preferable combination of L¹ and L² in the formula (a1') is a combination of a divalent C₆₋₁₀ aromatic hydrocarbon group for L¹ and a single bond for L². A more preferable combination of L¹ and L² in the formula (a1') is a combination of a phenylene group for L¹ and a single bond for L².
R¹ and R² in the formula (a1') are each independently preferably a C₁₋₂₂ alkyl group, more preferably a C₁₋₁₀ alkyl group.

A preferred embodiment of L represented by the formula (a1') is a group wherein
R¹ and R² are each independently a C₁₋₂₂ alkyl group;
L¹ is a divalent C₆₋₁₀ aromatic hydrocarbon group; and
L² is a single bond.

Another preferred embodiment of L represented by the formula (a1') is a group wherein
R¹ and R² are each independently a C₁₋₁₀ alkyl group;
L¹ is a phenylene group; and
L² is a single bond.

When Y in the formula (g-I) is NR, the aforementioned R is preferably a hydrogen atom, a C₁₋₆ alkyl group or a C₇₋₁₆ aralkyl group, more preferably a hydrogen atom, methyl, ethyl or benzyl, further preferably a hydrogen atom. Y is preferably a single bond, an oxygen atom or NR, more preferably a single bond or an oxygen atom.

Z in the formula (g-I) is preferably a group represented by the formula (a2) or the formula (a2"), more preferably a group represented by the formula (a2"). Using a pseudo-solid-phase protecting group having Z represented by the formula (a2") (i.e., structure of cyclohexylmethyl group), the solubility of the nucleoside, nucleotide or oligonucleotide (a) and the like in non-polar solvents can be strikingly improved as compared with a pseudo-solid-phase protecting group having Z represented by the formula (a2) (i.e., structure of benzyl group). As a result, the production method of the present invention can be performed at a higher concentration and productivity is strikingly improved.

In the formula (a2), R⁶ is preferably a hydrogen atom. In the formula (a2), R^{a} and R^{b} are each preferably a hydrogen atom, or are joined to form an oxo group.

An embodiment preferable for solid-liquid separation of Z represented by the formula (a2) is a group wherein
R^{a} and R^{b} are hydrogen atoms;
R⁶ is a hydrogen atom;
k is an integer of 1 - 3;
Q in the number of k are -O-; and
R⁷ in the number of k are each independently a linear C₁₀₋₄₀ alkyl group.

Another embodiment preferable for solid-liquid separation of Z represented by the formula (a2) is a group wherein
R^{a} and R^{b} are hydrogen atoms;
R⁶ is a hydrogen atom;
k is an integer of 1 - 3;
Q in the number of k are -O-; and
R⁷ in the number of k are each independently a benzyl group to which 1 to 3 linear C₁₀₋₄₀ alkyl groups are bonded via -O-, or a cyclohexylmethyl group to which 1 to 3 linear C₁₀₋₄₀ alkyl groups are bonded via -O-; and
ring A optionally has, in addition to QR⁷ in the number of k, a substituent selected from the group consisting of a halogen atom, a C₁₋₆ alkyl group optionally substituted by a halogen atom, and a C₁₋₆ alkoxy group optionally substituted by a halogen atom.

Another embodiment preferable for solid-liquid separation of Z represented by the formula (a2) is a group wherein
R^{a} is a hydrogen atom; and
R^{b} is a group represented by the formula (a3) wherein * shows the bonding position, j is an integer of 0 - 3, Q in the number of j are -O-, R⁹ in the number of j are each independently a linear C₁₀₋₄₀ alkyl group, R⁶ and R⁸ are each a hydrogen atom.

Another embodiment preferable for solid-liquid separation of Z represented by the formula (a2) is a group wherein
R^{a} is a hydrogen atom; and
R^{b} is a group represented by the formula (a3) wherein * shows the bonding position, j is an integer of 0 - 3, Q in the number of j are -O-, R⁹ in the number of j are each independently a linear C₁₀₋₄₀ alkyl group, R⁸ shows, together with R⁶, a single bond or -O- to form, together with ring A and ring C, a fused ring.

Another embodiment preferable for solid-liquid separation of Z represented by the formula (a2) is a group wherein
R^{a} and R^{b} are joined to form an oxo group;
R⁶ is a hydrogen atom;
k is an integer of 1 - 3;
Q in the number of k are -O-; and
R⁷ in the number of k are each independently a linear C₁₀₋₄₀ alkyl group.

Another embodiment preferable for solid-liquid separation of Z represented by the formula (a2) is a group wherein
R^{a} and R^{b} are joined to form an oxo group;
R⁶ is a hydrogen atom;
k is an integer of 1 - 3;
Q in the number of k are -O-; and
R⁷ in the number of k are each independently a benzyl group to which 1 to 3 linear C₁₀₋₄₀ alkyl groups are bonded via -O-, or a cyclohexylmethyl group to which 1 to 3 linear C₁₀₋₄₀ alkyl groups are bonded via -O-; and
ring A optionally has, in addition to QR⁷ in the number of k, a substituent selected from the group consisting of a halogen atom, a C₁₋₆ alkyl group optionally substituted by a halogen atom, and a C₁₋₆ alkoxy group optionally substituted by a halogen atom.

In the formula (a2"), R⁶ is preferably a hydrogen atom. In the formula (a2"), R^{a} and R^{b} are each preferably a hydrogen atom, or are joined to form an oxo group.

A embodiment preferable for solid-liquid separation of Z represented by the formula (a2") is a group wherein
R^{a} and R^{b} are hydrogen atoms;
R⁶ is a hydrogen atom;
k is an integer of 1 - 3;
Q in the number of k are -O-; and
R⁷ in the number of k are each independently a linear C₁₀₋₄₀ alkyl group.

Another embodiment preferable for solid-liquid separation of Z represented by the formula (a2") is a group wherein
R^{a} and R^{b} is a hydrogen atom;
R⁶ is a hydrogen atom;
k is an integer of 1 - 3;
Q in the number of k are -O-;
R⁷ in the number of k are each independently a benzyl group to which 1 to 3 linear C₁₀₋₄₀ alkyl groups are bonded via -O-, or a cyclohexylmethyl group to which 1 to 3 linear C₁₀₋₄₀ alkyl groups are bonded via -O-; and
ring B optionally has, in addition to QR⁷ in the number of k, a substituent selected from the group consisting of a halogen atom, a C₁₋₆ alkyl group optionally substituted by a halogen atom, and a C₁₋₆ alkoxy group optionally substituted by a halogen atom.

Another embodiment preferable for solid-liquid separation of Z represented by the formula (a2") is a group wherein
R^{a} is a hydrogen atom;
R^{b} is a group represented by the formula (a3) wherein * shows the bonding position, j is an integer of 0 - 3, Q in the number of j are -O-, R⁹ in the number of j is are each independently a C₁₀₋₄₀ alkyl group, and R⁶ and R⁸ are hydrogen atoms.

Another embodiment preferable for solid-liquid separation of Z represented by the formula (a2") is a group wherein
R^{a} is a hydrogen atom;
R^{b} is a group represented by the formula (a3) wherein * shows the bonding position, j is an integer of 0 - 3, Q in the number of j is -O-, R⁹ in the number of j are each independently a linear C₁₀₋₄₀ alkyl group, R⁸ shows, together with R⁶, a single bond or -O- to form, together with ring B and ring C, a fused ring.

Another embodiment preferable for solid-liquid separation of Z represented by the formula (a2") is a group wherein
R^{a} and R^{b} are joined to form an oxo group;
R⁶ is a hydrogen atom;
k is an integer of 1 - 3;
Q in the number of k are -O-; and
R⁷ in the number of k are each independently a linear C₁₀₋₄₀ alkyl group.

Another embodiment preferable for solid-liquid separation of Z represented by the formula (a2") is a group wherein
R^{a} and R^{b} are joined to form an oxo group;
R⁶ is a hydrogen atom;
k is an integer of 1 - 3;
Q in the number of k are -O-;
R⁷ in the number of k are each independently a benzyl group to which 1 to 3 linear C₁₀₋₄₀ alkyl groups are bonded via -O-, or a cyclohexylmethyl group to which 1 to 3 linear C₁₀₋₄₀ alkyl groups are bonded via -O-; and
ring B optionally has, in addition to QR⁷ in the number of k, a substituent selected from the group consisting of a halogen atom, a C₁₋₆ alkyl group optionally substituted by a halogen atom, and a C₁₋₆ alkoxy group optionally substituted by a halogen atom.

Pseudo-solid-phase protecting group (g-I) preferable for solid-liquid separation is preferably a group wherein
L is a succinyl group, or a group represented by the formula (a1') (in the formula (a1'), R¹ and R² are each independently a C₁₋₁₀ alkyl group, L¹ is a divalent phenylene group, L² is a single bond), and
Y-Z is a 3,4,5-tris(octadecyloxy)benzyloxy group, a 3,5-bis(docosyloxy)benzyloxy group, a 3,5-bis[3',4',5'-tris(octadecyloxy)benzyloxy]benzyloxy group, a 3,4,5-tris[3',4',5'-tris(octadecyloxy)benzyloxy]benzyloxy group, a 3,4,5-tris(octadecyloxy)benzylamino group, a 2,4-bis(docosyloxy)benzylamino group, 3,5-bis(docosyloxy)benzylamino group, a bis(4-docosyloxyphenyl)methylamino group, a 4-methoxy-2-[3',4',5'-tris(octadecyloxy)benzyloxy]benzylamino group, a 4-methoxy-2-[3',4',5'-tris(octadecyloxy)cyclohexylmethyloxy]benzylamino group, a 2,4-bis(dodecyloxy)benzylamino group, a phenyl(2,3,4-tris(octadecyloxy)phenyl)methylamino group, a bis[4-(12-docosyloxydodecyloxy)phenyl]methylamino group, a 3,5-bis[3',4',5'-tris(octadecyloxy)benzyloxy]benzylamino group, a 3,4,5-tris[3',4',5'-tris(octadecyloxy)benzyloxy]benzylamino group, a 3,4,5-tris(octadecyloxy)cyclohexylmethyloxy group, a 3,5-bis(docosyloxy)cyclohexylmethyloxy group, a 3,5-bis[3',4',5'-tris(octadecyloxy)cyclohexylmethyloxy]cyclohexylmethyloxy group, a 3,4,5-tris[3',4',5'-tris(octadecyloxy)cyclohexylmethyloxy]cyclohexylmethyloxy group, a 3,4,5-tris(octadecyloxy)cyclohexylmethylamino group, a 2,4-bis(docosyloxy)cyclohexylmethylamino group, a 3,5-bis(docosyloxy)cyclohexylmethylamino group, a bis(4-docosyloxycyclohexyl)methylamino group, a 4-methoxy-2-[3',4',5'-tris(octadecyloxy)cyclohexylmethyloxy]cyclohexylmethylamino group, a 4-methoxy-2-[3',4',5'-tris(octadecyloxy)cyclohexylmethyloxy]cyclohexylmethylamino group, a 2,4-bis(dodecyloxy)cyclohexylmethylamino group, phenyl(2,3,4-tris(octadecyloxy)cyclohexyl)methylamino group, a bis[4-(12-docosyloxydodecyloxy)cyclohexyl]methylamino group, a 3,5-bis[3',4',5'-tris(octadecyloxy)cyclohexylmethyloxy]cyclohexylmethylamino group or a 3,4,5-tris[3',4',5'-tris(octadecyloxy)cyclohexylmethyloxy]cyclohexylmethylamino group, or a group wherein
L-Y is a single bond or a succinyl-1,4-piperazinediyl group, and
Z is a 3,4,5-tris(octadecyloxy)benzoyl group, a 3,5-bis(docosyloxy)benzoyl group, a 3,5-bis[3',4',5'-tris(octadecyloxy)benzyloxy]benzoyl group or a 3,4,5-tris[3',4',5'-tris(octadecyloxy)benzyloxy]benzoyl group.

Pseudo-solid-phase protecting group (g-I) preferable for solid-liquid separation is more preferably a group wherein
L is a succinyl group, and
Y-Z is a 3,4,5-tris(octadecyloxy)benzyloxy group, a 3,5-bis(docosyloxy)benzyloxy group, a 3,5-bis[3',4',5'-tris(octadecyloxy)benzyloxy]benzyloxy group, a 3,4,5-tris[3',4',5'-tris(octadecyloxy)benzyloxy]benzyloxy group, a 3,4,5-tris(octadecyloxy)benzylamino group, a 2,4-bis(docosyloxy)benzylamino group, a 3,5-bis(docosyloxy)benzylamino group, a bis(4-docosyloxyphenyl)methylamino group, a 4-methoxy-2-[3',4',5'-tris(octadecyloxy)benzyloxy]benzylamino group, a 4-methoxy-2-[3',4',5'-tris(octadecyloxy)cyclohexylmethyloxy]benzylamino group, a 2,4-bis(dodecyloxy)benzylamino group, a phenyl(2,3,4-tris(octadecyloxy)phenyl)methylamino group, a bis[4-(12-docosyloxydodecyloxy)phenyl]methylamino group, a 3,5-bis[3',4',5'-tris(octadecyloxy)benzyloxy]benzylamino group, a 3,4,5-tris[3',4',5'-tris(octadecyloxy)benzyloxy]benzylamino group, a 3,4,5-tris(octadecyloxy)cyclohexylmethyloxy group, a 3,5-bis(docosyloxy)cyclohexylmethyloxy group, a 3,5-bis[3',4',5'-tris(octadecyloxy)cyclohexylmethyloxy]cyclohexylmethyloxy group, a 3,4,5-tris[3',4',5'-tris(octadecyloxy)cyclohexylmethyloxy]cyclohexylmethyloxy group, a 3,4,5-tris(octadecyloxy)cyclohexylmethylamino group, a 2,4-bis(docosyloxy)cyclohexylmethylamino group, a 3,5-bis(docosyloxy)cyclohexylmethylamino group, a bis(4-docosyloxycyclohexyl)methylamino group, a 4-methoxy-2-[3',4',5'-tris(octadecyloxy)cyclohexylmethyloxy]cyclohexylmethylamino group, a 4-methoxy-2-[3',4',5'-tris(octadecyloxy)cyclohexylmethyloxy]cyclohexylmethylamino group, a 2,4-bis(dodecyloxy)cyclohexylmethylamino group, phenyl(2,3,4-tris(octadecyloxy)cyclohexyl)methylamino group, a bis[4-(12-docosyloxydodecyloxy)cyclohexyl]methylamino group, a 3,5-bis[3',4',5'-tris(octadecyloxy)cyclohexylmethyloxy]cyclohexylmethylamino group or a 3,4,5-tris[3',4',5'-tris(octadecyloxy)cyclohexylmethyloxy]cyclohexylmethylamino group, or
a group wherein
   L-Y is a single bond or a succinyl-1,4-piperazinediyl group, and
   Z is a 3,4,5-tris(octadecyloxy)benzoyl group, a 3,5-bis(docosyloxy)benzoyl group, a 3,5-bis[3',4',5'-tris(octadecyloxy)benzyloxy]benzoyl group or a 3,4,5-tris[3',4',5'-tris(octadecyloxy)benzyloxy]benzoyl group.

Pseudo-solid-phase protecting group (g-I) preferable for solid-liquid separation is further preferably a group wherein
L is a succinyl group, and
Y-Z is a 3,4,5-tris(octadecyloxy)benzyloxy group, a 3,4,5-tris(octadecyloxy)cyclohexylmethyloxy group, a 3,5-bis(docosyloxy)cyclohexylmethyloxy group, a 3,5-bis[3',4',5'-tris(octadecyloxy)cyclohexylmethyloxy]cyclohexylmethyloxy group, a 3,4,5-tris[3',4',5'-tris(octadecyloxy)cyclohexylmethyloxy]cyclohexylmethyloxy group, a 3,4,5-tris(octadecyloxy)cyclohexylmethylamino group, a 2,4-bis(docosyloxy)cyclohexylmethylamino group, a 3,5-bis(docosyloxy)cyclohexylmethylamino group, a 4-methoxy-2-[3',4',5'-tris(octadecyloxy)cyclohexylmethyloxy]cyclohexylmethylamino group, a 4-methoxy-2-[3',4',5'-tris(octadecyloxy)cyclohexylmethyloxy]cyclohexylmethylamino group, a 2,4-bis(dodecyloxy)cyclohexylmethylamino group, a 3,5-bis[3',4',5'-tris(octadecyloxy)cyclohexylmethyloxy]cyclohexylmethylamino group or a 3,4,5-tris[3',4',5'-tris(octadecyloxy)cyclohexylmethyloxy]cyclohexylmethylamino group, or
   a group wherein
L-Y is a single bond or a succinyl-1,4-piperazinediyl group, and
Z is a 3,4,5-tris(octadecyloxy)benzoyl group.

Pseudo-solid-phase protecting group (g-I) preferable for solid-liquid separation is particularly preferably a group wherein
L is a succinyl group, and
Y-Z is a 3,4,5-tris(octadecyloxy)benzyloxy group, a 3,4,5-tris(octadecyloxy)cyclohexylmethyloxy group or a phenyl(2,3,4-tris(octadecyloxy)phenyl)methylamino group, or a group wherein
   L-Y is a succinyl-1,4-piperazinediyl group, and
   Z is a 3,4,5-tris(octadecyloxy)benzoyl group.

Pseudo-solid-phase protecting group (g-I) preferable for solid-liquid separation is most preferably a group wherein
L is a succinyl group, and
Y-Z is a 3,4,5-tris(octadecyloxy)benzyloxy group or a 3,4,5-tris(octadecyloxy)cyclohexylmethyloxy group, or a group wherein
L-Y is a succinyl-1,4-piperazinediyl group, and
Z is a 3,4,5-tris(octadecyloxy)benzoyl group.

Pseudo-solid-phase protecting group (g-I) preferable for extraction is preferably a 2-{2,4-di(2',3'-dihydrophytyloxy)benzylaminocarbonyl}ethylcarbonyl group; a 3,5-di(2',3'-dihydrophytyloxy)benzyloxysuccinyl group; a 4-(2',3'-dihydrophytyloxy)benzyloxysuccinyl group; a 2-{1-[(2-chloro-5-(2',3'-dihydrophytyloxy)phenyl)]benzylaminocarbonyl}ethylcarbonyl group; a 3,4,5-tri(2',3'-dihydrophytyloxy)benzyloxysuccinyl group; a 2-{3,4,5-tri(2',3'-dihydrophytyloxy)benzylaminocarbonyl}ethylcarbonyl group; a 2-{4-(2',3'-dihydrophytyloxy)benzylaminocarbonyl}ethylcarbonyl group; a 2-{2-[3',4',5'-tri(2",3"-dihydrophytyloxy)benzyloxy]-4-methoxybenzylaminocarbonyl}ethylcarbonyl group; a 2-{4-(2',3'-dihydrophytyloxy)-2-methoxybenzylaminocarbonyl}ethylcarbonyl group; a 4-(2',3'-dihydrophytyloxy)-2-methylbenzyloxysuccinyl group; a 2-{4-(2',3'-dihydrophytyloxy)-2-methylbenzylaminocarbonyl}ethylcarbonyl group; a 4-[2,2,4,8,10,10-hexamethyl-5-dodecanoylamino]benzyloxysuccinyl group; a 2-{4-[2,2,4,8,10,10-hexamethyl-5-dodecanoylamino]benzylaminocarbonyl}ethylcarbonyl group; a 4-(3,7,11-trimethyldodecyloxy)benzyloxysuccinyl group; a 2-{4-(3,7,11-trimethyldodecyloxy)benzylaminocarbonyl}ethylcarbonyl group; a 2-{3,5-di(2',3'-dihydrophytyloxy)benzylaminocarbonyl}ethylcarbonyl group; a 2-{1-[2,3,4-tri(2',3'-dihydrophytyloxy)phenyl]benzylaminocarbonyl}ethylcarbonyl group; a 2-{1-[4-(2',3'-dihydrophytyloxy)phenyl]-4'-(2',3'-dihydrophytyloxy)benzylaminocarbonyl}ethylcarbonyl group; a 3,4,5-tris[3,4,5-tri(2',3'-dihydrophytyloxy)benzyl]benzyloxysuccinyl group; or a 2-{3,4,5-tris[3,4,5-tri(2',3'-dihydrophytyloxy)benzyl]benzylaminocarbonyl}ethylcarbonyl group.

The "temporary protecting group removable under acidic conditions" that protects the 5'-hydroxy group in nucleic acid b is not particularly limited as long as it can be deprotected under acidic conditions and can be used as a hydroxy-protecting group. Examples thereof include trityl group, 9-(9-phenyl)xanthenyl group, 9-phenylthioxanthenyl group, bis(C₁₋₆ alkoxy)trityl groups such as 1,1-bis(4-methoxyphenyl)-1-phenylmethyl group (dimethoxytrityl group) and the like, mono(C₁₋₁₈ alkoxy) trityl groups such as 1-(4-methoxyphenyl)-1,1-diphenylmethyl group (monomethoxytrityl group) and the like, and the like. Among these, the temporary protecting group of hydroxy group is preferably a monomethoxytrityl group or a dimethoxytrityl group, more preferably a dimethoxytrityl group, in view of easiness of deprotection and easy availability.

The organic group for Lⁿ¹ means a group in which a hydrocarbon group or a carbon atom in a hydrocarbon group is replaced by a hetero atom. Examples of the hetero atom include oxygen atom, nitrogen atom, sulfur atom and the like. The organic group may have a substituent such as hydroxy group, amino group, oxo group (=O) or the like. The hydroxy group and the amino group that the organic group may have are preferably protected by a protecting group. The shape of the organic group may be a chain (linear or branched chain), a ring or a combination of these.

The organic group may have a group having functionality to cells. The group having functionality to cells is preferably bonded to a terminal of the main chain or a side chain of the organic group. Examples of the group having functionality to cells include "a group that improves cellular membrane permeability of a compound by improving liposolubility of the compound", "a group that improves intracellular uptake of a compound via cellular membrane receptor" and the like. Examples of the "group that improves cellular membrane permeability of a compound by improving liposolubility of the compound" include cholesterol residue, tocopherol residue and the like. Examples of the "group that improves intracellular uptake of a compound via cellular membrane receptor" include N-acetylgalactosamine residue and the like. These groups that have functionality for cells are described in WO2017/104836.

Specific examples of -OLⁿ¹-OH include the following (in the following formulas, * shows the bonding position to phosphorus atom and Ac is an acetyl group).

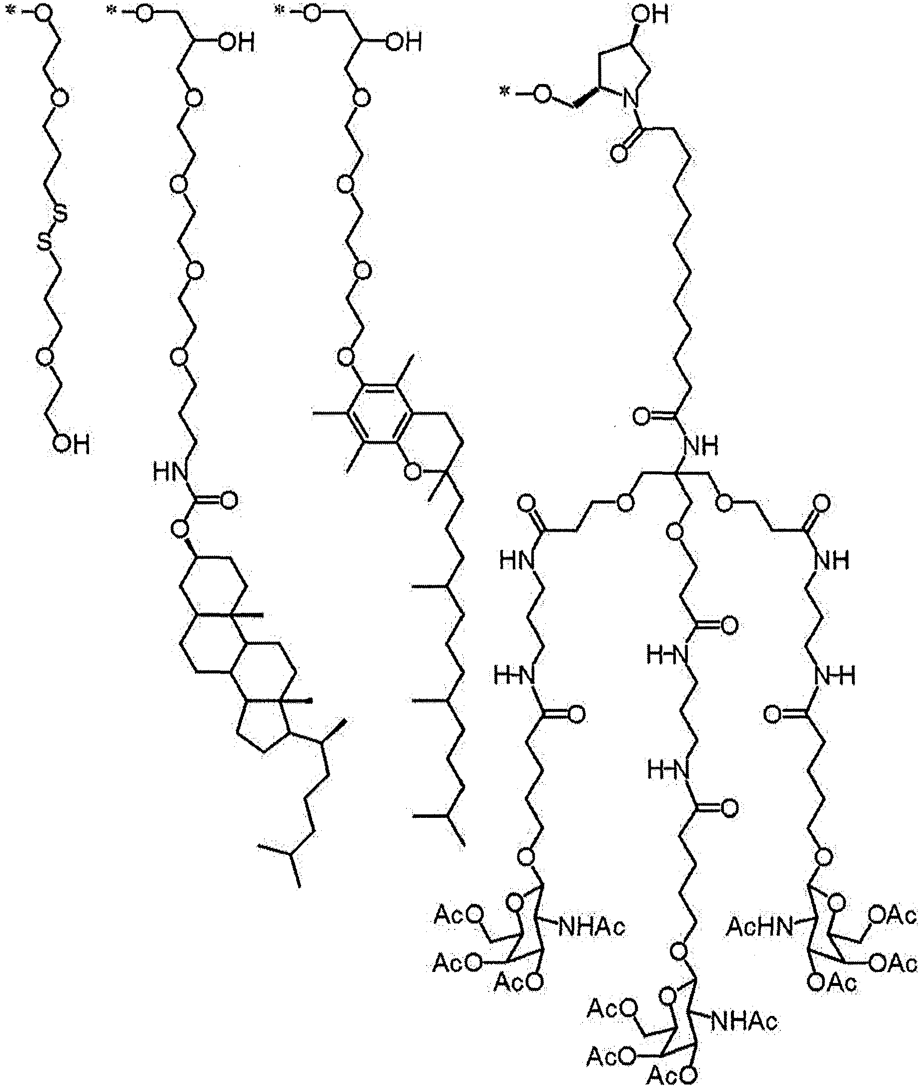

Lⁿ¹ is preferably a C₂₋₆ alkylene group, more preferably an ethylene group.

Nucleic acid a, nucleic acid α and nucleic acid b suitable in performing the production method of the present invention are described in detail below.

Examples of the nucleic acid a used in step (1) of the present invention include a compound represented by the following formula (a-I) (i.e., nucleoside or oligonucleotide).

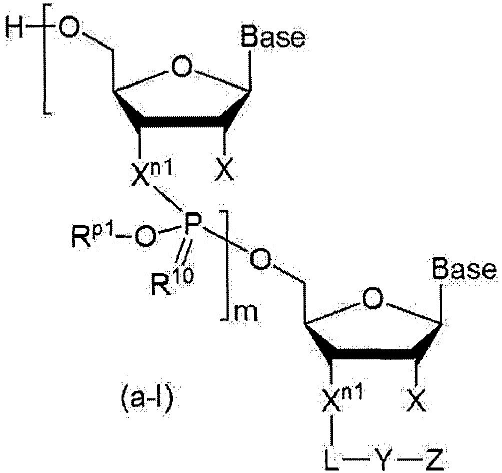

wherein
m is an integer of not less than 0;
Base in the number of m+1 are each independently optionally protected nucleic acid base;
X in the number of m+1 are each independently a hydrogen atom, a halogen atom, an optionally protected hydroxy group, or a divalent organic group bonded to 2-position carbon atom and 4-position carbon atom;
Xⁿ¹ in the number of m+1 are each independently an oxygen atom or NH;
R¹⁰ in the number of m are each independently an oxygen atom or a sulfur atom;
R^{p1} in the number of m are each independently is a protecting group of phosphate group;
L, Y and Z are as defined above.

In the following, a compound represented by the formula (a-I) is sometimes to be abbreviated as " compound (a-I)". Also, compounds represented by other formulas are sometimes abbreviated similarly.

The amino group of the nucleic acid base is preferably protected by a protecting group. As the protecting group, acetyl group, phenoxyacetyl group, 4-isopropylphenoxyacetyl group, benzoyl group, isobutyryl group, (2-hexyl)decanoyl group, dimethylformamidinyl group, and =NC(R¹¹)-N(R¹²)(R¹³) group wherein R¹¹ is a methyl group, R¹² and R¹³ are each independently a C₁₋₅ alkyl group, or R¹¹ and R¹² are optionally joined to form, together with the carbon atom and nitrogen atom bonded thereto, a 5-membered or 6-membered nitrogen-containing hydrocarbon ring are preferable. Examples of the aforementioned =NC(R¹¹)-N(R¹²)(R¹³) group include a 1-(dimethylamino)ethylidene group. When compound (a-I) has plural amino groups, the amino-protecting group may be only one or more kinds.

When m is 0, compound (a-I) is a nucleoside, and when m is one or more, compound (a-I) is an oligonucleotide. m is preferably not more than 49, more preferably not more than 29, further preferably not more than 19, particularly preferably not more than 4, and most preferably not more than 2.

As the halogen atom for X, a fluorine atom or a chlorine atom is preferable, and a fluorine atom is more preferable.

The protecting group of the optionally protected hydroxy group for X is not particularly limited and for example, any protecting group described in PROTECTIVE GROUPS IN ORGANIC SYNTHESIS, 3rd ed., JOHN WILLY&SONS (1999) and the like can be mentioned. Specifically, a methyl group, benzyl group, p-methoxybenzyl group, a tert-butyl group, methoxymethyl group, methoxyethyl group, 2-tetrahydropyranyl group, ethoxyethyl group, cyanoethyl group, cyanoethoxymethyl group, phenylcarbamoyl group, 1,1-dioxothiomorpholine-4-thiocarbamoyl group, acetyl group, pivaloyl group, benzoyl group, trimethylsilyl group, triethylsilyl group, triisopropylsilyl group, tert-butyldimethylsilyl group, [(triisopropylsilyl)oxy]methyl (Tom) group, 1-(4-chlorophenyl)-4-ethoxypiperidin-4-yl (Cpep) group and the like can be mentioned. The protecting group of the optionally protected hydroxy group is preferably a triethylsilyl group, a triisopropylsilyl group or a tert-butyldimethylsilyl group, particularly preferably a tert-butyldimethylsilyl group, from the aspects of economic efficiency and easy availability.

The "divalent organic group bonded to 2-position carbon atom and 4-position carbon atom" for X is not particularly limited as long as it is bonded to 2-position carbon atom and 4-position carbon atom of nucleoside. Examples of the divalent organic group include an optionally substituted C₂₋₇ alkylene group, and a divalent organic group constituted of an optionally substituted C₁₋₇ alkylene group and a divalent linker selected from -O-, -NR³³- (R³³ is a hydrogen atom or a C₁₋₆ alkyl group), -S-, -CO-, -COO-, -OCONR³⁴- (R³⁴ is a hydrogen atom or a C₁₋₆ alkyl group) and -CONR³⁵- (R³⁵ is a hydrogen atom or a C₁₋₆ alkyl group), and the like. Examples of the substituent that the C₁₋₇ alkylene group and C₂₋₇ alkylene group optionally have include a methylidene group (CH₂=).

As the "divalent organic group bonded to 2-position carbon atom and 4-position carbon atom", an optionally substituted C₂₋₇ alkylene group, -ORⁱ- (Rⁱ is a C₁₋₆ alkylene group bonded to 4-position carbon atom), -O-NR³³-R^{j}- (R^{j} is a C₁₋₆ alkylene group bonded to 4-position carbon atom, R³³ is as defined above), -O-R^{k}-O-R^{l}- (R^{k} is a C₁₋₆ alkylene group, R¹ is a C₁₋₆ alkylene group bonded to and crosslinked with 4-position carbon atom) are preferable, -ORⁱ- (Rⁱ is as defined above),-O-NR³³-R^{j}- (R^{j} and R³³ are as defined above), -O-R^{k}O-R^{l}- (R^{k} and R^{l} are as defined above) are more preferable. Cₗ-₆ alkylene groups for Rⁱ, R^{j}, R^{k} and R^{l} are preferably each independently a methylene group or an ethylene group.

As the "divalent organic group bonded to 2-position carbon atom and 4-position carbon atom", -O-CH₂-, -O-CH₂-CH₂-,-O-NR³³-CH₂- (R³³ is as defined above), -O-CH₂-O-CH₂- are more preferable, -O-CH₂-, -O-CH₂-CH₂-, -O-NH-CH₂-, -O-N(CH₃)-CH₂-, -O-CH₂-O-CH₂- (in each of which the left side is bonded to 2-position carbon atom and the right side is bonded to 4-position carbon atom) are further preferable.

X in the number of m+1 are each independently preferably a hydrogen atom, a halogen atom or an optionally protected hydroxy group, more preferably a hydrogen atom or an optionally protected hydroxy group.

The protecting group of phosphate group for R^{p1} is not particularly limited as long as it is removable under basic conditions and can be used as a protecting group of phosphate group. A group represented by -CH₂CH₂WG (WG is an electron-withdrawing group) is preferable.

Examples of the electron-withdrawing group for WG include cyano group, nitro group and the like, preferably cyano group.

R^{p1} in the number of m are preferably each independently a group represented by -CH₂CH₂WG.

Xⁿ¹ in the number of m+1 are preferably oxygen atoms. Explanations of L, Y and Z are as mentioned above.

Compound (a-I) is preferably a compound represented by the following formula (a-i) (definition and explanation of symbols in the following formula are as mentioned above).

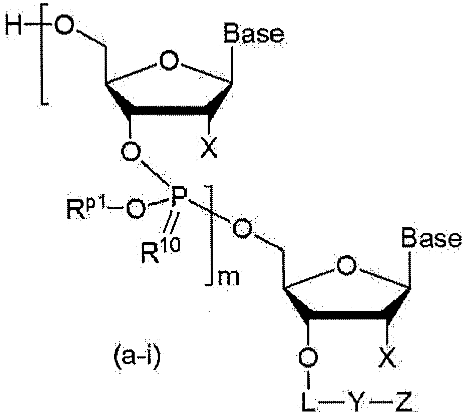

Compound (a-I) can be produced by a method known per se or a method analogous thereto. For example, it can be produced according to method described in WO 2017/104836.

Of compounds (a-i), a compound represented by the formula (a-II) (i.e., nucleoside or oligonucleotide) is preferable.

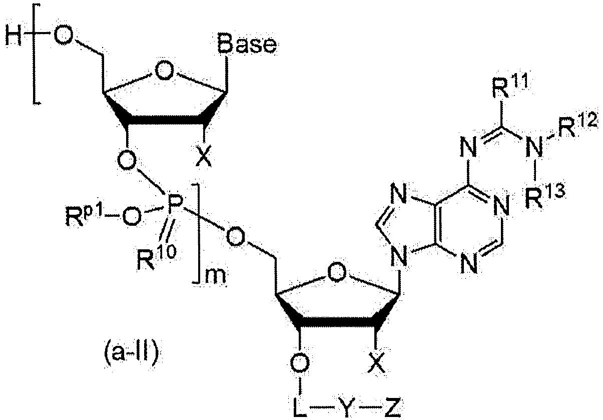

wherein
m, Base in the number of m, X in the number of m+1, R¹⁰ in the number of m, R^{p1} in the number of m, L, Y and Z are each independently as defined above;
R¹¹ is a methyl group, R¹² and R¹³ are each independently a C₁₋₅ alkyl group, or R¹¹ and R¹² are optionally joined to form, together with the carbon atom and nitrogen atom bonded thereto, a 5-membered or 6-membered nitrogen-containing hydrocarbon ring.

In the formula (a-II), preferably, R^{p1} in the number of m are each independently a group represented by -CH₂CH₂WG.

In the formula (a-II), m is preferably 0. That is, of compounds (a-II), a compound represented by the formula (a-III) (i.e., nucleoside) is preferable (definition and explanation of the symbols in the following formula are as mentioned above).

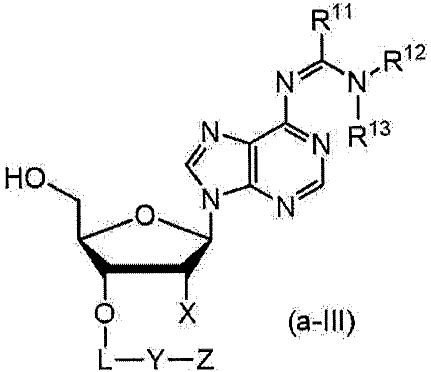

In the formula (a-II) and the formula (a-III), R¹¹ is preferably a methyl group and R¹² and R¹³ are preferably each independently a C₁₋₅ alkyl group, and R¹¹, R¹² and R¹³ are more preferably methyl groups.

Explanations of other symbols in the formula (a-II) and the formula (a-III) are as mentioned above.

In the present invention, when oligonucleotide having a phosphorothioated site wherein the first residue has adenine as a nucleic acid base is produced, production of a branched product and the like can be suppressed by using compound (a-II) (particularly, compound (a-III)) as a nucleoside, nucleotide or oligonucleotide which is a starting material. As used herein, the branch product refers to a by-product produced when an amino-protecting group of the nucleic acid base of the object compound is detached and the amino group and a monomer are bonded.

In compounds (a-i), a compound represented by the formula (a-IV) (i.e., nucleoside or oligonucleotide) is preferable.

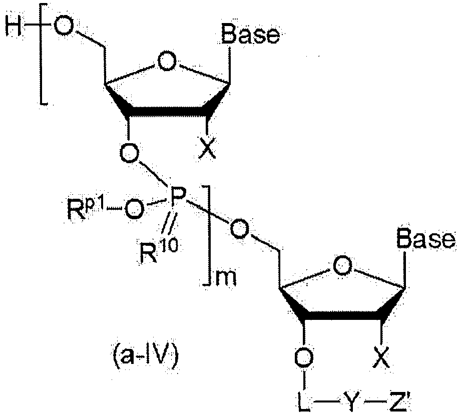

wherein
m, Base in the number of m+1, X in the number of m+1, R¹⁰ in the number of m, R^{p1} in the number of m, L and Y are each independently as defined above;
Z' is a group represented by the formula (a2"):

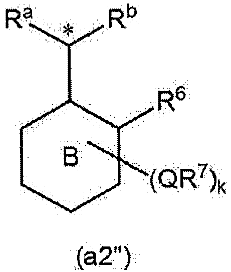

wherein
* indicates a bonding position;
R⁶ is a hydrogen atom or when R^{b} is a group represented by the following formula (a3), it optionally shows, together with R⁸, a single bond or -O- to form, together with ring B and ring C, a fused ring;
k is an integer of 1 - 4;
Q in the number of k are each independently -O-, -C(=O)-, -C(=O)O-, -OC(=O)-, -C(=O)NH- or -NHC(=O)-;
R⁷ in the number of k are each independently a hydrocarbon group wherein a linear aliphatic hydrocarbon group having a carbon number of not less than 10 is bonded via a single bond or a linker, or an organic group having at least one aliphatic hydrocarbon group having one or more branched chains and having a total carbon number of not less than 14 and not more than 300;
ring B optionally has, in addition to QR⁷ in the number of k, a substituent selected from the group consisting of a halogen atom, a C₁₋₆ alkyl group optionally substituted by a halogen atom, and a C₁₋₆ alkoxy group optionally substituted by a halogen atom;
Rₐ is a hydrogen atom; and
R_{b} is a hydrogen atom, or a group represented by the formula (a3):

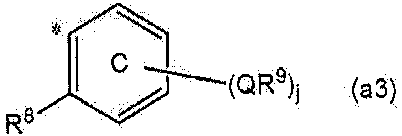

wherein * indicates a bonding position;
j is an integer of 0 to 4;
Q in the number of j are each independently as defined above;
R⁹ in the number of j are each independently a hydrocarbon group wherein a linear aliphatic hydrocarbon group having a carbon number of not less than 10 is bonded via a single bond or a linker, or an organic group having at least one aliphatic hydrocarbon group having one or more branched chains and having a total carbon number of not less than 14 and not more than 300;
R⁸ is a hydrogen atom, or optionally shows, together with R⁶, a single bond or -O- to form a fused ring with ring B and ring C; and
ring C optionally has, in addition to QR⁹ in the number of j, a substituent selected from the group consisting of a halogen atom, a C₁₋₆ alkyl group optionally substituted by a halogen atom, and a C₁₋₆ alkoxy group optionally substituted by a halogen atom, or
R^{a} and R^{b} are joined to form an oxo group.

In the formula (a-IV), R^{p1} in the number of m are preferably each independently a group represented by -CH₂CH₂WG.

In the formula (a-IV), m is preferably 0.

Explanations of other symbols in the formula (a-IV) are as mentioned above.

In compounds (a-IV), a compound represented by the following formula (a-V) (i.e., nucleoside) is preferable (definition and explanation of the symbols in the following formula are as mentioned above).

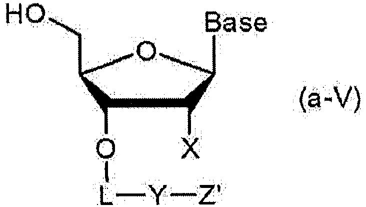

In compounds (a-I), compound (a-IV) (particularly, compound (a-V)) having Z' represented by the formula (a2") (i.e., structure of cyclohexylmethyl group) in the pseudo-solid-phase protecting group shows high solubility in non-polar solvents as compared with other compounds (a-I) having Z represented by the formula (a2) (i.e., structure of benzyl group) in the pseudo-solid-phase protecting group. Therefore, using compound (a-IV) (particularly, compound (a-V)), the production method of the present invention can be performed at a higher concentration and the productivity is strikingly improved. Explanation of Z' represented by the formula (a2") is the same as that of Z represented by the aforementioned formula (a2").

In the formulas (a-IV) and (a-V), a combination wherein L is a succinyl group or a group represented by the formula (a1') (in the formula (a1'), R¹ and R² are each independently a C₁₋₁₀ alkyl group, L¹ is a divalent phenylene group, and L² is a single bond), and Y-Z' is 3,4,5-tris(octadecyloxy)cyclohexylmethyloxy group, 3,5-bis(docosyloxy)cyclohexylmethyloxy group, 3,5-bis[3',4',5'-tris(octadecyloxy)cyclohexylmethyloxy]cyclohexylmethyloxy group, 3,4,5-tris[3',4',5'-tris(octadecyloxy)cyclohexylmethyloxy]cyclohexylmethyloxy group, 3,4,5-tris(octadecyloxy)cyclohexylmethylamino group, 2,4-bis(docosyloxy)cyclohexylmethylamino group, 3,5-bis(docosyloxy)cyclohexylmethylamino group, 4-methoxy-2-[3',4',5'-tris(octadecyloxy)cyclohexylmethyloxy]cyclohexylmethylamino group, 4-methoxy-2-[3',4',5'-tris(octadecyloxy)cyclohexylmethyloxy]cyclohexylmethylamino group, 2,4-bis(dodecyloxy)cyclohexylmethylamino group, 3,5-bis[3',4',5'-tris(octadecyloxy)cyclohexylmethyloxy]cyclohexylmethylamino group, or 3,4,5-tris[3',4',5'-tris(octadecyloxy)cyclohexylmethyloxy]cyclohexylmethylamino group is preferable for solid-liquid separation.

In the formulas (a-IV) and (a-V), a combination wherein L is a succinyl group, and Y-Z' is 3,4,5-tris(octadecyloxy)cyclohexylmethyloxy group, 3,5-bis(docosyloxy)cyclohexylmethyloxy group, 3,5-bis[3',4',5'-tris(octadecyloxy)cyclohexylmethyloxy]cyclohexylmethyloxy group, 3,4,5-tris[3',4',5'-tris(octadecyloxy)cyclohexylmethyloxy]cyclohexylmethyloxy group, 3,4,5-tris(octadecyloxy)cyclohexylmethylamino group, 2,4-bis(docosyloxy)cyclohexylmethylamino group, 3,5-bis(docosyloxy)cyclohexylmethylamino group, 4-methoxy-2-[3',4',5'-tris(octadecyloxy)cyclohexylmethyloxy]cyclohexylmethylamino group, 4-methoxy-2-[3',4',5'-tris(octadecyloxy)cyclohexylmethyloxy]cyclohexylmethylamino group, 2,4-bis(dodecyloxy)cyclohexylmethylamino group, 3,5-bis[3',4',5'-tris(octadecyloxy)cyclohexylmethyloxy]cyclohexylmethylamino group, or 3,4,5-tris[3',4',5'-tris(octadecyloxy)cyclohexylmethyloxy]cyclohexylmethylamino group is more preferable for solid-liquid separation.

In the formulas (a-IV) and (a-V), a combination wherein L is a succinyl group, and Y-Z' is 3,4,5-tris(octadecyloxy)cyclohexylmethyloxy group, 3,5-bis(docosyloxy)cyclohexylmethyloxy group, 3,5-bis[3',4',5'-tris(octadecyloxy)cyclohexylmethyloxy]cyclohexylmethyloxy group, or 3,4,5-tris[3',4',5'-tris(octadecyloxy)cyclohexylmethyloxy]cyclohexylmethyloxy group is more preferable for solid-liquid separation.

In the formulas (a-IV) and (a-V), a combination wherein L is a succinyl group, and Y-Z' is 3,4,5-tris(octadecyloxy)cyclohexylmethyloxy group is particularly preferable for solid-liquid separation.

Examples of the nucleic acid a include a compound represented by the following formula (a-VI) (i.e., nucleoside or oligonucleotide).

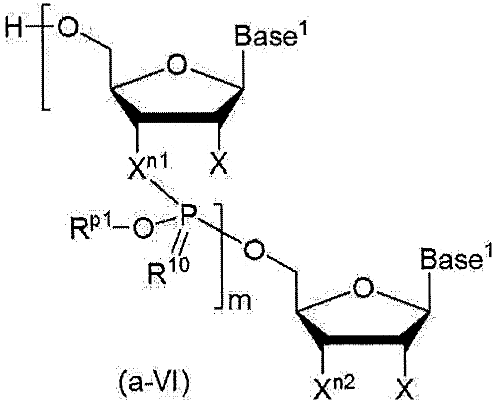

wherein
at least one of Base¹ in the number of m+1 is a nucleic acid base protected by -L-Y-Z, and the rest is an optionally protected nucleic acid base;
Xⁿ¹ in the number of m are each independently an oxygen atom or NH;
Xⁿ² is a protected hydroxy group or amino group;
m, X in the number of m+1, R¹⁰ in the number of m, R^{p1} in the number of m, L, Y and Z are each independently as defined above.

In the formula (a-VI), at least one of Base¹ in the number of m+1 is a nucleic acid base protected by -L-Y-Z. Explanations of the nucleic acid base and -L-Y-Z are as mentioned above.

Explanation of the optionally protected nucleic acid base is also as mentioned above.

The protecting group of the protected hydroxy group (Xⁿ²) is not particularly limited and for example, any protecting group described in PROTECTIVE GROUPS IN ORGANIC SYNTHESIS, 3rd ed., JOHN WILLY&SONS (1999) and the like can be mentioned. Specifically, a methyl group, benzyl group, p-methoxybenzyl group, a tert-butyl group, methoxyethyl group, ethoxyethyl group, cyanoethyl group, cyanoethoxymethyl group, phenylcarbamoyl group, 1,1-dioxothiomorpholine-4-thiocarbamoyl group, acetyl group, pivaloyl group, benzoyl group, triethylsilyl group, triisopropylsilyl group, tert-butyldimethylsilyl group, [(triisopropylsilyl)oxy]methyl (Tom) group, 1-(4-chlorophenyl)-4-ethoxypiperidin-4-yl (Cpep) group and the like can be mentioned. The hydroxy-protecting group is preferably a triethylsilyl group, triisopropylsilyl group or tert-butyldimethylsilyl group, more preferably a tert-butyldimethylsilyl group from the aspects of economic efficiency and easy availability. Protection and deprotection of the hydroxy group are well known and can be performed by, for example, the method described in the aforementioned PROTECTIVE GROUPS IN ORGANIC SYNTHESIS.

The protecting group of the protected amino group (Xⁿ²) is not particularly limited and for example, the protecting groups described in Greene's PROTECTIVE GROUPS in ORGANIC SYNTHESIS, 4th ed., Wiley-Interscience (2006) and the like can be mentioned. Specific examples of each protecting group include pivaloyl group, pivaloyloxymethyl group, acetyl group, trifluoroacetyl group, phenoxyacetyl group, 4-isopropylphenoxyacetyl group, 4-tert-butylphenoxyacetyl group, benzoyl group, isobutyryl group, (2-hexyl)decanoyl group, dimethylformamidinyl group, 1-(dimethylamino)ethylidene group and 9-fluorenylmethyloxycarbonyl group. The amino-protecting group is preferably a acetyl group, phenoxyacetyl group, 4-isopropylphenoxyacetyl group, benzoyl group, isobutyryl group, (2-hexyl)decanoyl group, dimethylformamidinyl group, or 1-(dimethylamino)ethylidene group. Protection and deprotection of the amino group are well known and can be performed by, for example, the method described in the aforementioned PROTECTIVE GROUPS in ORGANIC SYNTHESIS.

In the formula (a-VI), Xⁿ¹ in the number of m are preferably oxygen atoms.

In the formula (a-VI), Xⁿ² is preferably a protected hydroxy group.

In the formula (a-VI), R^{p1} in the number of m are preferably each independently a group represented by -CH₂CH₂WG.

In the formula (a-VI), explanations of other symbols are as mentioned above.

Compound (a-VI) is preferably a compound represented by the following formula (a-vi).

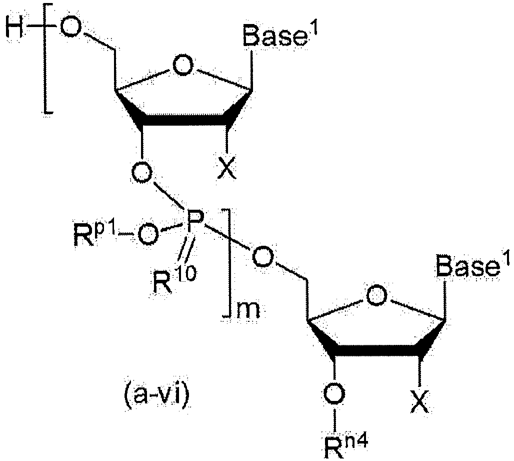

wherein
Rⁿ⁴ is a hydroxy-protecting group;
m, Base¹ in the number of m+1, X in the number of m+1, R¹⁰ in the number of m, R^{p1} in the number of m, L, Y and Z are each independently as defined above.

Explanation of Rⁿ⁴ in the formula (a-vi) is the same as that of the hydroxy-protecting group for Xⁿ².

Explanations of other symbols in the formula (a-vi) are as mentioned above.

Examples of the nucleic acid α include a compound represented by the following formula (a-VII) (i.e., nucleotide or oligonucleotide).

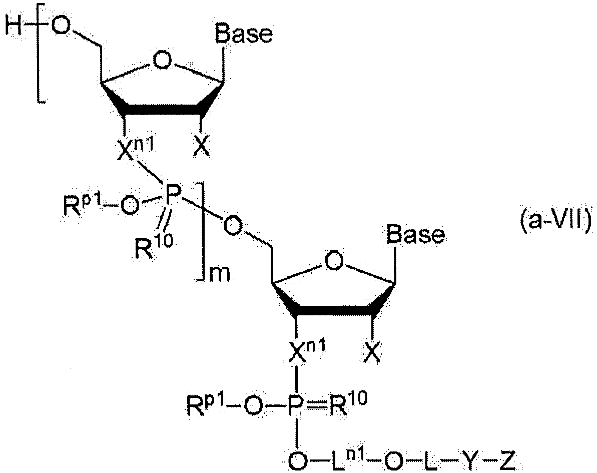

wherein
Xⁿ¹ in the number of m+1 are each independently an oxygen atom or NH;
Lⁿ¹ is an organic group;
m, Base in the number of m+1, X in the number of m+1, R¹⁰ in the number of m+1, R^{p1} in the number of m+1, L, Y and Z are each independently as defined above.

In the formula (a-VII), Lⁿ¹ is preferably a C₂₋₆ alkylene group, more preferably an ethylene group.

In the formula (a-VII), Xⁿ¹ in the number of m+1 are preferably oxygen atoms.

In the formula (a-VII), R^{p1} in the number of m+1 are preferably each independently a group represented by -CH₂CH₂WG.

Explanations of other symbols in the formula (a-VII) are as mentioned above.

Compound (a-VII) is preferably a compound represented by the following formula (a-vii) (definition and explanation of the symbols in the following formula are as mentioned above).

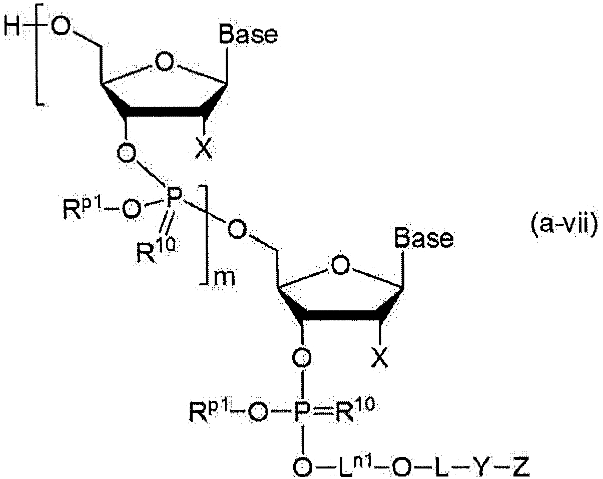

Compound (a-VII) can be produced by a method known per se or a method analogous thereto. Compound (a-VII) wherein m is 0, Xⁿ¹ is an oxygen atom, and L is a succinyl group can be produced, for example, by the following steps.
(i) condensing compound Z-Y-H and succinic anhydride to produce Z-Y-CO(CH₂)₂COOH,
(ii) condensing the obtained Z-Y-CO(CH₂)₂COOH and a compound represented by formula: HO-Lⁿ¹-OQ" (wherein Q" is a temporary protecting group and Lⁿ¹ is an organic group) in the presence of a condensing agent, and deprotecting same to produce Z-Y-CO(CH₂)₂CO-O-Lⁿ¹-OH,
(iii) reacting the obtained Z-Y-CO(CH₂)₂CO-O-Lⁿ¹-OH with phosphoramidited nucleoside to produce compound (a-VII) wherein m is 0, Xⁿ¹ is an oxygen atom and L is a succinyl group.

The aforementioned condensation reaction and deprotection are well known to those of ordinary skill in the art and those of ordinary skill in the art can perform them by appropriately setting the conditions.

Compound (a-VII) wherein L is other than a succinyl group can also be produced by performing a similar reaction by using the corresponding acid anhydride, corresponding dicarboxylic acid halide, active ester of corresponding dicarboxylic acid or the like instead of succinic anhydride. Compound (a-VII) wherein Xⁿ¹ is NH can be produced by performing a similar reaction by using nucleoside wherein 3'-amino group is phosphoramidited. Compound (a-VII) wherein m is one or more can be produced by repeating an elongation process using compound (a-VII) wherein m is 0 as the starting material.

The nucleic acid a or nucleic acid α used in step (1) of the present invention is preferably compound (a-I), compound (a-VI) or compound (a-VII), more preferably compound (a-i), compound (a-vi) or compound (a-vii), still more preferably compound (a-i) or compound (a-vi), further preferably compound (a-i), still further preferably compound (a-II) or compound (a-IV), particularly preferably compound (a-III) or compound (a-V).

The nucleic acid b used in step (1) of the present invention has a 5'-hydroxy group protected by a protecting group (temporary protecting group). The protecting group of hydroxy group is not particularly limited as long as it can be deprotected under acidic conditions and can be used as a hydroxy-protecting group. Examples thereof include trityl group, 9-(9-phenyl)xanthenyl group, 9-phenylthioxanthenyl group, bis(C₁₋₆ alkoxy)trityl groups such as 1,1-bis(4-methoxyphenyl)-1-phenylmethyl group (dimethoxytrityl group) and the like, mono(C₁₋₁₈ alkoxy)trityl groups such as 1-(4-methoxyphenyl)-1,1-diphenylmethyl group (monomethoxytrityl group) and the like, and the like. Among these, the temporary protecting group of hydroxy group is preferably a monomethoxytrityl group or a dimethoxytrityl group, more preferably a dimethoxytrityl group, in view of easiness of deprotection and easy availability.

Modification of the 3'-hydroxy group or 3'-amino group in nucleic acid b by a method selected from phosphoramidite method, H-phosphonate method, dihalophosphine method and oxazaphospholidine method can be performed according to a method known in the art, as described in the aforementioned step (1).

As to the case where the phosphoramidite method is applied as one example of the method for producing nucleic acid b, for example, the description of WO 2017/104836 can be referred to.

Examples of the nucleic acid b include a compound represented by the following formula (b-I) (i.e., nucleoside or oligonucleotide).

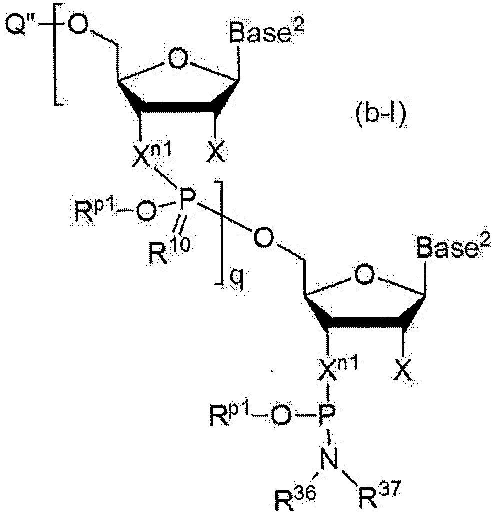

wherein
q is an integer of not less than 0;
Base² in the number of q+1 are each independently a nucleic acid base optionally protected by a protecting group selected from -L-X-Z and protecting groups used for nucleic acid synthesis;
X in the number of q+1, R^{p1} in the number of q+1, R¹⁰ in the number of q, L, X and Z are each independently as defined above;
Xⁿ¹ in the number of q+1 are each independently an oxygen atom or NH;
Q" is a temporary protecting group of hydroxy group removable under acidic conditions;
R³⁶ and R³⁷ are each independently an alkyl group, or a 5- or 6-membered saturated cyclic amino group formed together with the adjacent nitrogen atom, and the saturated cyclic amino group, optionally has, as a ring-constituting atom, one oxygen atom or sulfur atom besides nitrogen atom.

R³⁶ and R³⁷ are preferably each independently a C₁₋₁₀ alkyl group, or a 5- or 6-membered saturated cyclic amino group formed together with the adjacent nitrogen atom, more preferably a C₁₋₁₀ alkyl group, further preferably a C₁₋₆ alkyl group.

Explanation of the temporary protecting group of hydroxy group removable under acidic conditions is as mentioned above. Q" is preferably a monomethoxytrityl group or a dimethoxytrityl group, more preferably a dimethoxytrityl group.

The amino group of the nucleic acid base in the formula (b-I) is preferably protected by a protecting group. As the protecting group, a protecting group selected from -L-X-Z and protecting groups used for nucleic acid synthesis can be mentioned. Explanations of L, X and Z are as mentioned above. As the protecting group used for nucleic acid synthesis, acetyl group, phenoxyacetyl group, 4-isopropylphenoxyacetyl group, benzoyl group, isobutyryl group, (2-hexyl)decanoyl group, dimethylformamidinyl group, and =NC(R¹¹)-N(R¹²)(R¹³) group wherein R¹¹ is a methyl group, R¹² and R¹³ are each independently a C₁₋₅ alkyl group, or R¹¹ and R¹² are optionally joined to form, together with the carbon atom and nitrogen atom bonded thereto, a 5-membered or 6-membered nitrogen-containing hydrocarbon ring is preferable. Examples of the aforementioned =NC(R¹¹)-N(R¹²)(R¹³) group include a 1-(dimethylamino)ethylidene group. When compound (b-I) has plural amino groups, the amino-protecting group may be only one or more kinds.

When q is 0, compound (b-I) is a nucleoside, and when q is one or more, compound (b-I) is an oligonucleotide. As compound (b-I) used in this step, q is preferably not more than 49, more preferably not more than 29, further preferably not more than 19, particularly preferably not more than 4, and most preferably not more than 2.

X in the number of q+1 are each independently preferably a hydrogen atom, a halogen atom or an optionally protected hydroxy group, more preferably a hydrogen atom or an optionally protected hydroxy group.

Base² are each independently preferably a nucleic acid base optionally protected by a protecting group selected from protecting groups used for nucleic acid synthesis.

R^{p1} in the number of q+1 are preferably each independently a group represented by -CH₂CH₂WG.

Xⁿ¹ in the number of q+1 are preferably oxygen atoms. Explanations of L, X and Z are as mentioned above. Compound (b-I) is preferably a compound represented by the following formula (b-i).

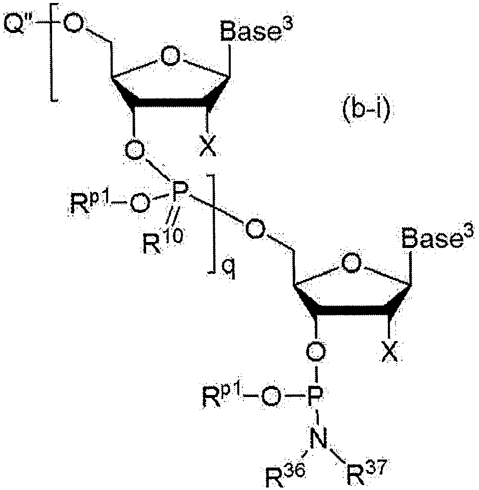

wherein
Base³ in the number of q+1 are each independently a nucleic acid base optionally protected by protecting groups used for nucleic acid synthesis,
q, X in the number of q+1, R^{p1} in the number of q+1, R¹⁰ in the number of q, Q", R³⁶ and R³⁷ are each independently as defined above.

In the formula (b-i), explanations of the protecting groups used for nucleic acid synthesis, q, X in the number of q+1, R^{p1} in the number of q+1, R¹⁰ in the number of q, Q", R³⁶ and R³⁷ are as mentioned above.

The combination of the nucleic acid a or nucleic acid α, and nucleic acid b used in step (1) of the present invention is
preferably a combination of compound (a-I), compound (a-VI) or compound (a-VII), and compound (b-I),
more preferably a combination of compound (a-i), compound (a-vi) or compound (a-vii), and compound (b-i),
still more preferably a combination of compound (a-i) or compound (a-vi), and compound (b-i),
further preferably a combination of compound (a-i) and compound (b-i),
still further preferably a combination of compound (a-II) or compound (a-IV), and compound (b-i),
particularly preferably a combination of compound (a-III) or compound (a-V), and compound (b-i).

The method for producing an oligonucleotide having a phosphorothioated site of the present invention has been described in detail above. This production method is a method for producing an oligonucleotide having a phosphorothioated site by elongating a nucleic acid chain from the "3'-side to the 5'-side direction", which is now generally performed in the chain elongation reaction of a nucleic acid chain in the art. The nucleic acid A is preferably a nucleoside, nucleotide or oligonucleotide wherein 5'-hydroxy group is not protected, and other group is optionally protected by a protecting group used for nucleic acid synthesis. The method for producing an oligonucleotide having a phosphorothioated site of the present invention can also be applied to the production of an oligonucleotide having a phosphorothioated site by elongating a nucleic acid chain in the opposite direction from the "5'-side to the 3'-side direction". The production of an oligonucleotide having a phosphorothioated site by elongating a nucleic acid chain in the "5'-side to the 3'-side direction" can be performed by the method shown below.

[A] A method for producing an oligonucleotide having a phosphorothioated site, comprising a step of obtaining a phosphite form or a phosphorous acid diester form by coupling a nucleoside, nucleotide or oligonucleotide wherein a 3'-hydroxy group or 3'-amino group is not protected, and other group is optionally protected by a protecting group used for nucleic acid synthesis or bonded to a solid phase carrier (nucleic acid A-1) (preferably, the 3'-position in nucleic acid A-1 is a hydroxy group), and a nucleoside, nucleotide or oligonucleotide wherein a 5'-hydroxy group is modified by a method for forming a phosphite form or a phosphorous acid diester form (e.g., a method selected from a phosphoramidite method, an H-phosphonate method, a dihalophosphine method and an oxazaphospholidine method), and other group is optionally protected by a protecting group used for nucleic acid synthesis (nucleic acid B-1), in the presence of an antioxidant (step (1-1)).
[B] A method for producing an oligonucleotide having a phosphorothioated site, comprising a step of obtaining a phosphite form or a phosphorous acid diester form by coupling a nucleoside, nucleotide or oligonucleotide wherein a 3'-hydroxy group or 3'-amino group is not protected, and other group is optionally protected by a protecting group used for nucleic acid synthesis or bonded to a solid phase carrier (nucleic acid A-1) (preferably, the 3'-position in nucleic acid A-1 is a hydroxy group), and a nucleoside, nucleotide or oligonucleotide wherein a 5'-hydroxy group is modified by a method for forming a phosphite form or a phosphorous acid diester form (e.g., a method selected from a phosphoramidite method, an H-phosphonate method, a dihalophosphine method and an oxazaphospholidine method), and other group is optionally protected by a protecting group used for nucleic acid synthesis (nucleic acid B-1), in the presence of an antioxidant (step (1-1)), and further,
   a step of reacting the obtained phosphite form or phosphorous acid diester form with a sulfurizing reagent (step (2-1)).

When a coupling product is obtained using nucleic acid A-1 having an amino group at the 3'-position instead of nucleic acid A-1 having a hydroxy group at the 3'-position, and applying a method such as the phosphoroamide method, the H-phosphonate method, the dihalophosphine method, or the oxazaphospholidine method, the bond between the 3'-position and the phosphorus atom becomes an -N-P bond instead of the -O-P bond. In the present specification, this case is to be referred to as "phosphite form" or "phosphorous acid diester form" for convenience according to the -O-P bond. The corresponding "PO impurity" is also to be referred to as "phosphotriester form" or "phosphodiester form" in the same manner.

The definition of each constituent element such as "antioxidant", "sulfurizing reagent", "nucleic acid A-1", "nucleic acid B-1" and the like and the detailed reaction conditions of the two steps in the production method including the above-mentioned two steps are the same as the definition of each constituent element and the reaction conditions of each step described in detail in the above-mentioned method for producing an oligonucleotide having a phosphorothioated site by elongating the nucleic acid chain from the "3'-side to the 5'-side direction". By referring thereto, those of ordinary skill in the art can appropriately produce an oligonucleotide having a phosphorothioated site by elongating a nucleic acid chain in the "5'-side to the 3'-side direction".

The present invention also includes a method for producing an oligonucleotide having a phosphorothioated site by using a nucleoside, nucleotide or oligonucleotide wherein a 5'-hydroxy group is not protected, and other group is optionally bonded to a solid phase carrier (hereinafter sometimes to be referred to as "nucleic acid A"), and a nucleoside, nucleotide or oligonucleotide wherein a 3'-hydroxy group or 3'-amino group is not protected, and other group is optionally bonded to a solid phase carrier (nucleic acid A-1). When the production method of the present invention is performed under solid-phase conditions, one group of nucleic acid A or nucleic acid A-1 binds to a solid-phase carrier. The solid phase support or resin used as the solid phase carrier may be any support known in the pertinent technical field and suitable for use in solid phase synthesis. In the present specification, the term "solid phase" includes the binding or linking of nucleoside, nucleotide or oligonucleotide to the above-mentioned solid phase support or resin via a commonly-used functional linker or handle group. Thus, the "solid phase" in this context also includes such linkers. Examples of the solid phase include polystyrene supports (which may be further functionalized by, for example, p-methylbenzyl-hydrylamine), or rigid functionalized supports such as diatomaceous earth-encapsulated polydimethylacrylamide (pepsin K), silica, microporous glass, and the like. The resin matrix of the solid phase may be composed of amphiphilic polystyrene-PEG resin or PEG-polyamide or PEG-polyester resin. Examples of the solid phase carrier include Wang-PEG resin and Rink-amide PEG resin.

### [Example]

The present invention is explained in further detail in the following by referring to Examples, which are not to be construed as limiting the scope of the present invention. The reagents, apparatuses and materials used in the present invention are commercially available unless otherwise specified. In the present specification, when amino acid and the like are indicated by abbreviation, each indication is based on the abbreviation of the IUPAC-IUB Commission on Biochemical Nomenclature or conventional abbreviation in the art.

The meanings of the abbreviations used in the below-mentioned Examples and the like are as described below.
SUC: succinyl
TOB: 3,4,5-tris (octadecyloxy)benzyloxy
2'-OMe-C-CE phosphoramidite: 5'-O-(4,4'-dimethoxytrityl)-2'-O-methyl-cytidine-3'-[O-(2-cyanoethyl)-(N,N-diisopropyl)]-phosphoramidite
DDTT: 5-[(N,N-dimethylaminomethylidene)amino]-3H-1,2,4-dithiazole-3-thione
ETT: 5-ethylthio-1H-tetrazole
TFE: 2,2,2-trifluoroethanol
TOF-MS: time-of-flight mass spectrometer
HO-CmT-SUC-TOB: (2'-O-methyl-cytidine 3'-[O-(2-cyanoethyl)]phosphorothionyl thymidin-3'-yl-[3,4,5-tris (octadecyloxy)benzyl]succinate)
HO-CmCmT-SUC-TOB: (2'-O-methyl-cytidine 3'-[O-(2-cyanoethyl)]phosphorothionyl 2'-O-methyl-cytidine 3'-[O-(2-cyanoethyl)]phosphorothionyl thymidin-3'-yl-[3,4,5-tris (octadecyloxy)benzyl]succinate)
HO-CmCmCmT-SUC-TOB: (2'-O-methyl-cytidine 3'-[O-(2-cyanoethyl)]phosphorothionyl 2'-O-methyl-cytidine 3'-[O-(2-cyanoethyl)]phosphorothionyl 2'-O-methyl-cytidine 3'-[O-(2-cyanoethyl)]phosphorothionyl thymidin-3'-yl-[3,4,5-tris (octadecyloxy)benzyl]succinate)
HO-CmCmCmCmT-SUC-TOB: (2'-O-methyl-cytidine 3'-[O-(2-cyanoethyl)]phosphorothionyl (2'-O-methyl-cytidine 3'-[O-(2-cyanoethyl)]phosphorothionyl 2'-O-methyl-cytidine 3'-[O-(2-cyanoethyl)]phosphorothionyl 2'-O-methyl-cytidine 3'-[O-(2-cyanoethyl)]phosphorothionyl thymidin-3'-yl-[3,4,5-tris (octadecyloxy)benzyl]succinate)
T-CE phosphoramidite: 5'-O-(4,4'-dimethoxytrityl)-thymidine-3'-[O-(2-cyanoethyl)-(N,N-diisopropyl)]-phosphoramidite HO-TT-SUC-TOB: (thymidine 3'-[O-(2-cyanoethyl)]phosphorothionyl thymidin-3'-yl-[3,4,5-tris (octadecyloxy)benzyl]succinate)
POS: 5-phenyl-3H-1,2,4-dithiazol-3-one

Piv-TOB: 3,4,5-tris (octadecyloxy)benzyl pivalate
PADS: phenylacetyl disulfide
DMTr: 4,4'-dimethoxytrityl
LC-TOF MS: liquid chromatograph-time-of-flight mass spectrometer
DBU: 1,8-diazabicyclo[5.4.0]-7-undecene
PivCl: pivaloyl chloride
CMPT: N-(cyanomethyl)pyrrolidinium triflate

2'-OMe-C-CE phosphoramidite: 5'-O-(4,4'-dimethoxytrityl)-N⁴-benzoyl-2'-O-methyl-cytidine-3'-[O-(2-cyanoethyl)-(N,N-diisopropyl)]-phosphoramidite 2'-OMe-G-CE phosphoramidite: 5'-O-(4,4'-dimethoxytrityl)-N²-isobutyryl-2'-O-methyl-guanosine-3'-[O-(2-cyanoethyl)-(N,N-diisopropyl)]-phosphoramidite

5-Me-dC(Bz)-CE phosphoramidite: 5'-O-(4,4'-dimethoxytrityl)-N⁴-benzoyl-2'-deoxy-5-methylcytidine-3'-[O-(2-cyanoethyl)-(N,N-diisopropyl)]-phosphoramidite
dG-CE phosphoramidite: 5'-O-(4,4'-dimethoxytrityl)-N²-isobutyryl-deoxyguanosine-3'-[O-(2-cyanoethyl)-(N,N-diisopropyl)]-phosphoramidite
dT-CE phosphoramidite: 5'-O-(4,4'-dimethoxytrityl)-deoxygthymidine-3'-[O-(2-cyanoethyl)-(N,N-diisopropyl)]-phosphoramidite

2'-O-TBDMS-C(Ac)-CE phosphoramidite: 5'-O-(4,4'-dimethoxytrityl)-N⁴-acetyl-2'-O-(tert-butyldimethylsilyl)-cytidine-3'-[O-(2-cyanoethyl)-(N,N-diisopropyl)]-phosphoramidite

LNA-A(Bz)-CE phosphoramidite: 5'-O-(4,4'-dimethoxytrityl)-N⁶-benzoyl-2'-O,4'-C-methylene-adenosine-3'-[O-(2-cyanoethyl)-(N,N-diisopropyl)]-phosphoramidite

2'-F-C(Bz)-CE phosphoramidite: 5'-O-(4,4'-dimethoxytrityl)-N⁴-benzoyl-2'-fluoro-cytidine-3'-[O-(2-cyanoethyl)-(N,N-diisopropyl)]-phosphoramidite

dT-H-phosphonate TEA salt: 5'-O-(4,4'-dimethoxytrityl)-deoxythymidine-3'-H-phosphonate, triethylamine salt

HO-T-SUC-TOB: deoxythymidin-3'-yl 3,4,5-tris (octadecyloxy)benzylsuccinate
HO-C-SUC-TOB: N⁴-benzoyl-deoxycytidin-3'-yl-[3,4,5-tris (octadecyloxy)benzyl]succinate
HO-TC-SUC-TOB: deoxythymidine-3'-[O-(2-cyanoethyl)]phosphorothionyl N⁴-benzoyl-deoxycytidin-3'-yl-[3,4,5-tris (octadecyloxy)benzyl]succinate

DMTrO-A·T·C·G·A·C·T·C·T·C·G·A·G·C·G·T·T·C·T·C-SUC-TOB (wherein "•" means phosphorothionyl bond):
5'-O-(4,4'-dimethoxytrityl)-N⁶-[1-(dimethylamino)ethylidene]-deoxyadenosine-3'-[O-(2-cyanoethyl)]phosphorothionyl-deoxythymidine-3'-[O-(2-cyanoethyl)]phosphorothionyl N⁴-benzoyl-deoxycytidine-3'-[O-(2-cyanoethyl)]phosphorothionyl N²-isobutyryl-deoxyguanosine-3'-[O-(2-cyanoethyl)]phosphorothionyl N⁶-[1-(dimethylamino)ethylidene]-deoxyadenosine-3'-[O-(2-cyanoethyl)]phosphorothionyl N⁴-benzoyl-deoxycytidine-3'-[O-(2-cyanoethyl)]phosphorothionyl -deoxythymidine-3'-[O-(2-cyanoethyl)]phosphorothionyl N⁴-benzoyl-deoxycytidine-3'-[O-(2-cyanoethyl)]phosphorothionyl-deoxythymidine-3'-[O-(2-cyanoethyl)]phosphorothionyl N⁴-benzoyl-deoxycytidine-3'-[O-(2-cyanoethyl)]phosphorothionyl N²-isobutyryl-deoxyguanosine-3'-[O-(2-cyanoethyl)]phosphorothionyl N⁶-[1-
(dimethylamino)ethylidene]-deoxyadenosine-3'-[O-(2-cyanoethyl)]phosphorothionyl N²-isobutyryl-deoxyguanosine-3'-[O-(2-cyanoethyl)]phosphorothionyl N⁴-benzoyl-deoxycytidine-3'-[O-(2-cyanoethyl)]phosphorothionyl N²-isobutyryl-deoxyguanosine-3'-[O-(2-cyanoethyl)]phosphorothionyl-deoxythymidine-3'-[O-(2-cyanoethyl)]phosphorothionyl-deoxythymidine-3'-[O-(2-cyanoethyl)]phosphorothionyl N⁴-benzoyl-deoxycytidine-3'-[O-(2-cyanoethyl)]phosphorothionyl-deoxythymidine-3'-[O-(2-cyanoethyl)]phosphorothionyl N⁴-benzoyl-deoxycytidin-3'-yl-[3,4,5-
tris(octadecyloxy)benzyl]succinate

HO-CmT-SUC-TOB: N⁴-benzoyl-2'-O-methyl-cytidine-3'-[O-(2-cyanoethyl)]phosphorothionyl deoxythymidin-3'-yl-[3,4,5-tris(octadecyloxy)benzyl]succinate
HO-GmCmT-SUC-TOB: N²-isobutyryl-2'-O-methyl-guanosine 3'-[O-(2-cyanoethyl)]phosphoryl N⁴-benzoyl-2'-O-methyl-cytidine 3'-[O-(2-cyanoethyl)]phosphorothionyl deoxythymidin-3'-yl-[3,4,5-tris (octadecyloxy)benzyl]succinate
HO-2'-OMe-U-SUC-TOB: 2'-O-methyl-uridin-3'-yl 3,4,5-tris(octadecyloxy)benzylsuccinate
HO-Um-SUC-TOB: 2'-O-methyl-uridin-3'-yl 3,4,5-tris(octadecyloxy)benzylsuccinate
HO-CmUm-SUC-TOB: N⁴-benzoyl-2'-O-methyl-cytidine-3'-[O-(2-cyanoethyl)]phosphorothionyl 2'-O-methyl-uridin-3'-yl-[3,4,5-tris(octadecyloxy)benzyl]succinate
HO-GUm-SUC-TOB: N²-isobutyryl-deoxyguanosine 3'-[O-(2-cyanoethyl)]phosphorothionyl 2'-O-methyl-uridin-3'-yl-[3,4,5-tris (octadecyloxy)benzyl]succinate
HO-A_{L}T-SUC-TOB: N⁶-benzoyl-2'-O,4'-C-methylene-adenosine-3'-[O-(2-cyanoethyl)]phosphorothionyl deoxythymidin-3'-yl-[3,4,5-tris (octadecyloxy)benzyl]succinate
HO-C_{f}A_{L}T-SUC-TOB: N⁴-benzoyl-2'-fluoro-cytidine-3'-[O-(2-cyanoethyl)]phosphorothionyl N⁶-benzoyl-2'-O,4'-C-methylene-adenosine-3'-[O-(2-cyanoethyl)]phosphorothionyl deoxythymidin-3'-yl-[3,4,5-tris(octadecyloxy)benzyl]succinate HO-rCC_{f}A_{L}T-SUC-TOB: N⁴-acetyl-2'-O-(tert-butyldimethylsilyl)-cytidine-3'-[O-(2-cyanoethyl)]phosphorothionyl N⁴-benzoyl-2'-fluoro-cytidine-3'-[O-(2-cyanoethyl)]phosphorothionyl N⁶-benzoyl-2'-O,4'-C-methylene-adenosine-3'-[O-(2-cyanoethyl)]phosphorothionyl deoxythymidin-3'-yl-[3,4,5-tris(octadecyloxy)benzyl]succinate
HO-(CH₂)₂-SUC-TOB: 3,4,5-tris(octadecyloxy)benzyl 2-hydroxyethyl succinate
HO-A_{L}(CH₂)₂-SUC-TOB: N⁶-benzoyl-2'-O,4'-C-methylene-adenosine-3'-[O-(2-cyanoethyl)]phosphorothionyl ethyloxy-[3,4,5-tris(octadecyloxy)benzyl]succinate
HO-C_{f}A_{L}(CH₂)₂-SUC-TOB: N⁴-benzoyl-2'-fluoro-cytidine-3'-[O-(2-cyanoethyl)]phosphorothionyl N⁶-benzoyl-2'-O,4'-C-methylene-adenosine-3'-[O-(2-cyanoethyl)]phosphorothionyl ethyloxy-[3,4,5-tris(octadecyloxy)benzyl]succinate
HO-rCC_{f}A_{L}(CH₂)₂-SUC-TOB: N⁴-acetyl-2'-O-(tert-butyldimethylsilyl)-cytidine-3'-[O-(2-cyanoethyl)]phosphorothionyl N⁴-benzoyl-2'-fluoro-cytidine-3'-[O-(2-cyanoethyl)]phosphorothionyl N⁶-benzoyl-2'-O,4'-C-methylene-adenosine-3'-[O-(2-cyanoethyl)]phosphorothionyl ethyloxy-[3,4,5-tris(octadecyloxy)benzyl]succinate HO-T_{H}T-SUC-TOB: deoxythymidine-3'-phosphonate deoxythymidin-3'-yl-[3,4,5-tris(octadecyloxy)benzyl]succinate
HO-T_{OH}T-SUC-TOB: deoxythymidine-3'-phosphorothionyl deoxythymidin-3'-yl-[3,4,5-tris(octadecyloxy)benzyl]succinate HO-GmT-SUC-TOB: N²-isobutyryl-2'-O-methyl-guanosine 3'-[O-(2-cyanoethyl)]phosphorothionyl deoxythymidin-3'-yl-[3,4,5-
tris(octadecyloxy)benzyl]succinate
HO-T_{SCNEt}T-SUC-TOB: deoxythymidine-3'-[S-(2-cyanoethyl)]phosphorothionyl deoxythymidin-3'-yl-[3,4,5-tris(octadecyloxy)benzyl]succinate

In the following, the TOB-added compounds were synthesized according to the method described in WO 2012/157723. The phosphoramidite monomers such as 2'-OMe-C-CE phosphoramidite and the like used in Examples and Comparative Examples were commercially available.

### Example 1: synthesis of phosphorothioated dimer to pentamer (with antioxidant)

Under an argon atmosphere, HO-T-SUC-TOB (248.2 mg, 0.20 mmol) was placed in a 50-mL two-necked flask, and dissolved in dehydrated dichloromethane (10.0 mL) and dehydrated acetonitrile (1.0 mL) added thereto. 2'-OMe-C-CE phosphoramidite (518.5 mg, 0.60 mmol) and methyldiphenylphosphine (37.8 µL, 0.20 mmol) were added and the mixture was stirred for 30 min. Thereafter, ETT (5-ethylthio-1H-tetrazole, 78.1 mg, 0.60 mmol) was added, and the mixture was stirred at room temperature for 1 hr.

Successively, to the reaction solution was added TFE (2,2,2-trifluoroethanol, 219 µL, 3.01 mmol), and the mixture was stirred at room temperature for 30 min. 2,6-Xylidine (638 µL, 5.18 mmol) and DDTT (5-[(N,N-dimethylaminomethylidene)amino]-3H-1,2,4-dithiazole-3-thione, 215.6 mg, 1.05 mmol) were successively added, and the mixture was stirred at room temperature for 1 hr. The obtained dimer (sometimes denoted as 2 mer, hereinafter the same) was analyzed to find that the PO impurity was 0.09%. m/z(TOF-MS):Calcd.1729.07, Found 1730.08 [M+H]⁺

In the same manner as in the above, elongated dimer HO-CmT-SUC-TOB (279.6 mg, 0.16 mmol) and 2'-OMe-C-CE phosphoramidite (419.2 mg, 0.485 mmol) were used to synthesize a trimer HO-CmCmT-SUC-TOB (290.8 mg). The obtained trimer was analyzed to find that the PO impurity was 0.25%. m/z(TOF-MS):Calcd.2221.16, Found 2222.17 [M+H]⁺

In the same manner, elongated trimer HO-CmCmT-SUC-TOB (290.8 mg, 0.13 mmol) and 2'-OMe-C-CE phosphoramidite (340.3 mg, 0.394 mmol) were used to synthesize tetramer HO-CmCmCmT-SUC-TOB(283.0 mg). The obtained tetramer was analyzed to find that it contained 0.27% of PO impurity. m/z(TOF-MS):Calcd.2713.24, Found 2714.23 [M+H]⁺

In the same manner, elongated tetramer HO-CmCmCmT-SUC-TOB (283.0 mg, 0.10 mmol) and 2'-OMe-C-CE phosphoramidite (270.1 mg, 0.313 mmol) were used to synthesize a pentamer HO-CmCmCmCmT-SUC-TOB (254 mg). The obtained pentamer was analyzed to find that 0.30% of PO impurity was contained. m/z(TOF-MS):Calcd.3205.33, Found 1604.69 [M+2H]²⁺

The content ratio of PO impurity at each chain length in the case of using an antioxidant is shown in Table 1.

**[Table 1] content ratio of PO impurity at each chain length (with antioxidant)**

| chain length | 2mer | 3mer | 4mer | 5mer |
|---|---|---|---|---|
| content ratio of PO impurity | 0.09% | 0.25% | 0.27% | 0.30% |

### Comparative Example 1: synthesis of phosphorothioated dimer to pentamer (without antioxidant)

Under an argon atmosphere, HO-T-SUC-TOB (247.6 mg, 0.20 mmol) was placed in a 50-mL two-necked flask, and dissolved in dehydrated dichloromethane (10.0 mL) and dehydrated acetonitrile (1.0 mL) added thereto. 2'-OMe-C-CE phosphoramidite (518.4 mg, 0.60 mmol) and ETT (5-ethylthio-1H-tetrazole, 78.1 mg, 0.60 mmol) were successively added, and the mixture was stirred at room temperature for 1 hr. Successively, TFE (2,2,2-trifluoroethanol, 218.6 µL, 3.0 mmol) was added to the reaction solution and the mixture was stirred at room temperature for 30 min. 2,6-Xylidine (380.6 µL, 3.0 mmol) and DDTT (5-[(N,N-dimethylaminomethylidene)amino]-3H-1,2,4-dithiazole-3-thione, 129.3 mg, 0.63 mmol) were successively added, and the mixture was stirred at room temperature for 1 hr. The obtained dimer was analyzed to find that the PO impurity was 0.11%.

m/z(TOF-MS):Calcd.1729.07, Found 1730.08 [M+H]⁺

In the same manner as in the above, the dimer HO-CmT-SUC-TOB (297.4 mg, 0.17 mmol) and 2'-OMe-C-CE phosphoramidite (445.5 mg, 0.52 mmol) were used to synthesize trimer HO-CmCmT-SUC-TOB (349.1 mg). The obtained trimer was analyzed to find that the PO impurity was 0.46%.

m/z(TOF-MS):Calcd.2221.16, Found 2222.17 [M+H]⁺

In the same manner, the trimer HO-CmCmT-SUC-TOB (349.1 mg, 0.16 mmol) and 2'-OMe-C-CE phosphoramidite (407.5 mg, 0.47 mmol) were used to synthesize a tetramer HO-CmCmCmT-SUC-TOB (415.3 mg). The obtained tetramer was analyzed to find that the PO impurity was 0.68%.

m/z(TOF-MS):Calcd.2713.24, Found 2714.23 [M+H]⁺

In the same manner, the tetramer HO-CmCmCmT-SUC-TOB (415.3 mg, 0.15 mmol) and 2'-OMe-C-CE phosphoramidite (399.2 mg, 0.46 mmol) were used to synthesize a pentamer HO-CmCmCmCmT-SUC-TOB(439.3 mg). The obtained pentamer was analyzed to find that the PO impurity was 0.99%.

m/z(TOF-MS):Calcd.3205.33, Found 1604.69 [M+2H]²⁺

The content ratio of PO impurity at each chain length in the case of without using an antioxidant is shown in Table 2.

**[Table 2] content ratio of PO impurity at each chain length (without antioxidant)**

| chain length | 2mer | 3mer | 4mer | 5mer |
|---|---|---|---|---|
| content ratio of PO impurity | 0.11% | 0.46% | 0.68% | 0.99% |

### Example 2: synthesis of phosphorothioated dimer to trimer (with various antioxidants)

Under an argon atmosphere, HO-TT-SUC-TOB (56.4 mg, 0.035 mmol) synthesized in the same manner as in Example 1 except that T-CE phosphoramidite was used instead of 2'-OMe-C-CE phosphoramidite in Example 1 was placed in a 20-mL two-necked flask, and dissolved in dehydrated dichloromethane (1.8 mL) and dehydrated acetonitrile (0.5 mL) added thereto. 2'-OMe-C-CE phosphoramidite (91.8 mg, 0.105 mmol) and various antioxidants (0.035 mmol) were added, and the mixture was stirred for 30 min. Thereafter, ETT (5-ethylthio-1H-tetrazole, 14 mg, 0.105 mmol) was added, and the mixture was stirred at room temperature for 3 hr. Successively, to the reaction solution was added TFE (2,2,2-trifluoroethanol, 38.4 µL, 0.53 mmol), and the mixture was stirred at room temperature for 2 hr. 2,6-Xylidine (50.2 µL, 0.407 mmol) and POS (5-phenyl-3H-1,2,4-dithiazol-3-one, 33.3 mg, 0.171 mmol) were successively added, and the mixture was stirred at room temperature for 1 hr. Each reaction solution was analyzed. The results relating to the content ratio of PO impurity are shown in Table 3.

### Comparative Example 2: synthesis of phosphorothioated dimer to trimer (without antioxidant)

Performed in the same manner as in Example 2 except that various antioxidants (0.035 mmol) in Example 2 were not contained. The results relating to the content ratio of PO impurity are shown in Table 3.

**[Table 3]**

| Example/Comparative Example No. | kind of antioxidant | | content ratio of PO impurity |
|---|---|---|---|
| Example 2 (1) | triphenyl phosphine | PPh₃ | 0.08% |
| Example 2 (2) | methyldiphenyl phosphine | PMePh₂ | 0.09% |
| Example 2 (3) | triethyl phosphite | P(OEt)₃ | 0.12% |
| Example 2 (4) | ethoxydiphenyl phosphine | PPh₂OEt | 0.08% |
| Example 2 (5) | diethoxyphenyl phosphine | PPh(OEt)₂ | 0.12% |
| Example 2 (6) | 9,10-dihydro-9-oxa-10-phospha-phenanthrene 10-oxide | 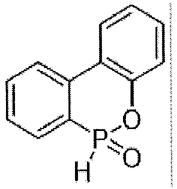 | 0.15% |
| Example 2 (7) | isobutylene sulfide | 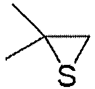 | 0.16% |
| Comparative Example 2 | none | none | 0.21% |

Synthetic Examples of various phosphorothioated oligomers in the presence of an antioxidant are shown below.

### Example 3: synthesis of phosphorothioated tetramer (phosphoramidite method) elongation reaction

Under an argon atmosphere, HO-T-SUC-TOB (300 mg, 242 µmol) and Piv-TOB (450 mg, 451 µmol) were placed in a 200 mL two-necked flask, and dissolved in dehydrated dichloromethane (12.1 mL) and dehydrated acetonitrile (3.6 mL) added thereto. Thereafter, triphenylphosphine (31.8 mg, 121 µmol), LNA-A(Bz)-CE phosphoramidite (644 mg, 727 µmol) and 5-ethylthio-1H-tetrazole (94.6 mg, 727 µmol) were successively added, and the mixture was stirred at room temperature for 1.0 hr. Successively, to the reaction solution was added 2,2,2-trifluoroethanol (265 µL, 3.6 mmol) and the mixture was stirred at room temperature for 30 min. 2,6-Xylidine (506 µL, 4.1 mmol) and DDTT (174 mg, 848 µmol) were successively added, and the mixture was stirred at room temperature for 1.7 hr. Thereafter, indole (424 mg, 3.6 mmol) and trifluoroacetic acid (1.1 mL, 14.4 mmol) were successively added, and the mixture was stirred at room temperature for 1.0 hr. Successively, pyridine (3.5 mL, 43.0 mmol) and water (180 µL) were successively added to the reaction solution, and the mixture was stirred at room temperature for 1.0 hr. Thereafter, acetonitrile (112 mL) was added, the precipitated solid was filtered by suction with Kiriyama funnel, and dried to give a dimer HO-A_{L}T-SUC-TOB as a white solid (829.9 mg, 95%).

m/z:Calcd.1751.07, Found 1753.09[M+H]⁺

In the same manner as in the above, the white solid (830 mg) obtained in the above, 2'-F-C(Bz)-CE phosphoramidite, and 4,5-dicyanoimidazole instead of 5-ethylthio-1H-tetrazole were used to synthesize a trimer HO-C_{f}A_{L}T-SUC-TOB as a white solid (780.1 mg, 83%).

m/z:calcd.2231.13, found 1117.07[M+2H]²⁺

In the same manner, the white solid (718 mg) obtained above, 2'-O-TBDMS-C(Ac)-CE phosphoramidite, and ethylthio-1H-tetrazole instead of 4,5-dicyanoimidazole5- were used to synthesize a tetramer HO-rCC_{f}A_{L}T-SUC-TOB as a white solid (758.5 mg, 94%).

m/z:calcd.2761.27, found 1382.15 [M+2H]²⁺ deprotection reaction

The white solid (10 mg) obtained above and 28% aqueous ammonia (5 mL) were placed in an autoclave, heated at 65°C for 4 hr, and then cooled to room temperature. After removing the insoluble material in the reaction solution with a syringe filter, the mixture was concentrated under reduced pressure by a centrifugal evaporator. Then, the concentrate was freeze-dried to give the desired product 2'-O-(tert-butyldimethylsilyl)-cytidine-3'-phosphorothionyl-2'-fluoro-cytidine-3'-phosphorothionyl-2'-O,4'-C-methylene-adenosine-3'-phosphorothionyl-deoxythymidine.

m/z:calcd.1357.24,found 1356.23[M-H]⁻

### Example 4: synthesis of phosphorothioated dimer (phosphoramidite method)

Under an argon atmosphere, HO-C-SUC-TOB(3.0 g,2.3 mmol) and Piv-TOB (3.0 g, 3 mmol) were placed in a 1000-mL three-necked flask, and dissolved in dehydrated chloroform (113 mL) and dehydrated acetonitrile (34 mL) added thereto. Thereafter, triphenylphosphine (296 mg, 1.1 mmol), dT-CE phosphoramidite (3.7 g, 5.0 mmol), and 5-ethylthio-1H-tetrazole (648 mg, 5.0 mmol) were successively added and the mixture was stirred at room temperature for 1.5 hr.

Successively, to the reaction solution was added 2,2,2-trifluoroethanol (1.8 mL, 24.9 mmol), and the mixture was stirred at room temperature for 30 min. 2,6-Xylidine (2.2 mL, 17.6 mmol) and POS (1.2 g, 6.1 mmol) were successively added, and the mixture was stirred at room temperature for 1.5 hr.

Thereafter, 2,3-dimethylfuran (2.6 mL, 24.8 mmol) and trifluoroacetic acid (5.6 mL, 72.8 mmol) were successively added, and the mixture was stirred at room temperature for 1.5 hr. Successively, to the reaction solution were successively added pyridine (44.1 mL, 546 mmol) and water (960 µL), and the mixture was stirred at room temperature for 1.5 hr. Thereafter, acetonitrile (1L) was added, and the precipitated solid was filtered by suction with Kiriyama funnel, and dried to give a dimer HO-TC-SUC-TOB as a white solid (6.7 g, 99%).

### Example 5: synthesis of phosphorothioated trimer (phosphoramidite method)

### elongation reaction

In the same manner as in Example 3 and using HO-(CH₂)₂-SUC-TOB (256 mg) instead of HO-T-SUC-TOB, HO-A_{L}(CH₂)₂-SUC-TOB, then a dimer HO-C_{f}A_{L}(CH₂)₂-SUC-TOB, and finally, a trimer HO-rCC_{f}A_{L}(CH₂)₂-SUC-TOB were obtained as white solids (691 mg). m/z:calcd.2581.22, found 1292.11 [M+2H]²⁺

### deprotection reaction

The white solid (10 mg) obtained above and 28% aqueous ammonia (5 mL) were placed in an autoclave, heated at 65°C for 4 hr, and then cooled to room temperature. After removing the insoluble material in the reaction solution with a syringe filter, the mixture was concentrated under reduced pressure by a centrifugal evaporator. Then, the concentrate was freeze-dried to give the desired product 2-[2'-O-(tertbutyldimethylsilyl)-cytidine-3'-phosphorothionyl-2'-fluoro-cytidine-3'-phosphorothionyl-2'-O,4'-C-methylene-adenosine-3'-phosphorothionyl]ethanol.

m/z:calcd.1177.18,found 1176.18[M-H]⁻

### Example 6: synthesis of phosphorothioated dodecamer (phosphoramidite method)

### elongation reaction

Using dichloromethane instead of chloroform, the same operation as in Example 4 was repeated 18 times to give a dodecamer DMTrO-A·T·C·G·A·C·T·C·T·C·G·A·G·C·G·T·T·C·T·C-SUC-TOB (wherein "·" means a phosphorothionyl bond) as a white solid (8.5 g).

### deprotection reaction

The obtained white solid (5 mg) and 28% aqueous ammonia (5 mL) were placed in an autoclave, heated at 65°C for 4 hr, and then cooled to room temperature. After removing the insoluble material in the reaction solution with a syringe filter, the mixture was concentrated under reduced pressure by a centrifugal evaporator. Then, the concentrate was freeze-dried to give the desired product 5'-O-(4,4'-dimethoxytrityl)-deoxyadenosine-3'-phosphorothionyl-deoxythymidine-3'-phosphorothionyl-deoxycytidine-3'-phosphorothionyl-deoxyguanosine-3'-phosphorothionyl-deoxyadenosine-3'-phosphorothionyl-deoxycytidine-3'-phosphorothionyl-deoxythymidine-3'-phosphorothionyl-deoxycytidine-3'-phosphorothionyl-deoxythymidine-3'-phosphorothionyl-deoxycytidine-3'-phosphorothionyl-deoxyguanosine-3'-phosphorothionyl-deoxyadenosine-3'-phosphorothionyl-deoxyguanosine-3'-phosphorothionyl-deoxycytidine-3'-phosphorothionyl-deoxyguanosine-3'-phosphorothionyl-deoxythymidine-3'-phosphorothionyl-deoxythymidine-3'-phosphorothionyl-deoxycytidine-3'-phosphorothionyl-deoxythymidine-3'-phosphorothionyl-deoxycytidine.

m/z:calcd.6648.73,found 1661.67[M-4H]⁴

### Example 7: synthesis of phosphorothioated dimer (H-phosphonate method)

Under an argon atmosphere, HO-T-SUC-TOB (100 mg, 80.8 µmol), dT-H-phosphonate TEA salt (116.0 mg, 163 µmol) and triphenylphosphine (10.0 mg, 38.1 µmol) were placed in a 50 mL two-necked flask, and dissolved in dehydrated dichloromethane (2.0 mL) and dehydrated pyridine (2.0 mL) added thereto. Thereafter, PivCl (29.5 µl, 242 µmol) was added and the mixture was stirred at room temperature for 1.0 hr.

Successively, to the reaction solution were added DBU (48.4 µL, 324 µmol) and DDTT (34.3 mg, 167 µmol) and the mixture was stirred at room temperature for 30 min. Thereafter, acetonitrile (30 mL) was added, and the precipitated solid was filtered by suction with Kiriyama funnel, and dried to give a white solid (145.7 mg). Under an argon atmosphere, the obtained solid was placed in a 50 mL two-necked flask, and dissolved in dehydrated dichloromethane (4.0 mL) and dehydrated acetonitrile (1.2 mL) added thereto.

Thereafter, indole (46.3 mg, 395 µmol) and trifluoroacetic acid (72.6 µL, 948 µmol) were successively added, and the mixture was stirred at room temperature for 1.0 hr. Successively, to the reaction solution were successively added pyridine (220 µL, 2.7 mmol) and water (42 µL), and the mixture was stirred at room temperature for 1.0 hr. Thereafter, acetonitrile (30 mL) was added, and the precipitated solid was filtered by suction with Kiriyama funnel, and dried to give a dimer HO-T_{OH}T-SUC-TOB as a white solid (85.9 mg, 71%).

m/z:calcd.1557.01, found 1558.02 [M+H]⁺

### Example 8: synthesis of phosphorothioated dimer (H-phosphonate method)

Under an argon atmosphere, HO-T-SUC-TOB (101 mg, 81.3 µmol), dT-H-phosphonate TEA salt (115.4 mg, 163 µmol) and triphenylphosphine (10.8 mg, 41.1 µmol) were placed in a 50-mL two-necked flask, and dissolved in dehydrated dichloromethane (2.0 mL) and dehydrated pyridine (2.0 mL) added thereto. Thereafter, PivCl (29.5 µl, 242 µmol) was added, and the mixture was stirred at room temperature for 1.0 hr. Thereafter, acetonitrile (30 mL) was added, and the precipitated solid was filtered by suction with Kiriyama funnel, and dried to give a white solid (145.2 mg). Under an argon atmosphere, the obtained solid was placed in a 50 mL two-necked flask, and dissolved in dehydrated dichloromethane (4.0 mL) and dehydrated acetonitrile (1.2 mL) added thereto.

Thereafter, indole (47.2 mg, 403 µmol) and trifluoroacetic acid (73.0 µL, 953 µmol) were successively added, and the mixture was stirred at room temperature for 1.0 hr. Then, to the reaction solution were successively added pyridine (230 µL, 2.8 mmol) and water (42 µL), and the mixture was stirred at room temperature for 1.0 hr. Thereafter, acetonitrile (30 mL) was added, and the precipitated solid was filtered by suction with Kiriyama funnel, and dried to give a dimer HO-T_{H}T-SUC-TOB as a white solid (104 mg, 86%).

m/z:calcd.1525.03, found 1526.05[M+H]⁺

### Example 9: synthesis of phosphorothioated dimer (sulfurizing reagent: PADS)

Under an argon atmosphere, HO-T-SUC-TOB (98.8 mg, 79.6 µmol) and Piv-TOB (102 mg, 103 µmol) were placed in a 50-mL two-necked flask, and dissolved in dehydrated dichloromethane (4.0 mL) and dehydrated acetonitrile (1.2 mL) added thereto. Thereafter, triphenylphosphine (13.2 mg, 50.3 µmol), 2'-OMe-G-CE phosphoramidite (144 mg, 165 µmol) and 5-ethylthio-1H-tetrazole (21.2 mg, 163 µmol) were successively added, and the mixture was stirred at room temperature for 1.0 hr.

Successively, to the reaction solution was added 2,2,2-trifluoroethanol (58.9 µL, 810 µmol), and the mixture was stirred at room temperature for 30 min. 2,6-Xylidine (71.2 µL, 577 µmol) and bis phenylacetyl disulfide (62.9 mg, 208 µmol) were successively added, and the mixture was stirred at room temperature for 1.0 hr. Thereafter, indole (98.2 mg, 838 µmol) and trifluoroacetic acid (220 µL, 2.87 mmol) were successively added, and the mixture was stirred at room temperature for 1.0 hr.

Successively, to the reaction solution were successively added pyridine (690 µL, 8.6 mmol) and methanol (57 µL), and the mixture was stirred at room temperature for 1.0 hr. Thereafter, acetonitrile (37 mL) was added, and the precipitated solid was filtered by suction with Kiriyama funnel, and dried to give a dimer HO-GmT-SUC-TOB as a white solid (222.7 mg, 93%).

m/z(TOF-MS):Calcd.1735.09, Found 1737.11[M+H]⁺

### Example 10: synthesis of phosphorothioated dimer (sulfurizing reagent: xanthane hydride)

Under an argon atmosphere, HO-T-SUC-TOB (96.8 mg, 78.2 µmol) and Piv-TOB (99.3 mg, 99.5 µmol) were placed in a 50-mL two-necked flask, and dissolved in dehydrated dichloromethane (4.0 mL) and dehydrated acetonitrile (1.2 mL) added thereto. Thereafter, triphenylphosphine (11.5 mg, 43.8 µmol), 2'-OMe-G-CE phosphoramidite (140 mg, 161 µmol) and 5-ethylthio-1H-tetrazole (20.9 mg, 161 µmol) were successively added and the mixture was stirred at room temperature for 1.0 hr.

Successively, to the reaction solution was added 2,2,2-trifluoroethanol (58.9 µL, 810 µmol), and the mixture was stirred at room temperature for 30 min. 2,6-Xylidine (71.2 µL, 577 µmol) and Xanthane hydride (31.2 mg, 208 µmol) were successively added, and the mixture was stirred at room temperature for 1.0 hr.

Thereafter, indole (96.5 mg, 824 µmol) and trifluoroacetic acid (220 µL, 2.87 mmol) were successively added, and the mixture was stirred at room temperature for 1.0 hr. Successively, to the reaction solution were successively added pyridine (690 µL, 8.6 mmol) and methanol (57 µL), and the mixture was stirred at room temperature for 1.0 hr. Thereafter, acetonitrile (37 mL) was added, and the precipitated solid was filtered by suction with Kiriyama funnel, and dried to give a dimer HO-GmT-SUC-TOB as a white solid (217.9 mg, 91%).

m/z(TOF-MS):Calcd.1735.09, Found 1737.11[M+H]⁺

### Example 11: synthesis of phosphorothioated dimer (sulfurizing reagent: sulfur)

Under an argon atmosphere, HO-T-SUC-TOB (101 mg, 81.3 µmol) and Piv-TOB (98.7 mg, 98.9 µmol) were placed in a 50-mL two-necked flask, and dissolved in dehydrated dichloromethane (4.0 mL) and dehydrated acetonitrile (1.2 mL) added thereto. Thereafter, triphenylphosphine (12.0 mg, 45.8 µmol), 2'-OMe-G-CE phosphoramidite (141 mg, 162 µmol) and 5-ethylthio-1H-tetrazole (21.9 mg, 168 µmol) were successively added, and the mixture was stirred at room temperature for 1.0 hr.

Successively, to the reaction solution was added 2,2,2-trifluoroethanol (58.9 µL, 810 µmol), and the mixture was stirred at room temperature for 30 min. 2,6-Xylidine (71.2 µL, 577 µmol) and sulfur (13.0 mg, 405 µmol) were successively added, and the mixture was stirred at room temperature for 1.0 hr.

Thereafter, indole (96.0 mg, 819 µmol) and trifluoroacetic acid (220 µL, 2.87 mmol) were successively added, and the mixture was stirred at room temperature for 1.0 hr. Then, to the reaction solution were successively added pyridine (690 µL, 8.6 mmol) and methanol (57 µL), and the mixture was stirred at room temperature for 1.0 hr. Thereafter, acetonitrile (37 mL) was added, and the precipitated solid was filtered by suction with Kiriyama funnel, and dried to give a dimer HO-GmT-SUC-TOB as a white solid (221.2 mg, 92%).

m/z(TOF-MS):Calcd.1735.09, Found 1737.11[M+H]⁺

### Example 12: synthesis of phosphorothioated dimer (deprotection: trichloroacetic acid)

Under an argon atmosphere, HO-Um-SUC-TOB (102.4 mg, 81.7 µmol) and Piv-TOB (105 mg, 105 µmol) were placed in a 50 mL two-necked flask, and dissolved in dehydrated dichloromethane (4.0 mL) and dehydrated acetonitrile (1.2 mL) added thereto. Thereafter, triphenylphosphine (9.8 mg, 37.3 µmol), dG-CE phosphoramidite (135.1 mg, 161 µmol) and 5-ethylthio-1H-tetrazole (21.4 mg, 164 µmol) were successively added, and the mixture was stirred at room temperature for 1.0 hr.

Successively, to the reaction solution was added 2,2,2-trifluoroethanol (58.1 µL, 797 µmol) and the mixture was stirred at room temperature for 30 min. 2,6-Xylidine (118 µL, 955 µmol) and DDTT (40.9 mg, 199 µmol) were successively added, and the mixture was stirred at room temperature for 1.0 hr. Thereafter, indole (93.6 mg, 799 µmol) and trichloroacetic acid (698 mg, 4.27 mmol) were successively added, and the mixture was stirred at room temperature for 1.0 hr. Successively, to the reaction solution were successively added pyridine (1.01 mL, 12.6 mmol) and water (66 µL), and the mixture was stirred at room temperature for 1.0 hr. Thereafter, acetonitrile (42 mL) was added, and the precipitated solid was filtered by suction with Kiriyama funnel, and dried to give a dimer HO-GUm-SUC-TOB as a white solid (241.5 mg).

m/z(TOF-MS):calcd.1721.07, found 1723.10[M+H]⁺

### Example 13: synthesis of phosphorothioated dimer (deprotection: dichloroacetic acid)

Under an argon atmosphere, HO-Um-SUC-TOB (99.6 mg, 79.4 µmol) and Piv-TOB (97.9 mg, 98.0 µmol) were placed in a 50 mL two-necked flask, and dissolved in dehydrated dichloromethane (4.0 mL) and dehydrated acetonitrile (1.2 mL) added thereto. Thereafter, triphenylphosphine (10.8 mg, 41.1 µmol), dG-CE phosphoramidite (134.2 mg, 160 µmol) and 5-ethylthio-1H-tetrazole (20.9 mg, 161 µmol) were successively added, and the mixture was stirred at room temperature for 1.0 hr.

Successively, to the reaction solution was added 2,2,2-trifluoroethanol (58.1 µL, 797 µmol) and the mixture was stirred at room temperature for 30 min. 2,6-Xylidine (118 µL, 955 µmol) and DDTT (40.9 mg, 199 µmol) were successively added, and the mixture was stirred at room temperature for 1.0 hr.

Thereafter, indole (93.2 mg, 796 µmol) and dichloroacetic acid (478 µl, 5.78 mmol) were successively added, and the mixture was stirred at room temperature for 1.0 hr. Successively, to the reaction solution were successively added pyridine (1.40 mL, 17.4 mmol) and water (61 µL), and the mixture was stirred at room temperature for 1.0 hr. Thereafter, acetonitrile (40 mL) was added, and the precipitated solid was filtered by suction with Kiriyama funnel, and dried to give a dimer HO-GUm-SUC-TOB as a white solid (228.7 mg, 96%).

m/z(TOF-MS):calcd.1721.07, found 1723.10[M+H]⁺

### [Industrial Applicability]

According to the present invention, the by-production of a phosphotriester form or a phosphodiester form ("PO impurity") can be suppressed by the co-presence of a specific antioxidant in the step of synthesizing a phosphite form or a phosphorous acid diester form, and the subsequent sulfurization reaction can be performed more efficiently. Therefore, it is useful as a production method of an oligonucleotide having a phosphorothioated site.

This application is based on a patent application No. 2019-065158 filed in Japan (filing date: March 28, 2019), the contents of which are incorporated in full herein.

## Claims

1. A method for producing an oligonucleotide having a phosphorothioated site, comprising a step of obtaining a phosphite form or a phosphorous acid diester form by coupling a nucleoside, nucleotide or oligonucleotide wherein a 5'-hydroxy group is not protected, and other group is optionally protected by a protecting group used for nucleic acid synthesis or bonded to a solid phase carrier (hereinafter sometimes to be referred to as "nucleic acid A"), and a nucleoside, nucleotide or oligonucleotide wherein a 3'-hydroxy group or a 3'-amino group is modified by a method for forming a phosphite form or a phosphorous acid diester form, and other group is optionally protected by a protecting group used for nucleic acid synthesis (hereinafter sometimes to be referred to as "nucleic acid B") in the presence of an antioxidant (step (1)).

2. The method for producing an oligonucleotide having a phosphorothioated site according to claim 1, wherein the 3'-hydroxy group or the 3'-amino group in nucleic acid B is modified by a method selected from a phosphoramidite method, an H-phosphonate method, a dihalophosphine method and an oxazaphospholidine method.

3. The method for producing an oligonucleotide having a phosphorothioated site according to claim 1 or 2, further comprising a step of reacting the phosphite form or the phosphorous acid diester form obtained in step (1) with a sulfurizing reagent (step (2)).

4. The method for producing an oligonucleotide having a phosphorothioated site according to any one of claims 1 to 3, wherein the antioxidant is selected from a phosphorus-based antioxidant, a sulfur-based antioxidant, and a phosphorus-sulfur-based antioxidant.

5. The method for producing an oligonucleotide having a phosphorothioated site according to any one of claims 1 to 3, wherein the antioxidant is selected from
a phosphorus-based antioxidant selected from
(1) P-(R)₃
(2) P-(OR)₃
(3) P(R)₂(OR)
(4) PR(OR)₂
(5) PH(O)(R)₂
(6) PH(O)(OR)₂, and
(7) PH(O)R(OR)
(in the above-mentioned formulas (1) - (7), each R is optionally the same or different and independently an optionally substituted aryl group or a lower alkyl group, and two Rs are optionally bonded to each other to form a ring together with the adjacent other atom;
wherein the above-mentioned antioxidant is optionally a dimer in which R portions of the same or different antioxidants are bonded,
a sulfur-based antioxidant selected from
(8) R-S-R, and
(9) 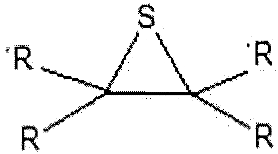 (in the above-mentioned formulas (8) and (9), each R is optionally the same or different and independently a lower alkyl group or an optionally substituted aryl group;
wherein the above-mentioned antioxidant is optionally a dimer in which R portions of the same or different antioxidants are bonded, and
a phosphorus-sulfur-based antioxidant represented by
(10) (OR)₂P(S)-S-M-S-P(S)(OR)₂
wherein each R is optionally the same or different and independently a lower alkyl group or an optionally substituted aryl group, and M is Zn, Ni or Cu;
wherein the above-mentioned antioxidant is optionally a dimer in which R portions of the same or different antioxidants are bonded.

6. The method for producing an oligonucleotide having a phosphorothioated site according to any one of claims 1 to 3, wherein the antioxidant is selected from
a phosphorus-based antioxidant selected from
(1) P-(R)₃
(2) P-(OR)₃
(3) P(R)₂(OR)
(4) PR(OR)₂, and
(5) PH(O)R(OR)
(in the above-mentioned formulas (1) - (5), each R is optionally the same or different and independently an optionally substituted aryl group or a lower alkyl group, and two Rs are optionally bonded to each other to form a ring together with the adjacent other atom;
wherein the above-mentioned antioxidant is optionally a dimer in which R portions of the same or different antioxidants are bonded), and
a sulfur-based antioxidant represented by
(6) 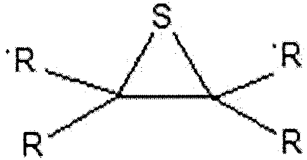 (in the above-mentioned formula (6), each R is optionally the same or different and independently a lower alkyl group or an optionally substituted aryl group;
wherein the above-mentioned antioxidant is optionally a dimer in which R portions of the same or different antioxidants are bonded.)

7. The method for producing an oligonucleotide having a phosphorothioated site according to any one of claims 1 to 3, wherein the antioxidant is selected from triphenylphosphine, methyldiphenylphosphine, triethyl phosphite, ethoxydiphenylphosphine, diethoxyphenylphosphine, 9,10-dihydro-9-oxa-10-phosphaphenanthrene 10-oxide (phosphorus-based antioxidant); and isobutylene sulfide (sulfur-based antioxidant).

8. The method for producing an oligonucleotide having a phosphorothioated site according to any one of claims 1 to 3, wherein the antioxidant is a phosphorus-based antioxidant.

9. The method for producing an oligonucleotide having a phosphorothioated site according to any one of claims 1 to 3, wherein the antioxidant is a phosphine represented by P-(R)₃.

10. The method for producing an oligonucleotide having a phosphorothioated site according to any one of claims 1 to 9, wherein the nucleic acid A is a nucleoside, nucleotide or oligonucleotide wherein the 5'-hydroxy group is not protected, and other group is optionally protected by a protecting group used for nucleic acid synthesis.

11. The production method according to any one of claims 1 to 10, wherein the nucleic acid A is a nucleoside, nucleotide or oligonucleotide wherein a 5'-hydroxy group is not protected, at least one group selected from an amino group and an imino group of a nucleic acid base, a 2'-hydroxy group, a 3'-hydroxy group and a 3'-amino group of a ribose residue, and a 3'-hydroxy group and a 3'-amino group of a deoxyribose residue is protected by a protecting group unremovable under acidic conditions but removable under basic conditions (hereinafter sometimes to be referred to as "pseudo-solid-phase protecting group"), and other group is optionally further protected by a protecting group used for nucleic acid synthesis (hereinafter sometimes to be referred to as "nucleic acid a"), or
a nucleoside, nucleotide or oligonucleotide wherein a 5'-hydroxy group is not protected, one OH of a 3'-terminal phosphate group is replaced by -OLⁿ¹-OH wherein Lⁿ¹ is an organic group, the hydroxy group of -OLⁿ¹-OH is protected by a protecting group unremovable under acidic conditions but removable under basic conditions, and other group is optionally protected by a protecting group used for nucleic acid synthesis (hereinafter sometimes to be referred to as "nucleic acid α"), and
the nucleic acid B is a nucleoside, nucleotide or oligonucleotide wherein a 3'-hydroxy group or 3'-amino group is modified by a method for forming a phosphite form or a phosphorous acid diester form, a 5'-hydroxy group is protected by a temporary protecting group removable under acidic conditions, and other group is optionally protected by a protecting group selected from protecting groups unremovable under acidic conditions but removable under basic conditions (hereinafter sometimes to be referred to as "pseudo-solid-phase protecting group") and protecting groups used for nucleic acid synthesis (hereinafter sometimes to be referred to as "nucleic acid b").

12. The method for producing an oligonucleotide having a phosphorothioated site according to any one of claims 1 to 11, wherein the nucleic acid B is a nucleoside, nucleotide or oligonucleotide wherein a 3'-hydroxy group is modified by a method for forming a phosphite form or a phosphorous acid diester form, and other group is optionally protected by a protecting group used for nucleic acid synthesis.

13. The method for producing an oligonucleotide having a phosphorothioated site according to any one of claims 3 to 12, wherein the sulfurizing reagent used in step (2) is selected from 5-[(N,N-dimethylaminomethylidene)amino]-3H-1,2,4-dithiazole-3-thione(DDTT), 3H-1,2-benzodithiol-3-one-1,1-dioxide (Beaucage reagent), 3H-1,2-benzodithiol-3-one, phenylacetyl disulfide (PADS), tetraethylthiuram disulfide (TETD), dipentamethylenethiuram tetrasulfide, 5-phenyl-3H-1,2,4-dithiazol-3-one (POS), 3-amino-1,2,4-dithiazole-5-thione (ADTT, Xanthane hydride), N-[(2-cyanoethyl)thio]phthalimide and sulfur.

14. The method for producing an oligonucleotide having a phosphorothioated site according to any one of claims 1 to 13, further comprising a step of removing all protecting groups of the obtained oligonucleotide having a phosphorothioated site, and then isolating an unprotected oligonucleotide having a phosphorothioated site.
